# EUROPEAN PATENT APPLICATION

(11) **EP 3 210 972 A1**
(43) Date of publication of application: **30.08.2017**
(21) Application number: 16201768.5
(22) Date of filing: 24.05.2013
(51) Int. Cl.: C07D 213/56, C07D 401/06, A61K 31/22, A61K 31/366, A61K 31/40, A61K 31/401, A61K 31/404, A61K 31/4418, A61K 31/505, A61P 3/00, A61P 3/10

(54) **METHODS OF LOWERING PROPROTEIN CONVERTASE SUBTILISIN/KEXIN TYPE 9 (PCSK9)**

(30) Priority: 25.05.2012 US 201261651870 P; 05.09.2012 US 201261697104 P; 13.03.2013 US 201361780445 P
(62) Divisional of application: 13794508.5
(71) Applicant: Catabasis Pharmaceuticals, Inc., Cambridge, MA 02139 (US)
(72) Inventor: MILNE, Jill C., Brookline, MA 02446 (US); JIROUSEK, Michael R., Chardon, OH 44024 (US); VU, Chi B., Boston, MA 02116 (US)
(74) Representative: Grund, Martin

(57) **Abstract**

The invention relates to compounds VI for use in modulating cholesterol by inhibiting proprotein convertase subtilisin/kexin type 9 (PCSK9) with fatty acid derivatives; and new methods for treating or preventing a metabolic disease comprising the administration of an effective amount of a fatty acid derivative. The present invention is also directed to fatty acid bioative derivatives and their use in the treatment of metabolic diseases.

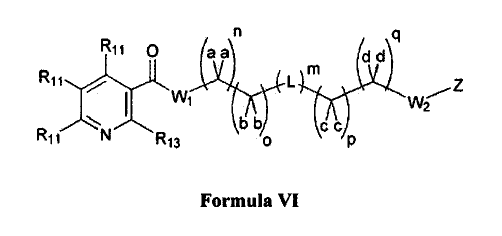

## Description

### PRIORITY

This application claims the benefit of U.S. Provisional Application No. 61/651,870 filed May 25, 2012; U.S. Provisional Application No. 61/697,104 filed September 5,2013; and U.S. Provisional Application No. 61/780,445 filed March 13, 2013, the entire disclosures of which are relied on and hereby incorporated into this application by reference.

### FIELD OF THE INVENTION

The invention relates to new methods of modulating cholesterol in a subject by inhibiting proprotein convertase subtilisin/kexin type 9 (PCSK9) protein with fatty acid derivatives; and new methods for treating or preventing a metabolic discase comprising the administration of an effective amount of a fatty acid derivative. The present invention is also directed to fatty acid bioative derivatives and their use in the treatment of metabolic diseases.

### BACKGROUND OF THE INVENTION

Recent studies have demonstrated that proprotein convertase subtilisin/kexin type 9 (PCSK9) could be an attractive therapeutic target for lowering low-density lipoprotein-cholesterol (LDL-C). In terms of validation, gain or loss-of-function PCSK9 variants in humans have been shown to result in hypercholesterolemia or hypocholesterolemia respectively. For instance, gain-of-function mutations in the PCSK9 gene are associated with elevated scrum LDL-C levels of > 300 mg/dL and premature cardiovascular heart disease (Abifadel et al Nat. Gent. 2003, 34, p. 154-156). On the other hand, loss-of-function mutations in the PCSK9 gene are associated with low serum LDL-C of ≤ 100 mg/dL and a reduction in cardiovascular heart disease (Cohen et al Nat. Gent. 2005, 37, p. 161-165). PCSK9 is a serine protease, made primarily by the liver and intestine, and consists of a signal peptide, a prodomain, a catalytic domain, and the histidine-rich C terminal domain (Piper et al Structure 2007, 15, p. 545-552). Data has shown that PCSK9 can exert its effects on LDL-C by binding to hepatocyte LDL receptor and preventing it from recycling to the cell surface after endocytosis. This sequence of events results in reduced LDL receptor levels, decreased cellular uptake of LDL-C, and higher LDL-C levels in blood (Horton et al J. Lip. Res. 2009, 50 (Suppl.), p. S 172-S 177). Neutralizing antibodies to PCSK9 have now been shown to significantly reduce serum LDL-C in mice and nonhuman primates (Chan et al PNAS 2009, 106, p. 9820-9825; Liang et al Pharmacology and Experimental Therapeutics 2012, 340, p. 228-236). REGN727, AMG 145, RN316, and LGT209 are some monoclonal antibodies that are currently being evaluated in human clinical trials for hypercholesterolemia.

The statin drug class has been used extensively in the clinic to lower cholesterol. However, statin treatment has been shown to significantly increase the expression of PCSK9 (Dubuc et al Arterioscler. Thromb. Vasc. 2004, p. 1453-1459). The increased level of PCSK9 essentially counteracts some of the beneficial effects of statins since PCSK9 enhances the degradation of LDL receptors, leading to higher plasma levels of LDC-C.

Oily cold water fish, such as salmon, trout, herring, and tuna are the source of dictary marine omega-3 fatty acids, cicosapentaenoic acid (EPA) and docosahcxacnoic acid (DHA) being the key marine derived omega-3 fatty acids. Both niacin and marine omega-3 fatty acids (EPA and DHA) have been shown to reduce cardiovascular disease, coronary heart disease, atherosclerosis and reduce mortality in patients with dyslipidemia, hypercholesterolemia, or Type 2 diabetes, and metabolic disease. Niacin at high dose (1.5 to 4 grams per day) has been shown to improve very low-density lipoprotein ("VLDL") levels through lowering Apolipoprotein B ("ApoB") and raising high density lipoprotein ("HDL") through increasing Apolipoprotein A1 ("ApoA1") in the liver. Niacin can also inhibit diacylglycerol acyltransferase-2, a key enzyme for TG synthesis (Kamanna, V. S.; Kashyap, M. L. Am. J. Cardiol. 2008, 101 (8A), 20B-26B). Unfortunately, niacin has many actions outside of the liver that detract from its therapeutic utility. The most common side effect of niacin is flushing, which can limit the dose a patient can tolerate. Flushing is thought to occur through the GPR109 receptor in the vasculature.

Omega-3 fatty acids have previously been shown to improve insulin sensitivity and glucose tolerance in normoglycemic men and in obese individuals. Omega-3 fatty acids have also been shown to improve insulin resistance in obese and non-obese patients with an inflammatory phenotype. Lipid, glucose, and insulin metabolism have been shown to improve in overweight hypertensive subjects through treatment with omega-3 fatty acids. Omega-3 fatty acids (EPA/DHA) have also been shown to decrease triglycerides and to reduce the risk for sudden death caused by cardiac arrhythmias in addition to improve mortality in patients at risk of a cardiovascular event. Omega-3 fatty acids have also been taken as dietary supplements part of therapy used to treat dyslipidemia, and anti-inflammatory properties. A higher intake of omega-3 fatty acids lower levels of circulating TNF-α and IL-6, two of the cytokines that are markedly increased during inflammation processes (Chapkin et al, Prostaglandins, Leukot Essent Fatty Acids 2009, 81, p. 187-191; Duda et al, Cardiovasc Res 2009, 84, p. 33-41). In addition, a higher intake of omega-3 fatty acids has also been shown to increase levels of the well-characterized anti-inflammatory cytokine IL-10 (Bradley et al, Obesity (Silver Spring) 2008, 16, p. 938-944). More recently, DHA has been shown to attenuate kidney disease and prolong the lifespan of autoimmune lupus-prone mice (Halade et al, J. Immunology 2010, 184, p. 5280-6). Studies have shown that DHA could potentially suppress glomerulonephritis because of its ability to lower LPS-mediated increase in serum IL-18 as well as its ability to dampen LPS-mediated PI3K, Akt and NF-κB activation in the kidney.

Hyperlipidemia are classified according to which types of lipids are elevated, that is hypercholesterolemia, hypertriglyceridemia, or both in combined hyperlipidemia. Elevated levels of lipoprotein may also be classified as a form of hyperlipidemia. There are five types of hyperlipoproteinemia (types I through V) and these are further classified according to the Fredrikson classification, based on the pattern of lipoproteins on electrophoresis or ultracentrifugation. **Type I hyperlipoproteinemia** has three subtypes: Type Ia (also called Buerger-Gruetz syndrome or familial hyperchylomicronemia), Type Ib (also called familial apoprotein CII deficiency) and Type Ic. Due to defects in either decreased in lipoprotein lipase (LPL), altered ApoC2 or LPL inhibitor in blood, all three subtypes of Type I hyperlipoproteinemia share the same characteristic increase in chylomicrons. The frequency of occurrence for Type I hyperlipoproteinemia is 1 in 1,000,000 and thus far no drug therapy is available and treatment has consisted only of diet. **Type II hyperlipoproteinemia** has two subtypes: Type IIa (also called familial hypercholesterolemia) is characterized by an elevated level of low-density lipoprotein (LDL); and Type IIb (also called familial combined hyperlipidemia) is characterized by an elevated level of LDL and very-low density lipoprotein (VLDL). **Type III hyperlipoproteinemia** (also called familial dysbetalipoproteinemia) is characterized by an elevated level of intermediate-density lipoprotein (IDL). **Type IV hyperlipoproteinemia** (also called familial hypertriglyceridemia) is characterized by an elevated level of VLDL. **Type V hyperlipoproteinemia** is characterized by an elevated level of VLDL and chylomicrons. Treatment for Type V hyperlipoproteinemia thus far has not been adequate with using just niacin or fibrate.

The present invention is directed to overcome the above-described deficiencies in the treatment of metabolic diseases.

### SUMMARY OF THE INVENTION

The invention is based in part on the discovery of fatty acid derivatives and their demonstrated effects in achieving improved treatment that cannot be achieved by administering the omega-3 fatty acid EPA or DHA alone or in combination with other bioactives. These omega-3 fatty acid derivatives are designed to be stable in the plasma and absorbed by cells where they inhibit the production or secretion of PCSK9. Inhibiting the production or secretion of PCSK9 has the effect of reducing plasma cholesterol levels in animals and humans. In addition, since omega-3 fatty acid derivatives are inhibitors of PCSK9, they can enhance the efficacy of statins when administered in combination.

Accordingly in one aspect, the present invention relates to a method of treating metabolic diseases. The method involves inhibiting the production or lowering serum levels of PCSK9 by administering to a patient in need thereof an effective amount of a fatty acid bioactive derivative. In one embodiment, the fatty acid bioactive derivative comprises a fatty acid covalently linked to a bioactive molecule, wherein the fatty acid is selected from the group consisting of omega-3 fatty acids and fatty acids that are metabolized *in vivo* to omega-3 fatty acids.

In another aspect, a method of inhibiting the production of PCSK9 or lowering serum levels of PCSK9 is provided. The method involves administering to a patient in need thereof a compound of the **Formula I** : 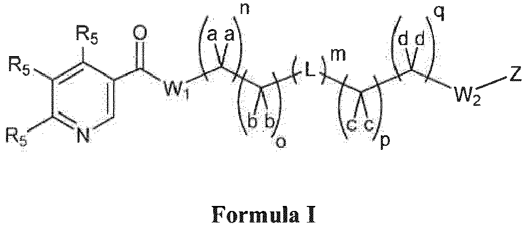 or a pharmaceutically acceptable salt, hydrate, solvate, prodrug, enantiomer or a stereoisomer thereof;
wherein
W₁ and W₂ are each independently null, O, S, NH, NR, or W₁ and W₂ can be taken together can form an imidazolidine or piperazine group, with the proviso that W₁ and W₂ can not be O simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a heteroaryl, 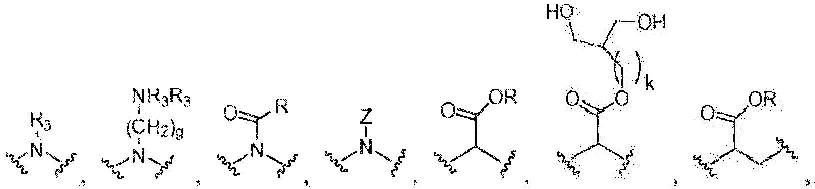 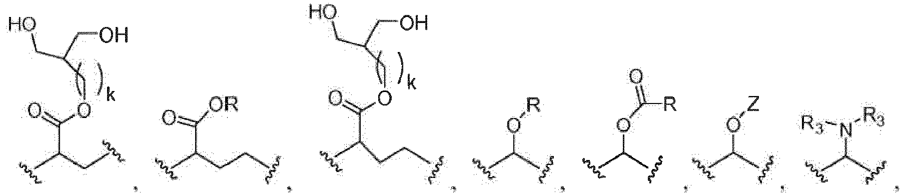 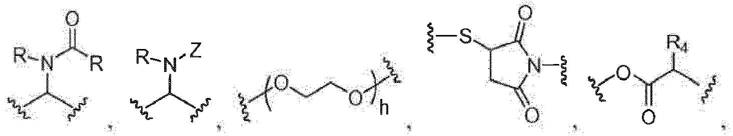 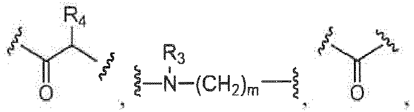 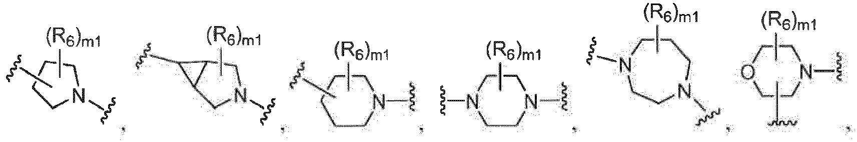 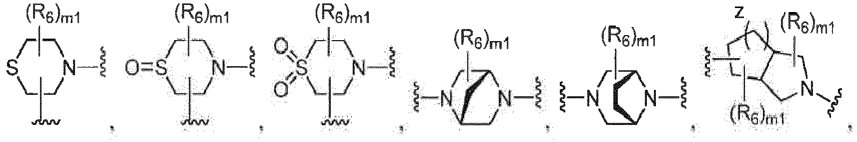 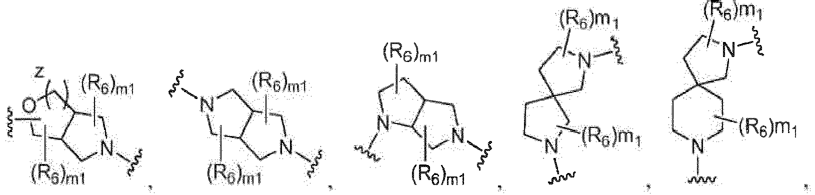 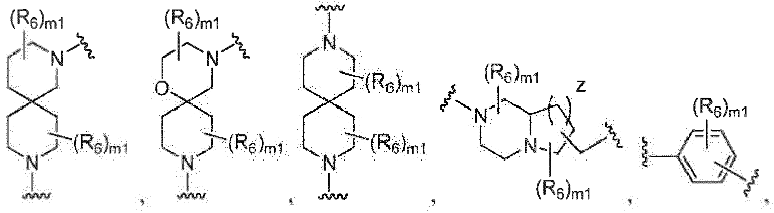 wherein the representation of L is not limited directionally left to right as is depicted, rather either the left side or the right side of L can be bound to the W₁ side of the compound of Formula I;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is cach independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
each g is independently 2, 3 or 4;
cach h is independently 1,2,3 3 or 4;
m is 0, 1, 2, or 3; ifm is more than 1, then L can be the same or different;
m1 is 0, 1, 2 or 3;
k is 0, 1, 2, or 3;
z is 1, 2, or 3;
each R₃ is independently H or C₁-C₆ alkyl, or both R₃ groups, when taken together with the nitrogen to which they are attached, can form a heterocycle;
each R₄ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, 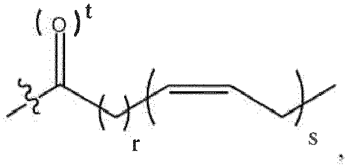 or 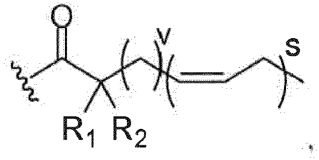 with the proviso that there is at least one 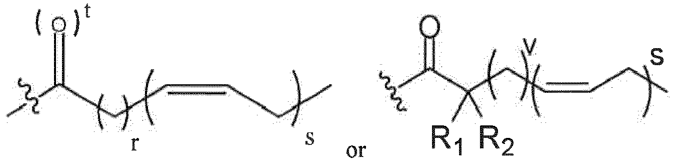 in the compound;
each r is independently 2, 3, or 7;
each s is independently 3, 5, or 6;
each t is independently 0 or 1;
each v is independently 1, 2, or 6;
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, phenyl or straight or branched C₁-C₄ alkyl optionally substituted with OH, or halogen.

Another aspect relates to a method of inhibiting the production or lowering scrum levels of PCSK9 which comprises administering to a patient in need thereof a compound of the **Formula II:** 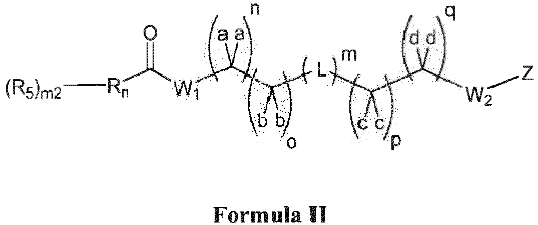 or a pharmaceutically acceptable salt, hydrate, solvate, prodrug, enantiomer or a stereoisomer thereof
wherein
Rₙ is a phenyl, naphthyl, heteroaryl, heterocycle , 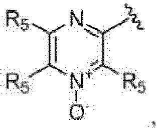 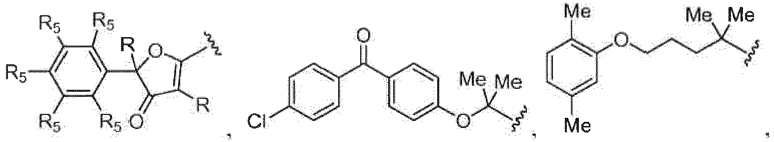 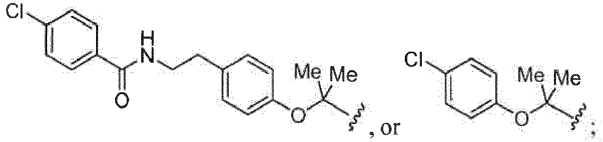 W₁ and W₂ are each independently null, O, S, NH, NR, or W₁ and W₂ can be taken together can form an imidazolidine or piperazine group, with the proviso that W₁ and W₂ can not be O simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a heteroaryl, 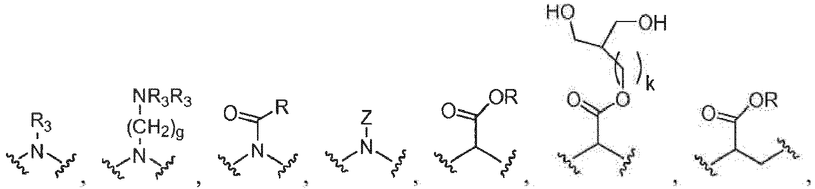 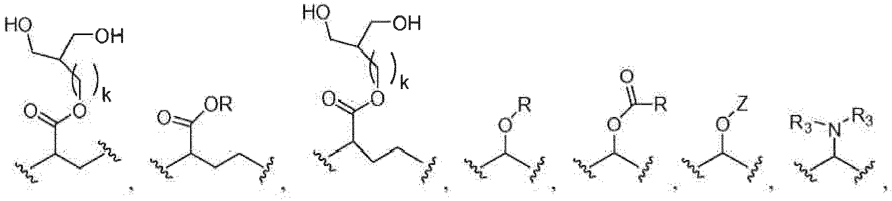 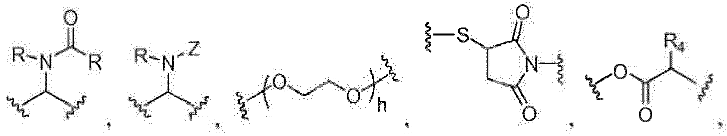 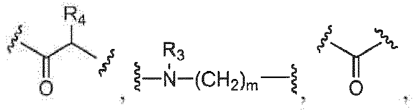 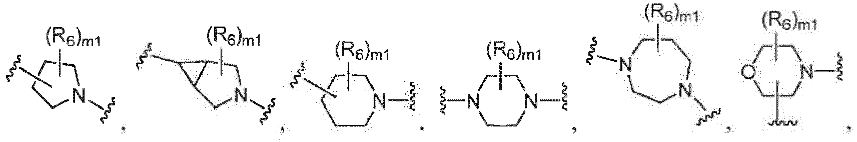 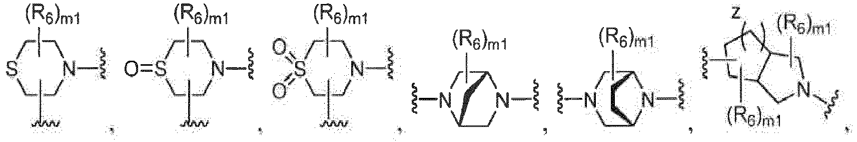 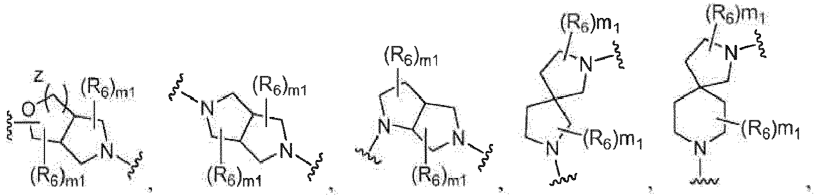 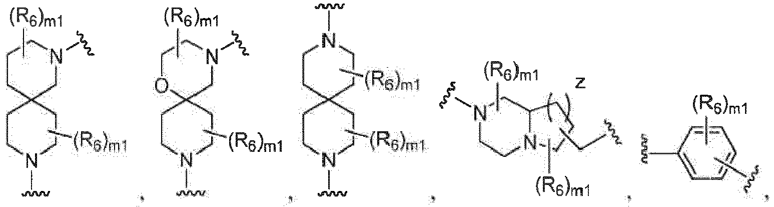 wherein the representation of L is not limited directionally left to right as is depicted, rather either the left side or the right side of L can be bound to the W₁ side of the compound of Formula II;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is cach independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
each g is independently 2, 3 or 4;
each h is independently 1, 2, 3 or 4;
m is 0, 1, 2, or 3; if m is more than 1, then L can be the same or different;
m1 is 0, 1,2 or 3;
m2 is 0, 1, 2, 3, 4 or 5;
k is 0, 1,2, or 3;
z is 1, 2, or 3;
each R₃ is independently H or C₁-C₆ alkyl, or both R₃ groups, when taken together with the nitrogen to which they are attached, can form a heterocycle;
each R₄ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, 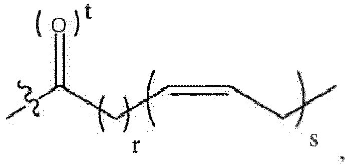 or 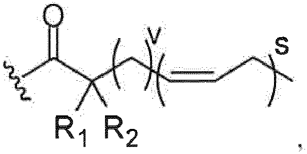 with the proviso that there is at least one 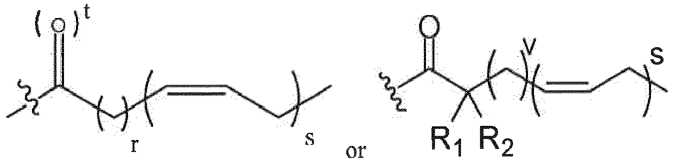 in the compound;
each r is independently 2, 3, or 7;
each s is independently 3, 5, or 6;
each t is independently 0 or 1;
each v is independently 1, 2, or 6;
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, phenyl or straight or branched C₁-C₄ alkyl optionally substituted with OH, or halogen;

In another aspect, a method of inhibiting the production of PCSK9 or lowering serum levels of PCSK9 is provided. The method involves administering to a patient in need thereof a compound of the **Formula III**: 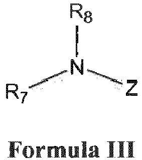 or a pharmaceutically acceptable salt, hydrate, solvate, enantiomer or a stereoisomer thereof;
wherein 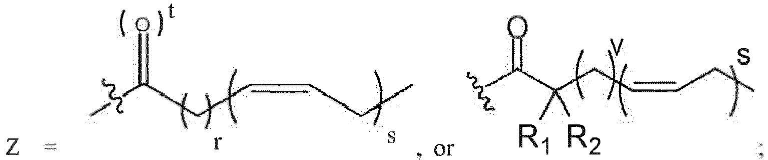 each r is independently 2, 3, or 7;
each s is independently 3, 5, or 6;
each t is independently 0 or 1;
each v is independently 1, 2, or 6;
R₁ and R₂ arc independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₇ and R₈ are independently 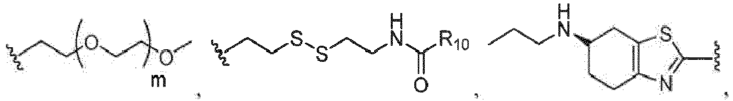 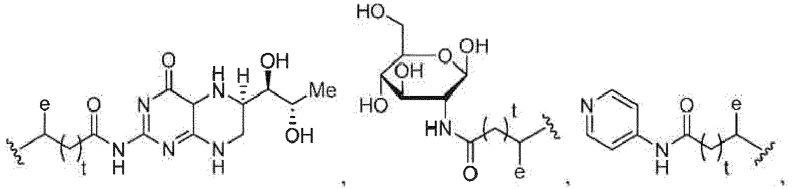 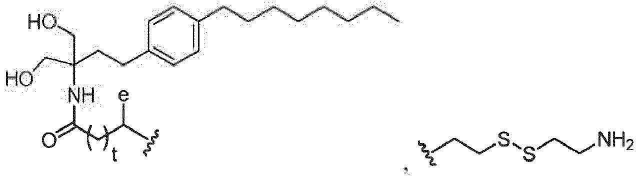 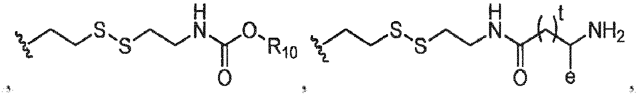 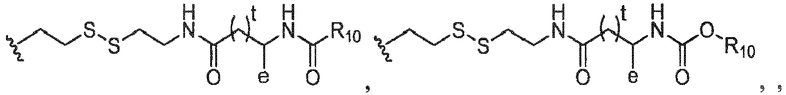 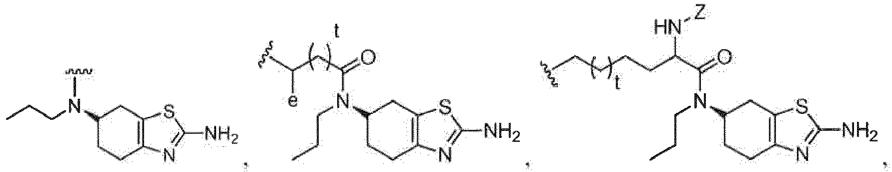 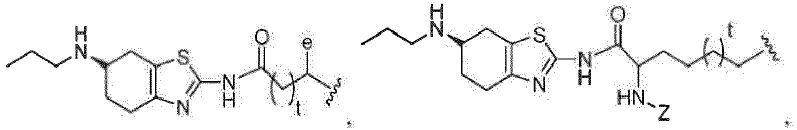 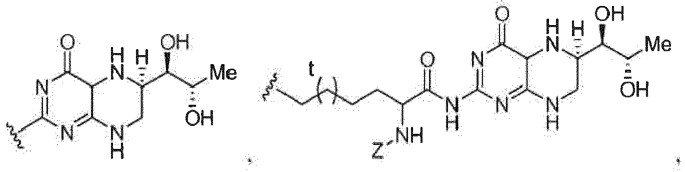 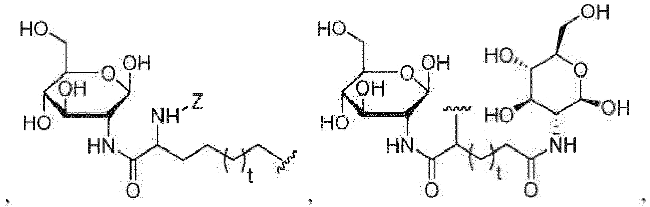 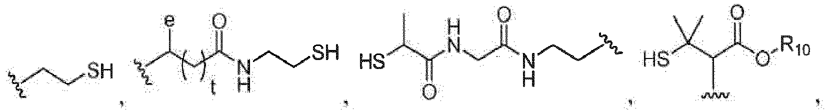 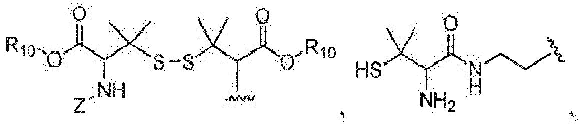 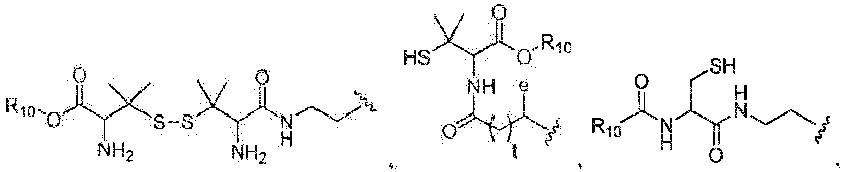 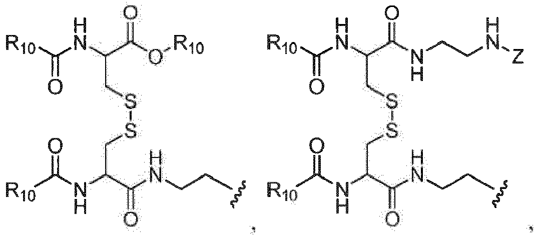 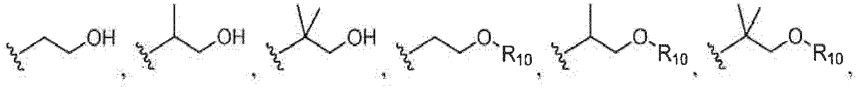 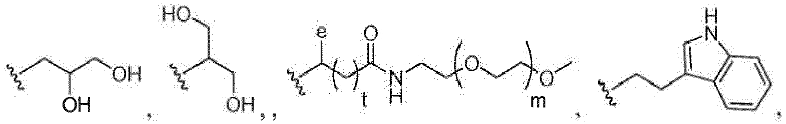 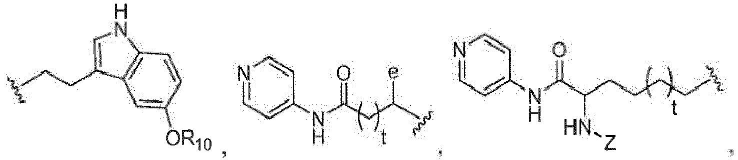 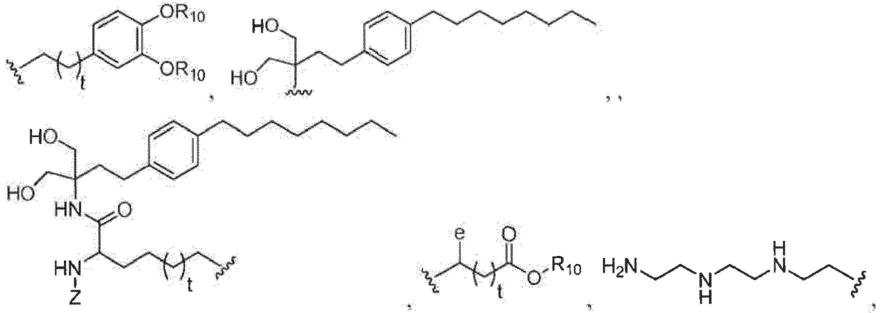 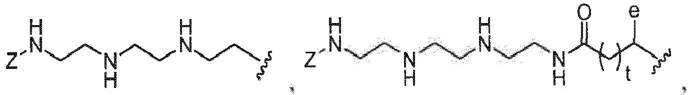 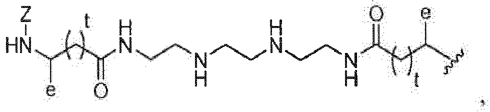 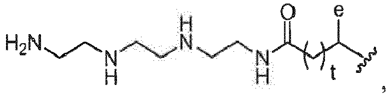 H, D, C₁-C₆ alkyl, C₁-C₆ cycloalkyl, aryl, heteroaryl, and heterocycle;
each e is independently H or any one of the side chains of the naturally occurring amino acids;
each m is independently 1, 2, 3, 4, 5, 6, 7, 8, 9, 10, 11, or 12;
cach R₉ is independently -H, -C₁-C₃ alkyl, or straight or branched C₁-C₄ alkyl optionally substituted with OH, or halogen; and
each R₁₀ is independently -H, straight or branched -C₁-C₆ alkyl, -C₁-C₆ cycloalkyl, aryl, heteroaryl or heterocyclic that is optionally substituted with one, two, three, four or five groups selected from OH, CN, halogen, CO₂R₉, CONHR₉, CONR₉R₉, S(O)₂NR₉R_{9.} NR₉R₉, NR₉COR₉, -(OCH₂CH₂)ₘ-OCH₃.

In another aspect, a method of inhibiting the production or lowering serum levels of PCSK9 is provided. The method comprises administering to a patient in need thereof a compound of the **Formula IV:** 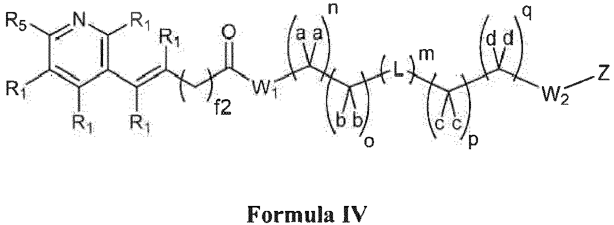 or a pharmaceutically acceptable salt, hydrate, solvate, prodrug, enantiomer or a stereoisomer thereof
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is independently selected from the group consisting of H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₆ alkyl, -O-C₁-C₃ alkyl, -S(O)C₁-C₃ alkyl, -S(O)₂C₁-C₃ alkyl, an aryl, a cycloalkyl, a heterocycle and 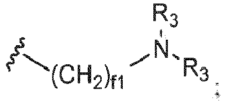 R₃ is independently H or C₁-C₆ alkyl, or both R₃ groups, when taken together with the nitrogen to which they are attached, can form 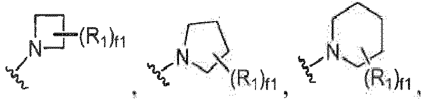 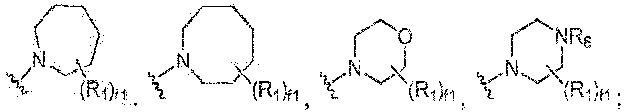 f1 = 1, 2, 3 or 4;
f2 = 1, 2 or 3;
W₁ and W₂ are each independently null, O, S, NH, NR, or W₁ and W₂ can be taken together can form an imidazolidine or piperazine group, with the proviso that W₁ and W₂ can not be O simultaneously;
cach a, b, c, and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
each n, o, p, and q is independently 0, 1 or 2;
each L is independently-O-, -S-, -S(O)-, -S(O)₂-, -S-S-, -(C₁-C₆alkyl)-, -(C₃-C₆cycloalkyl)-, a heterocycle, a heteroaryl, 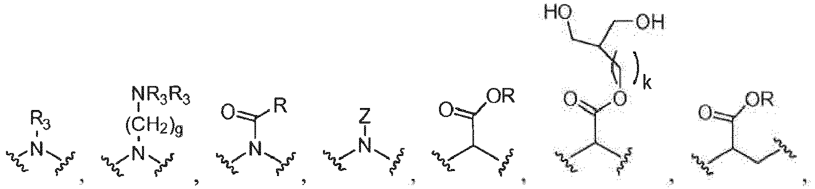 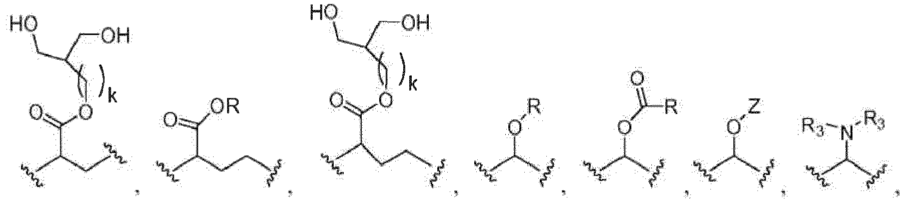 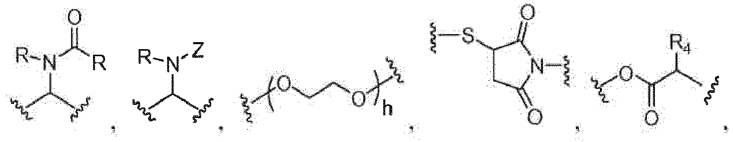 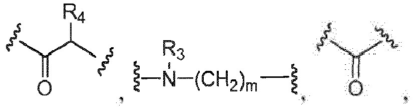 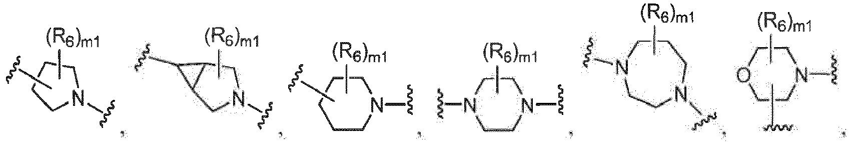 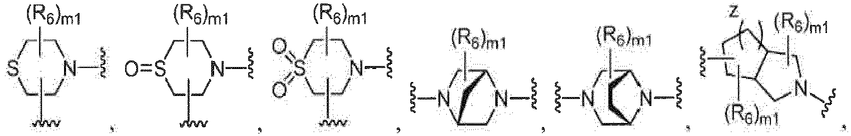 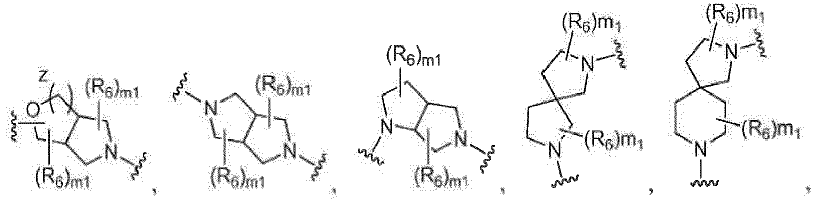 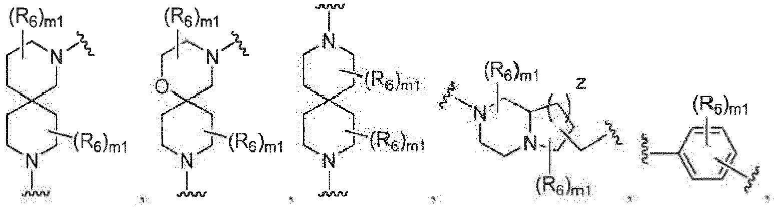 wherein the representation of L is not limited directionally left to right as is depicted, rather either the left side or the right side of L can be bound to the W₁ side of the compound of Formula IV;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
cach g is independently 2, 3 or 4;
each h is independently 1, 2, 3 or 4;
m is 0, 1, 2, 3, 4 or 5; if m is more than 1, then L can be the same or different;
m1 is 0, 1, 2 or 3;
k is 0, 1, 2, or 3;
z is 1, 2, or 3;
each R₄ independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 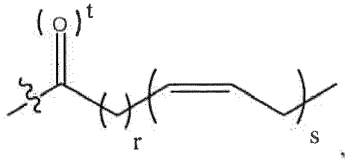 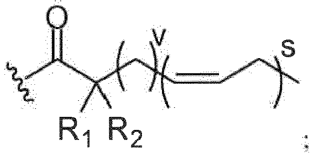 or 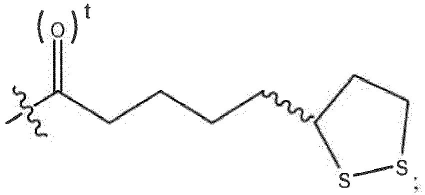 with the proviso that there is at least one 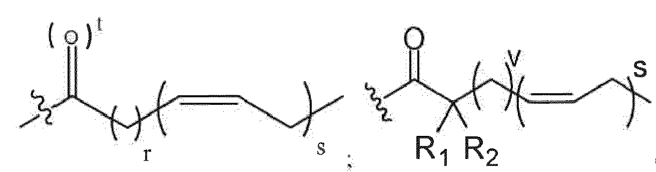 or 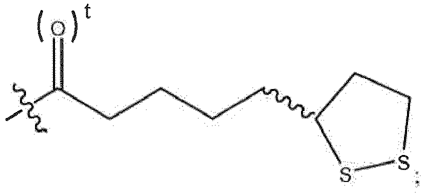 in the compound;
each r is independently 2, 3, or 7;
each s is independently 3, 5, or 6;
each t is independently 0 or 1;
each v is independently 1, 2, or 6;
each R is independently -H, -C₁-C₃ alkyl, or straight or branched C₁-C₄ alkyl optionally substituted with OH, or halogen;
provided that
when m, n, o, p, and q are each 0, W₁ and W₂ are each null, and Z is 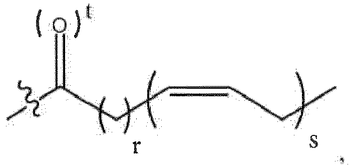 then t must be 0; and
when m, n, o, p, and q are each 0, and W₁ and W₂ are each null, then Z must not be

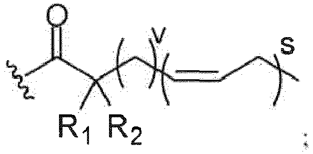

Another aspect relates to a method of inhibiting the production of or lowering serum levels of PCSK9; the method comprising administering to a patient in need thereof a compound of the Formula V: 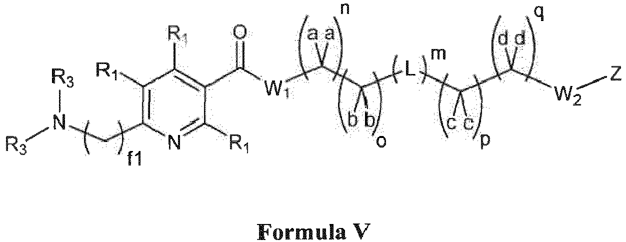 or a pharmaceutically acceptable salt, hydrate, solvate, prodrug, enantiomer or stereoisomer thereof;
wherein
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₃ is independently H or C₁-C₆ alkyl, or both R₃ groups, when taken together with the nitrogen to which they are attached, can form 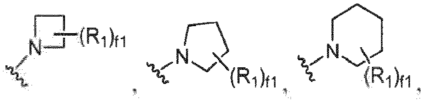 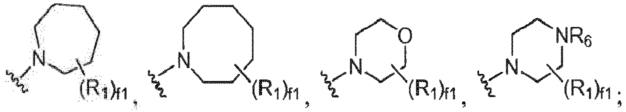 f1 = 1, 2, 3 or 4;
W₁ and W₂ are each independently null, O, S, NH, NR, or W₁ and W₂ can be taken together can form an imidazolidine or piperazine group, with the proviso that W₁ and W₂ can not be O simultaneously;
each a, b, c, and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
cach n, o, p, and q is independently 0, 1 or 2;
each L is independently -O-, -S-, -S(O)-, -S(O)₂-, -S-S-, -(C₁-C₆alkyl)-, -(C₃-C₆cycloalky)-, a heterocycle, a heteroaryl, 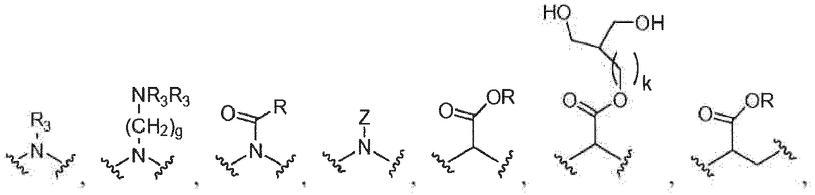 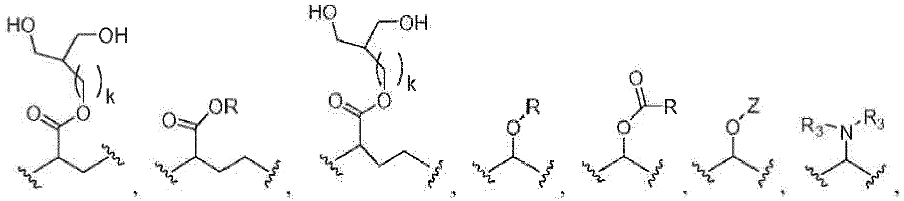 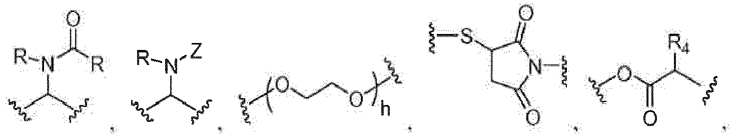 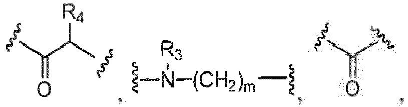 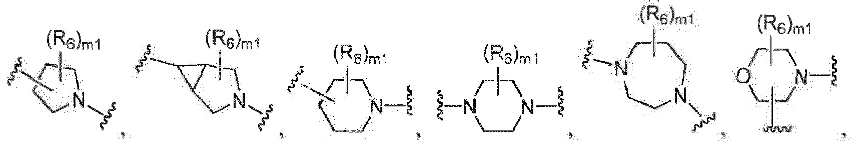 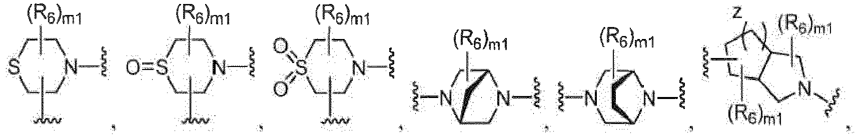 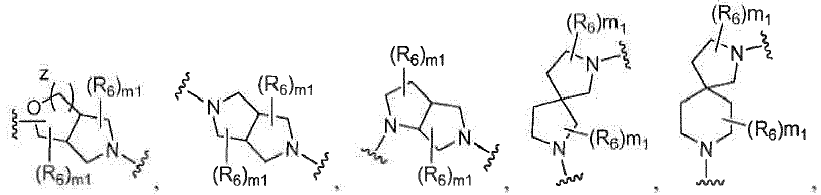 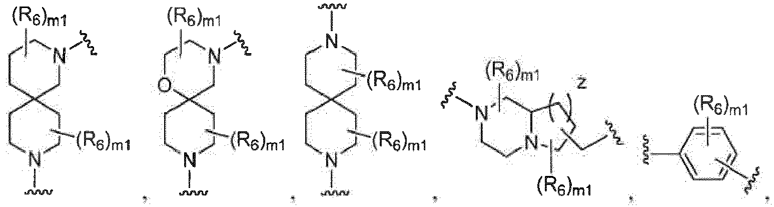 wherein the representation of L is not limited directionally left to right as is depicted, rather either the left side or the right side of L can be bound to the W₁ side of the compound of Formula V;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3 or 4;
each h is independently 1, 2, 3 or 4;
m is 0, 1, 2, 3, 4 or 5; if m is more than 1, then L can be the same or different;
m1 is 0, 1,2 or 3;
k is 0, 1, 2, or 3;
z is 1, 2, or 3;
each R₄ independently e, H or straight or branched C₁-C₁₀ alkyl which can be optionally substituted with OH, NH₂, CO₂R, CONH₂, phenyl, C₆H₄OH, imidazole or argininc;
each e is independently H or any one of the side chains of the naturally occurring amino acids;
each Z is independently -H, or 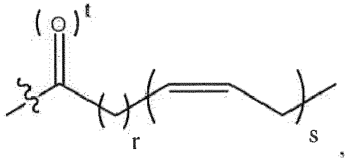 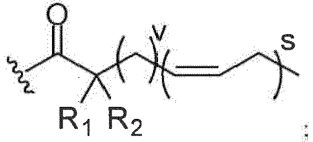 or 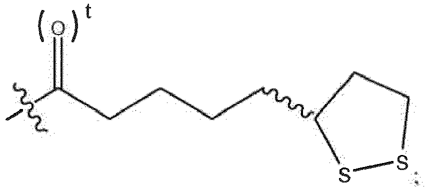 with the proviso that there is at least one 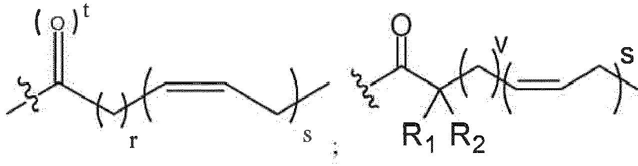 or 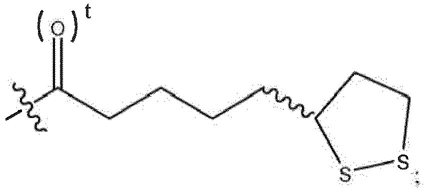 in the compound;
each r is independently 2, 3, or 7;
each s is independently 3, 5, or 6;
each t is independently 0 or 1;
each v is independently 1, 2, or 6;
each R is independently -H, -C₁-C₃ alkyl, or straight or branched C₁-C₄ alkyl optionally substituted with OH, or halogen;
provided that
when m, n, o, p, and q are each 0, W₁ and W₂ are each null, and Z is 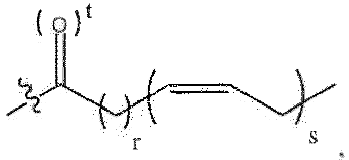 then t must be 0; and
when m, n, o, p, and q are each 0, and W₁ and W₂ are each null, then Z must not be

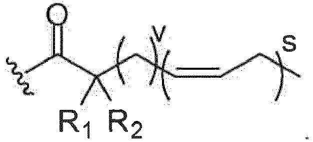

Yet another aspect of the invention relates to compounds of **Formula II':** 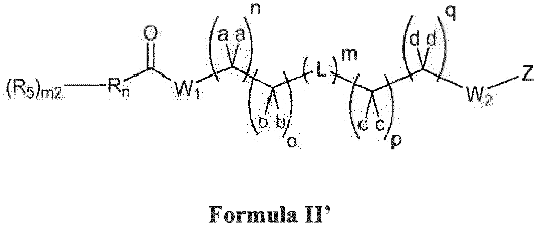 and pharmaceutically acceptable salts, hydrates, solvates, prodrugs, enantiomers and stereoisomers thereof
wherein
Rₙ is phenyl, naphthyl, heteroaryl, or a heterocycle ;
W₁ and W₂ are each independently null, O, S, NH, NR, or W₁ and W₂ can be taken together can form an imidazolidine or piperazine group, with the proviso that W₁ and W₂ can not be O simultaneously;
W₃ is independently O or null;
R₁₂ is independently H, OH, OR", R", or OC(O)R" where R" is independently C₁-C₆ alkyl;
each m1 is independently 0, 1, 2 or 3;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a hctcroaryl, 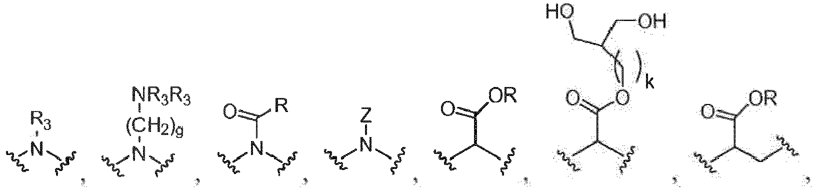 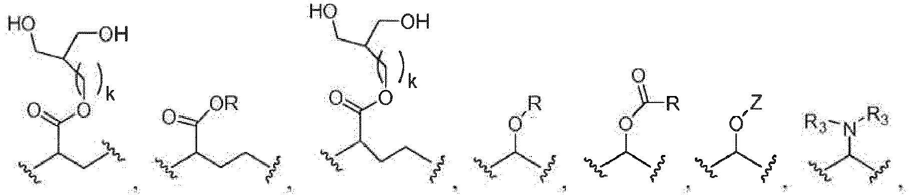 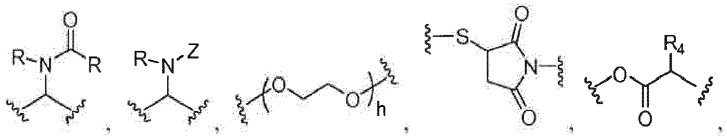 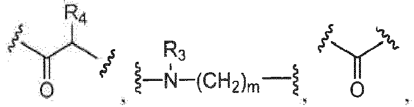 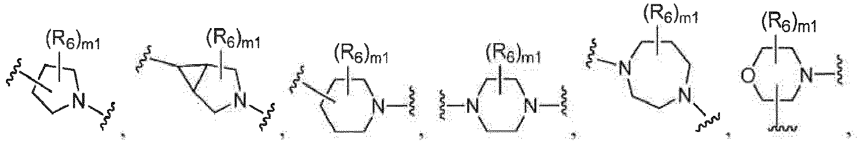 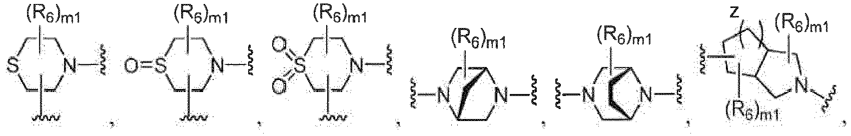 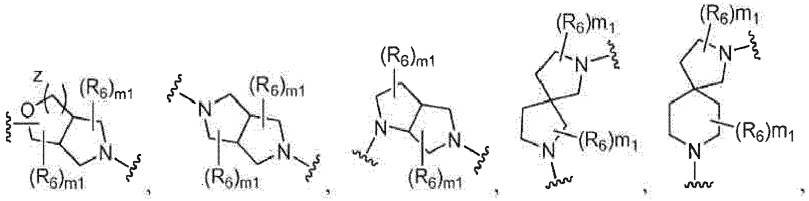 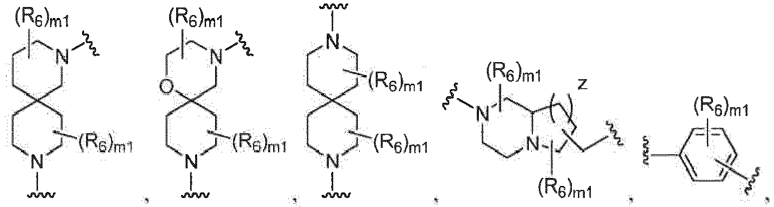 wherein the representation of L is not limited directionally left to right as is depicted, rather either the left side or the right side of L can be bound to the W₁ side of the compound of Formula II';
with the proviso that when L is independently -O-, -S-, -S(O)-, -S(O)₂-, -S-S-, 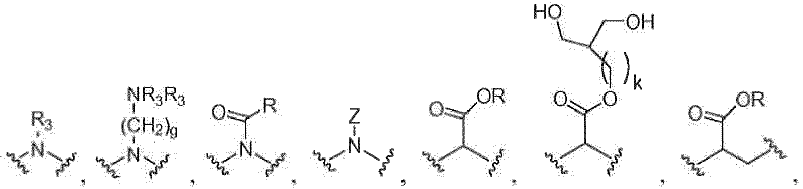 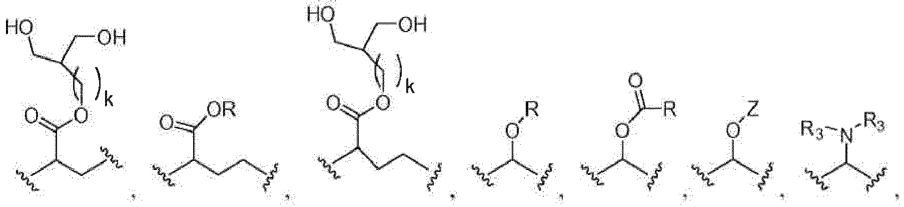 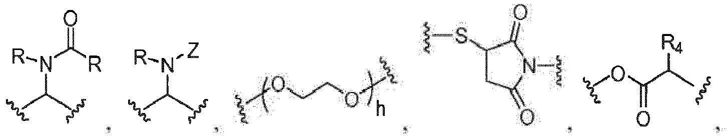 then Rn is not 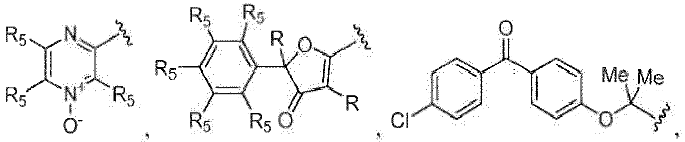 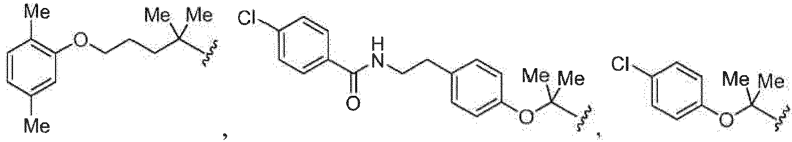 and in which g, h, k, R, R₃, R₅ and Z arc as defined below;
and with the further proviso that Rn is not: 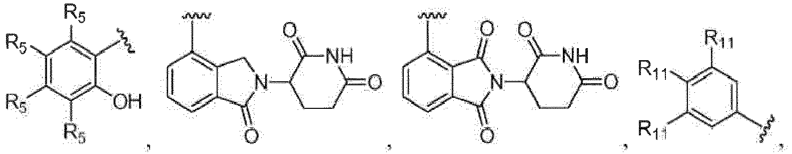 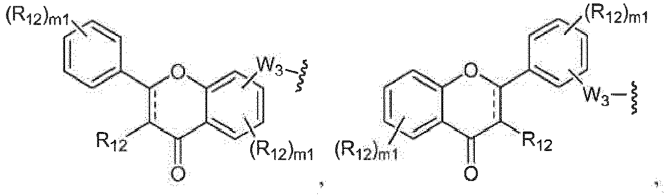 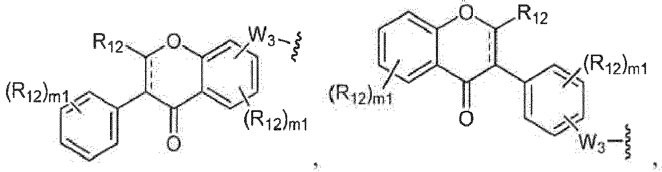 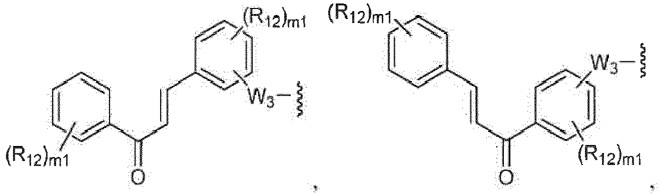 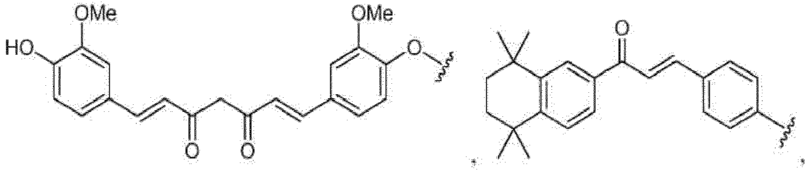 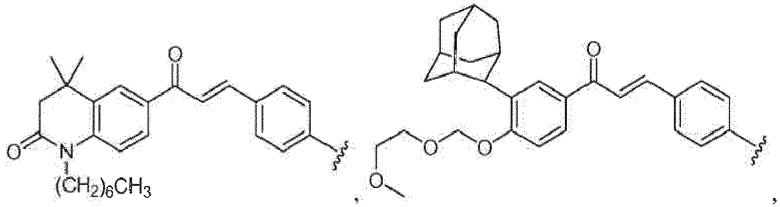 and 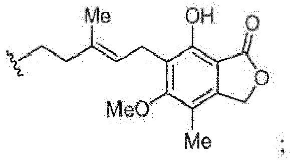 R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is each independently selected from the group consisting of -H, -D, -Cl, -F, -CN, OH, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
each g is independently 2, 3 or 4;
each h is independently 1, 2, 3 or 4;
m is 0, 1, 2, or 3; if m is more than 1, then L can be the same or different;
m1 is 0, 1, 2 or 3;
m2 is 0, 1, 2, 3, 4 or 5;
k is 0, 1, 2, or 3;
z is 1, 2, or 3;
each R₃ is independently H or C₁-C₆ alkyl, or both R₃ groups, when taken together with the nitrogen to which they are attached, can form a heterocycle;
each R₄ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, 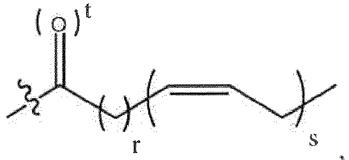 or 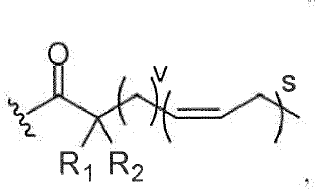 with the proviso that there is at least one 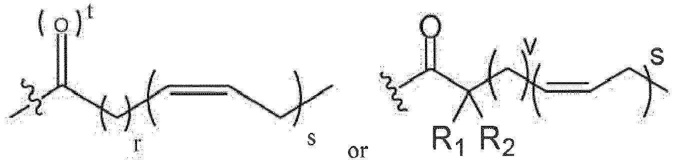 in the compound;
each r is independently 2, 3, or 7;
each s is independently 3, 5, or 6;
each t is independently 0 or 1;
each v is independently 1, 2, or 6;
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, phenyl or straight or branched C₁-C₄ alkyl optionally substituted with OH, or halogen;

In another aspect, compounds of Formula VI are described: 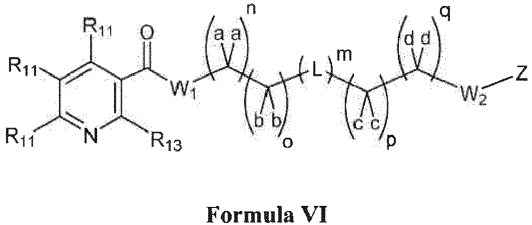 and pharmaceutically acceptable salts, hydrates, solvates, prodrugs, enantiomers and stercoisomers thereof;
wherein
W₁ and W₂ arc cach independently null, O, S, NH, NR, or W₁ and W₂ can be taken together can form an imidazolidine or piperazine group, with the proviso that W₁ and W₂ can not be O simultaneously;
R₁₁ is independently H, -OH, -OC(O)-R, -O-aryl, -aryl, -heteroaryl, or -heterocyclic;
R₁₃ is independently H, C₁-C₃alkyl, -OH, -OC(O)-R, or halogen;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a heteroaryl, 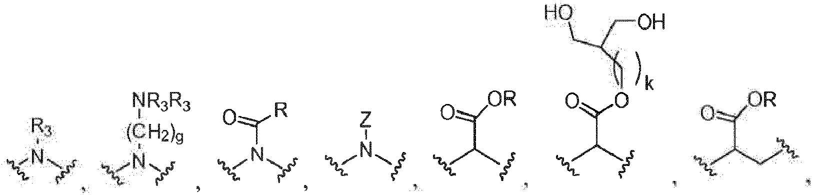 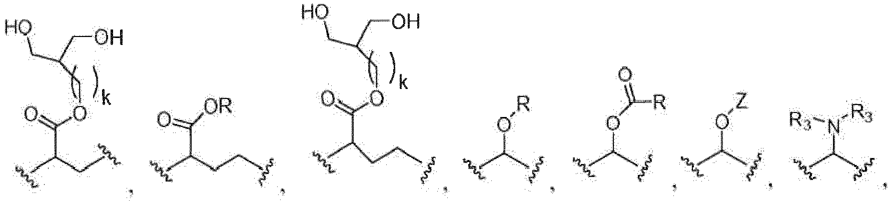 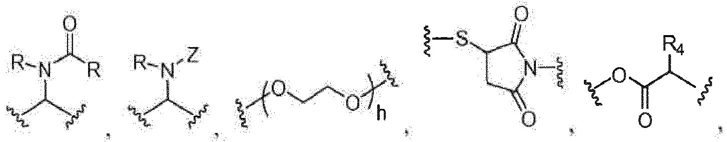 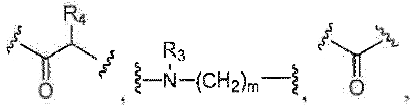 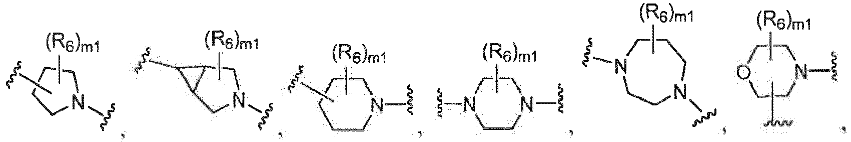 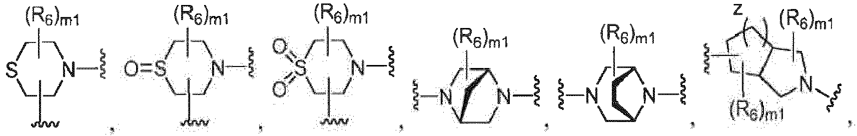 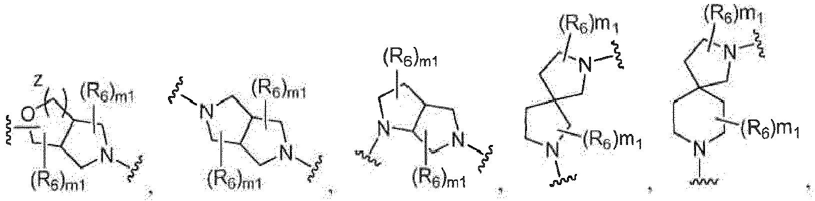 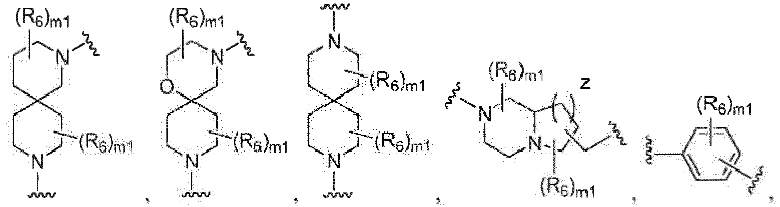 wherein the representation of L is not limited directionally left to right as is depicted, rather either the left side or the right side of L can be bound to the W₁ side of the compound of Formula I;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
each g is independently 2, 3 or 4;
each h is independently 1, 2, 3 or 4;
m is 0, 1, 2, or 3; if m is more than 1, then L can be the same or different;
m1 is 0, 1, 2 or 3;
k is 0, 1, 2, or 3;
z is 1, 2, or 3;
each R₃ is independently H or C₁-C₆ alkyl, or both R₃ groups, when taken together with the nitrogen to which they are attached, can form a heterocycle;
each R₄ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, 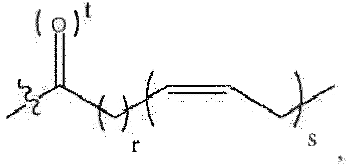 or 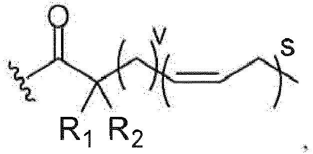 with the proviso that there is at least one 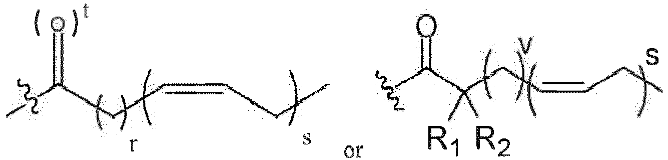 in the compound;
cach r is independently 2, 3, or 7;
each s is independently 3, 5, or 6;
each t is independently 0 or 1;
cach v is independently 1, 2, or 6;
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, phenyl or straight or branched C₁-C₄ alkyl optionally substituted with OH, or halogen.

Another aspect relates to compounds of **Formula VII:** 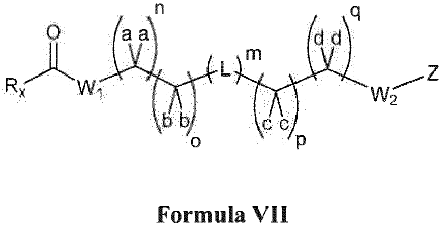 and pharmaceutically acceptable salts, hydrates, solvates, prodrugs, enantiomers and stereoisomers thereof;
wherein
Rₓ is independently 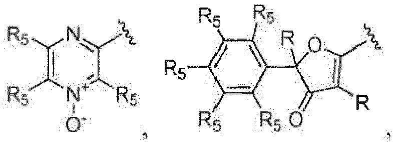 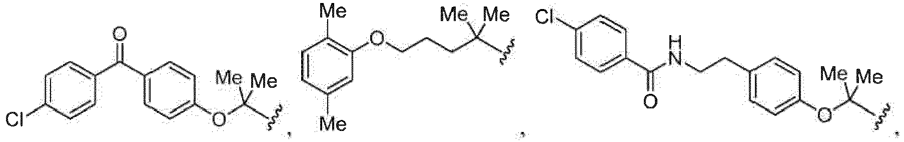 or 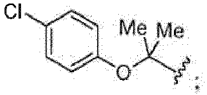 W₁ and W₂ are each independently null, O, S, NH, NR, or W₁ and W₂ can be taken together can form an imidazolidine or piperazine group, with the proviso that W₁ and W₂ can not be O simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -(C₁-C₆alkyl)-, -(C₃-C₆cycloalkyl)-, a heterocycle, a heteroaryl, 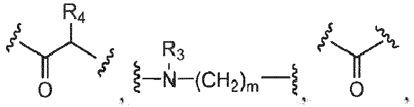 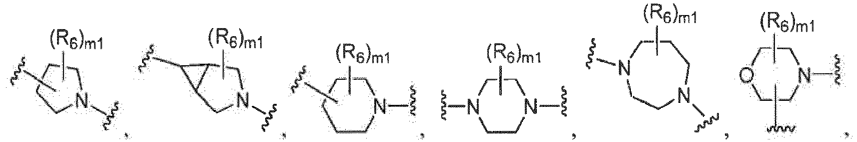 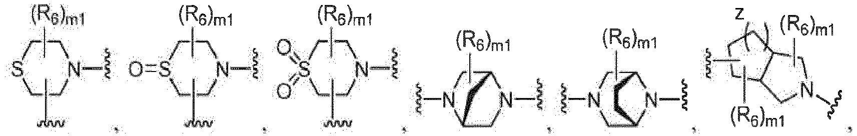 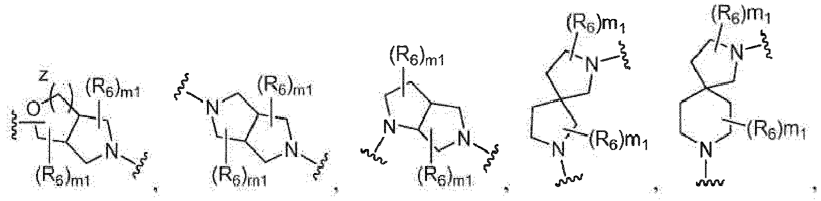 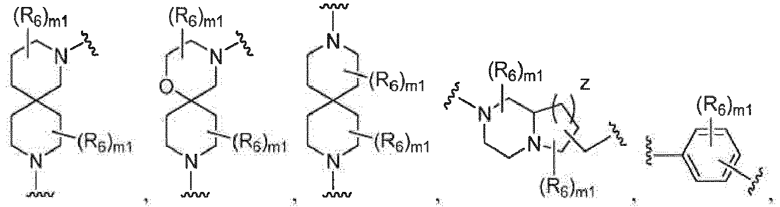 wherein the representation of L is not limited directionally left to right as is depicted, rather either the left side or the right side of L can be bound to the W₁ side of the compound of Formula I;
R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₅ is each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
each g is independently 2, 3 or 4;
cach h is independently 1, 2, 3 or 4;
m is 0, 1, 2, or 3; if m is more than 1, then L can be the same or different;
m1 is 0, 1, 2 or 3;
k is 0, 1, 2, or 3;
z is 1, 2, or 3;
each R₃ is independently H or C₁-C₆ alkyl, or both R₃ groups, when taken together with the nitrogen to which they are attached, can form a heterocycle;
cach R₄ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, 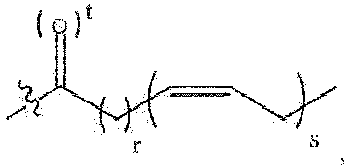 or 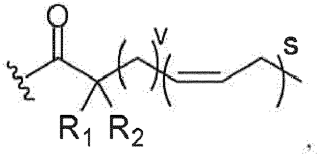 with the proviso that there is at least one 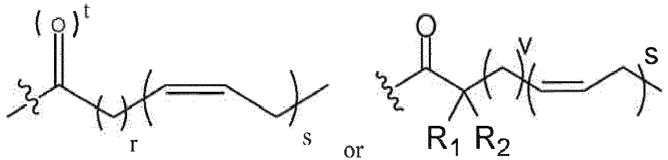 in the compound;
each r is independently 2, 3, or 7;
cach s is independently 3, 5, or 6;
each t is independently 0 or 1;
each v is independently 1, 2, or 6;
R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, phenyl or straight or branched C₁-C₄ alkyl optionally substituted with OH, or halogen.

In **Formula I, II, II', III, IV, V, VI** and **VII,** any one or more of H may be substituted with a deuterium. It is also understood in **Formula I, II, II', III, IV, V, VI** and **VII** that a methyl substituent can be substituted with a C₁-C₆ alkyl.

Also described are pharmaceutical formulations comprising at least one fatty acid derivative.

Also described herein are methods of treating a disease susceptible to treatment with a fatty acid derivative in a patient in need thereof by administering to the patient an effective amount of a fatty acid derivative.

Also described herein are methods of treating metabolic diseases by administering to a patient in need thereof an effective amount of a fatty acid derivative.

The invention also includes pharmaceutical compositions that comprise an effective amount of a fatty acid derivative and a pharmaceutically acceptable carrier. The compositions are useful for treating or preventing a metabolic disease. The invention includes a fatty acid derivative provided as a pharmaceutically acceptable prodrug, a hydrate, a salt, such as a pharmaceutically acceptable salt, enantiomer, stereoisomer, or mixtures thereof.

The details of the invention are set forth in the accompanying description below. Although methods and materials similar or equivalent to those described herein can be used in the practice or testing of the present invention, illustrative methods and materials arc now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs. All patents and publications cited in this specification arc incorporated herein by reference in their entireties.

### BRIEF DESCRIPTION OF THE FIGURES

Figure 1 is a graphic representation of the data showing the comparative effects of compounds I-8, II-1, and compound A on PCSK9.
Figure 2 is a graphic representation of the data showing the effects of compound I-8 and a combination of EPA and niacin on PCSK9 in HepG2 assay incubated with atorvastatin.
Figure 3 is a graphic representation of the data showing the effects of compound I-8 on the plasma triglyceride level of the Zucker fa/fa Rat model of Dyslipidemia.
Figure 4 is a graphic representation of the data showing the effects of a combination of compound I-8 and atorvastatin on plasma cholesterol and other lipids in ApoE3Leiden mice after 2 weeks of treatment.
Figure 5 is a graphic representation of the data showing the effects of a combination of compound I-8 and atorvastatin on plasma cholesterol and other lipids in ApoE3Leiden mice after 4 weeks of treatment.
Figure 6 is a graphic representation of the data showing the effects of a combination of compound I-8 and atorvastatin on plasma triglycerides and other lipids in ApoE3Leiden mice after 4 weeks of treatment.
Figure 7 is a graphic representation of the data showing the effects of administering compound 1-8 on ApoE3Leiden mice liver weight

### DETAILED DESCRIPTION OF THE INVENTION

Metabolic diseases are a wide variety of medical disorders that interfere with a subject's metabolism. Metabolism is the process a subject's body uses to transform food into energy. Metabolism in a subject with a metabolic disease is disrupted in some way. The fatty acid derivatives possess the ability to treat or prevent metabolic diseases. The fatty acid derivatives have been designed to bring together omega-3 fatty acids and an aryl, a heteroaryl or a heterocycle into a single fatty acid bioactive derivative. In some instances, the heteroaryl group can also be niacin or any other derivatives thereof. The activity of the fatty acid derivatives is substantially greater than the sum of the individual components of the fatty acid bioactive derivative, suggesting that the activity induced by the fatty acid derivatives is synergistic. Based on this information, it was conceived that the present fatty acid derivatives could be effective in lowering the production of PCSK9 in in vitro cell assays. In addition, these fatty acid derivatives could also lower the serum PCSK9 level when dosed in vivo. As a result of these activities, fatty acid derivatives can be used as a monotherapy or as a combination therapy with a statin or other cholesterol lowering agent to effectively treat hypercholesterolemia, dyslipidemia or metabolic disease.

### DEFINITIONS

The following definitions are used in connection with the fatty acid derivatives:

The term "fatty acid derivatives" includes any and all possible isomers, stereoisomers, enantiomers, diastereomers, tautomers, pharmaceutically acceptable salts, hydrates, solvates, and prodrugs of the fatty acid derivatives described herein.

The articles "a" and "an" are used in this disclosure to refer to one or more than one (*i.e*., to at least one) of the grammatical object of the article. By way of example, "an element" means one element or more than one element.

The term "and/or" is used in this disclosure to mean either "and" or "or" unless indicated otherwise.

Unless otherwise specifically defined, the term "aryl" refers to cyclic, aromatic hydrocarbon groups that have 1 to 2 aromatic rings, including monocyclic or bicyclic groups such as phenyl, biphenyl or naphthyl. Where containing two aromatic rings (bicyclic, *etc.*), the aromatic rings of the aryl group may be joined at a single point (*e.g.,* biphenyl), or fused (*e.g.,* naphthyl). The aryl group may be optionally substituted by one or more substituents, *e.g.,* 1 to 5 substituents, at any point of attachment. The substituents can themselves be optionally substituted.

"C₁-C₃ alkyl" refers to a straight or branched chain saturated hydrocarbon containing 1-3 carbon atoms. Examples of a C₁-C₃ alkyl group include, but are not limited to, methyl, ethyl, propyl and isopropyl.

C₁-C₄ alkyl" refers to a straight or branched chain saturated hydrocarbon containing 1-4 carbon atoms. Examples of a C₁-C₄ alkyl group include, but are not limited to, methyl, ethyl, propyl, butyl, isopropyl, isobutyl, *sec*-butyl and *tert*-butyl*.*

"C₁-C₅ alkyl" refers to a straight or branched chain saturated hydrocarbon containing 1-5 carbon atoms. Examples of a C₁-C₅ alkyl group include, but are not limited to, methyl, ethyl, propyl, butyl, pentyl, isopropyl, isobutyl, *sec*-butyl and *tert*-butyl, isopentyl and neopentyl.

"C₁-C₆ alkyl" refers to a straight or branched chain saturated hydrocarbon containing 1-6 carbon atoms. Examples of a C₁-C₆ alkyl group include, but are not limited to, methyl, ethyl, propyl, butyl, pentyl, hexyl, isopropyl, isobutyl, *sec*-butyl, *tert*-butyl, isopentyl, and neopentyl.

The term "cycloalkyl" refers to a cyclic hydrocarbon containing 3-6 carbon atoms. Examples of a cycloalkyl group include, but are not limited to, cyclopropyl, cyclobutyl, cyclopentyl and cyclohexyl. It is understood that any of the substitutable hydrogens on a cycloalkyl can be substituted with halogen, C₁-C₃ alkyl, hydroxyl, alkoxy and cyano groups.

The term "heterocycle" as used herein refers to a cyclic hydrocarbon containing 3-6 atoms wherein at least one of the atoms is an O, N, or S. Examples of heterocycles include, but are not limited to, aziridine, oxirane, thiirane, azetidine, oxetane, thietane, pyrrolidine, tetrahydrofuran, tetrahydrothiophene, piperidine, tetrahydropyran, thianc, imidazolidine, oxazolidine, thiazolidine, dioxolane, dithiolane, piperazine, oxazine, dithiane, and dioxane.

The term "heteroaryl" as used herein refers to a monocyclic or bicyclic ring structure having 5 to 12 ring atoms wherein one or more of the ring atoms is a heteroatom, e.g. N, O or S and wherein one or more rings of the bicyclic ring structure is aromatic. Some examples of heteroaryl are pyridyl, furyl, pyrrolyl, thienyl, thiazolyl, oxazolyl, imidazolyl, indolyl, tetrazolyl, benzofuryl, xanthenes and dihydroindole. It is understood that any of the substitutable hydrogens on a heteroaryl can be substituted with halogen, C₁-C₃ alkyl, hydroxyl, alkoxy and cyano groups.

The term "any one of the side chains of the naturally occurring amino acids" as used herein means a side chain of any one of the following amino acids: Isoleucine, Alanine, Leucine, Asparagine, Lysine, Aspartate, Methionine, Cysteine, Phenylalanine, Glutamate, Threonine, Glutamine, Tryptophan, Glycine, Valine, Proline, Arginine, Serine, Histidine, and Tyrosine.

The term "fatty acid" as used herein means an omega-3 fatty acid and fatty acids that are metabolized *in vivo* to omega-3 fatty acids. Non-limiting examples of fatty acids are *all*-*cis*-7,10,13-hexadecatrienoic acid, α-linolenic acid (ALA or *all-cis*-9,12,15-octadecatrienoic acid), stearidonic acid (STD or *all*-*cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis*-7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis*-6,9,12,15,18,21-tetracosenoic acid).

The term "niacin" as used herein means the molecule known as niacin and any derivative thereof.

The term "bioactive" or "bioactive molecule" as used herein means an aryl, including phenylor naphthyl, heteroaryl, or a heterocycle derivative which posseses biological activity.

A "subject" is a mammal, *e*.*g*., a human, mouse, rat, guinea pig, dog, cat, horse, cow, pig, or non-human primate, such as a monkey, chimpanzee, baboon or rhesus, and the terms "subject" and "patient" are used interchangeably herein.

The invention also includes pharmaceutical compositions comprising an effective amount of a fatty acid derivative of Formula 11', VI, or VII as described above and a pharmaceutically acceptable carrier. The invention includes a fatty acid niacin derivative provided as a pharmaceutically acceptable prodrug, hydrate, salt, such as a pharmaceutically acceptable salt, enantiomers, stereoisomers, or mixtures thereof.

Representative "pharmaceutically acceptable salts" include, *e.g.,* water-soluble and water-insoluble salts, such as the acetate, amsonate (4,4-diaminostilbene-2, 2 - disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, *N*-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "metabolic disease" as used herein refers to disorders, diseases and syndromes involving dyslipidemia, and the terms metabolic disorder, metabolic disease, and metabolic syndrome are used interchangeably herein.

An "effective amount" when used in connection with a fatty acid derivative is an amount effective for treating or preventing a metabolic disease.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body.

The term "treating", with regard to a subject, refers to improving at least one symptom of the subject's disorder. Treating can be curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a compound or pharmaceutically acceptable salt of the compound or a composition to a subject, or administering a prodrug derivative or analog of the compound or pharmaceutically acceptable salt of the compound or composition to the subject, which can form an equivalent amount of active compound within the subject's body.

The term "prodrug," as used in this disclosure, means a compound which is convertible *in vivo* by metabolic means (*e.g.,* by hydrolysis) to a fatty acid derivative.

The following abbreviations are used herein and have the indicated definitions: Boc and BOC are *tert*-butoxycarbonyl, Boc₂O is di-*tert*-butyl dicarbonate, BSA is bovine serum albumin, CDI is 1,1'-carbonyldiimidazole, DCC is *N*,*N'*-dicyclohexylcarbodiimide, DIEA is *N*,*N*-diisopropylethylamine, DMAP is 4-dimethylaminopyridine, DMEM is Dulbecco's Modified Eagle Medium, DMF is *N*,*N-*dimethylformamide, DOSS is sodium dioctyl sulfosuccinate, EDC and EDCI are 1-ethyl-3-(3-dimethylaminopropyl)carbodiimide hydrochloride, ELISA is enzyme-linked immunosorbent assay, EtOAc is ethyl acetate, FBS is fetal bovine serum, h is hour, HATU is 2-(7-aza-1*H*-benzotriazole-1-yl)-1,1,3,3-tetramethyluronium hexafluorophosphate, HIV is human immunodeficiency virus, HPMC is hydroxypropyl methylcellulose, oxone is potassium peroxymonosulfate, Pd/C is palladium on carbon, TFA is trifluoroacetic acid, TGPS is tocopherol propylene glycol succinate, and THF is tetrahydrofuran.

### COMPOUNDS

Accordingly in one aspect, the present invention provides a method of using a fatty acid bioactive derivative which comprises a fatty acid and an aryl, a heteroaryl or a heterocycle covalently linked, wherein the fatty acid is selected from the group consisting of omega-3 fatty acids and fatty acids that are metabolized *in vivo* to omega-3 fatty acids, and the derivative is capable of hydrolysis to produce free fatty acid and free aryl, heteroaryl or heterocycle.

In some embodiments, the fatty acid is selected from the group consisting of *all-cis*-7,10,13-hexadecatrienoic acid, α-linolenic acid, stearidonic acid, eicosatrienoic acid, eicosatetraenoic acid, eicosapentaenoic acid (EPA), docosapentaenoic acid, docosahexaenoic acid (DHA), tetracosapentaenoic acid and tetracosahexacnoic acid. In other embodiments, the fatty acid is selected from eicosapentaenoic acid and docosahexaenoic acid. In some embodiments, the hydrolysis is enzymatic.

In another aspect, the present invention also provides fatty acid bioactive derivatives according to Formulae: 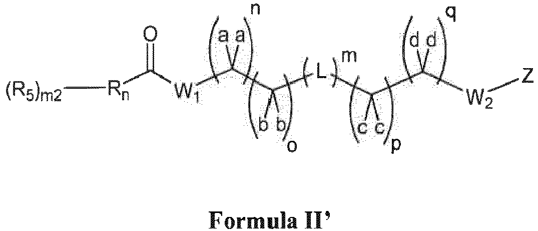 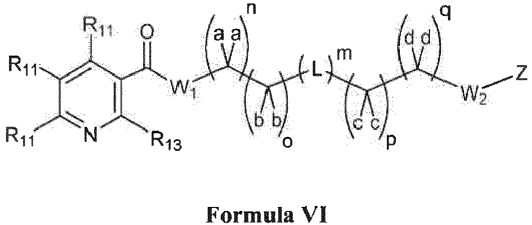 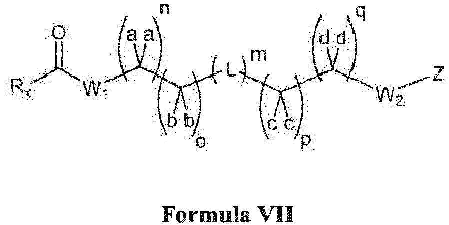 and pharmaceutically acceptable salts, hydrates, solvates, prodrugs, enantiomers, and stereoisomers thereof;
wherein
Rₙ, Rₓ, R₁, R₂, R₃, R₄, R₅, R₆, R₁₁, R, W₁, W₂, L, a, c, b, d, e, g, h, m, m1, m2, n, o, p, q, Z, r, s, t, and v are as defined above for formulae **II', VI,** and **VII.**
with the proviso that there is at least one 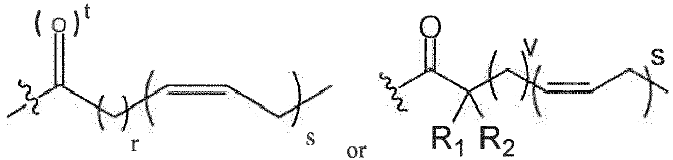 in the compound.

In some embodiments, Rₓ is

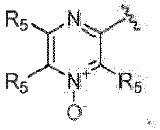

In some embodiments, Rₓ is

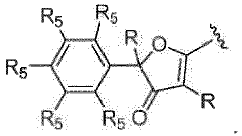

In some embodiments, Rₓ is

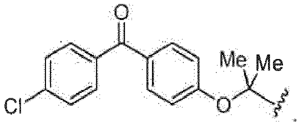

In some embodiments, Rₓ is

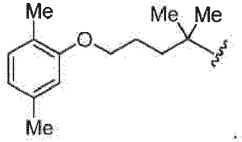

In some embodiments, Rₓ is

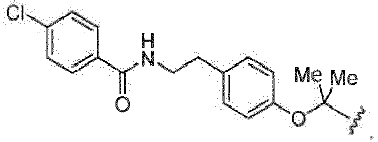

In some embodiments, Rₓ is

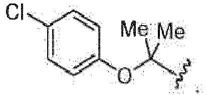

In some embodiments, Rₙ is phenyl.

In some embodiments, one Z is 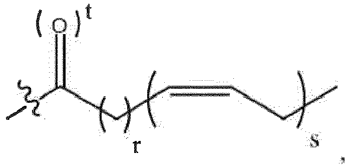 and r is 2.

In some embodiments, one Z is and r is 3.

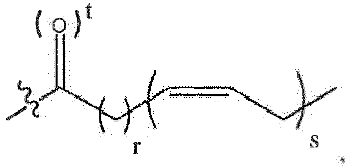

In some embodiments, one Z is 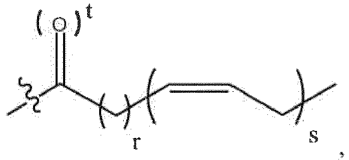 and r is 7.

In other embodiments, one Z is 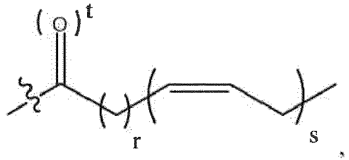 and s is 3.

In some embodiments, one Z is 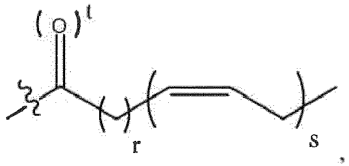 and s is 5.

In some embodiments, one Z is 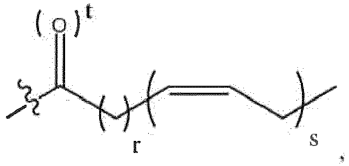 and s is 6.

In some embodiments, one Z is 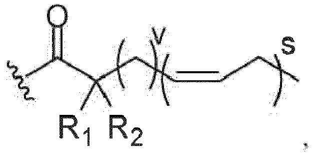 and v is 1.

In other embodiments, one Z is 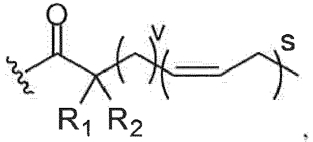 and v is 2.

In some embodiments, one Z is 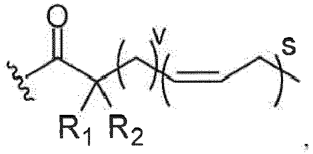 and v is 6.

In some embodiments, one Z is 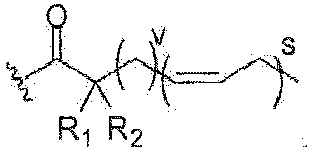 and s is 3.

In some embodiments, one Z is 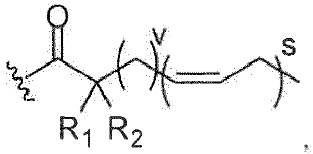 and s is 5.

In other embodiments, one Z is 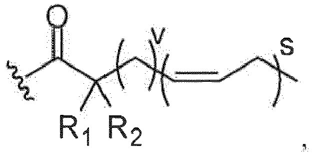 and s is 6.

In some embodiments, W₁ is NH.

In some embodiments, W₂ is NH.

In some embodiments, W₁ is O.

In some embodiments, W₂ is O.

In some embodiments, W₁ is null.

In some embodiments, W₂ is null.

In some embodiments, W₁ and W₂ are each NH.

In some embodiments, W₁ and W₂ are each null.

In some embodiments, W₁ is O and W₂ is NH.

In some embodiments, W₁ and W₂ are each NR, and R is CH₃.

In some embodiments, m is 0.

In other embodiments, m is 1.

In other embodiments, m is 2.

In some embodiments, L is -S- or -S-S-.

In some embodiments, L is -O-.

In some embodiments, L is -C(O)-.

In some embodiments, L is heteroaryl.

In some embodiments, L is heterocycle.

In some embodiments, L is

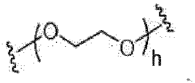

In some embodiments, L is

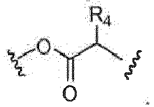

In some embodiments, L is

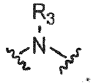

In some embodiments, L is

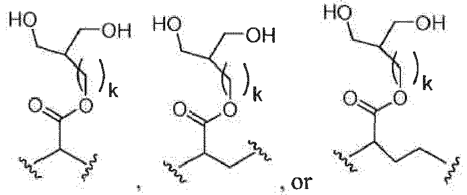

In some embodiments, L is

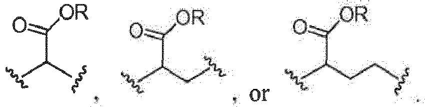

In some embodiments, L is

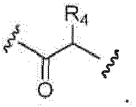

In some embodiments, L is 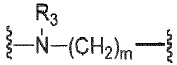 wherein m is 2.

In some embodiments, L is 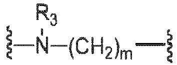 wherein m is 3.

In some embodiments, L is

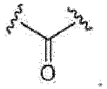

In some embodiments, L is

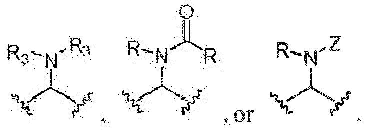

In some embodiments, L is

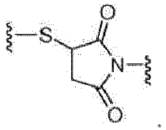

In some embodiments, L is

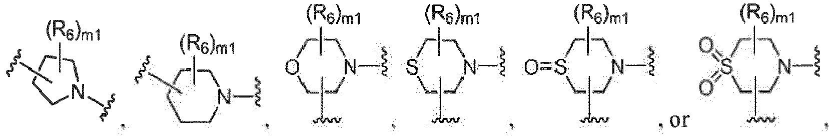

In some embodiments, L is

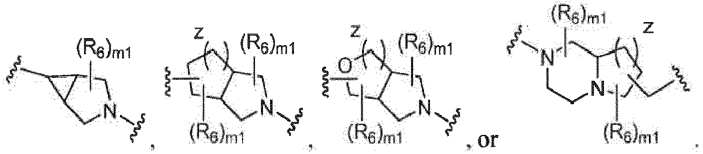

In some embodiments, L is

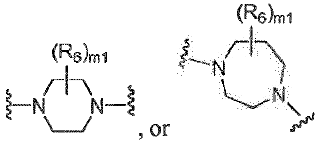

In some embodiments, L is

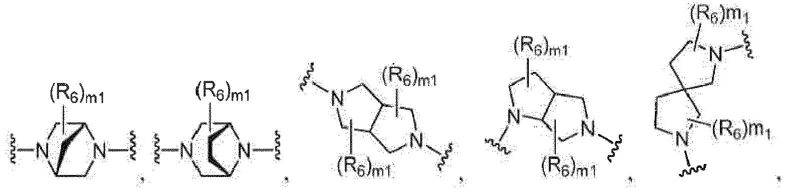

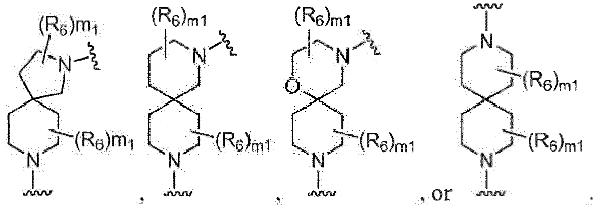

In other embodiments, one of n, o, p, and q is 1.

In some embodiments, two of n, o, p, and q are each 1.

In other embodiments, three of n, o, p, and q are each 1.

In some embodiments n, o, p, and q are each 1.

In some embodiments, one d is C(O)OR.

In some embodiments, r is 2 and s is 6.

In some embodiments, r is 3 and s is 5.

In some embodiments, t is 1.

In some embodiments, W₁ and W₂ are each NH, m is 0, n, and o are each 1, and p and q are each 0.

In some embodiments, W₁ and W₂ are each NH, m is 1, n, o, p, and q are each 1, and L is O.

In some embodiments, W₁ and W₂ are each NH, m is 1, n, o, p, and q are each 1, and L is

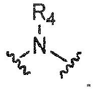

In some embodiments, W₁ and W₂ are each NH, m is 1, n, o, p, and q are each 1, and L is -S-S-.

In some embodiments, W₁ and W₂ are each NH, m is 1, n and o are each 0, p and q are each 1, and L is

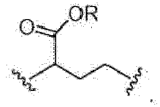

In some embodiments, W₁ and W₂ are each NH, m is 1, k is O, n and o are each 0, p and q are each 1, and L is

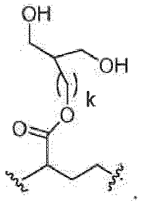

In some embodiments, W₁ and W₂ are each NH, m is 1, n and o are each 1, p and q are each 0, and L is

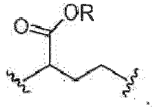

In some embodiments, W₁ and W₂ are each NH, m is 1, k is 0, n is 1, o, p and q are each 0, and L is

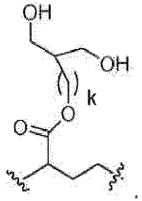

In some embodiments, W₁ and W₂ are each NH, m is 1, n, o, and p are each 0, and q is 1, and L is

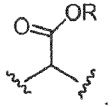

In some embodiments, W₁ and W₂ are each NH, m is 1, k is 1, n, o, and p are each 0, and q is 1, and L is

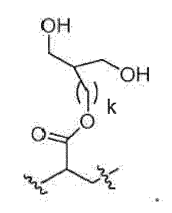

In some embodiments, W₁ and W₂ are each NH, m is 1, n is 1, and o, p, and q are each 0, and L is

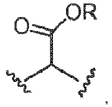

In some embodiments, W₁ and W₂ are each NH, m is 1, k is 1, o, p, and q are each 0, and L is

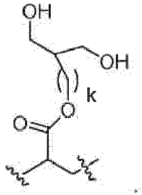

In some embodiments, W₁ and W₂ are each NH, m is 1, n, o, p, and q are each 1, and L is

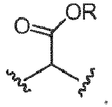

In some embodiments, W₁ and W₂ are each NH, m is 1, n, o, p, and q are each 1, and L is

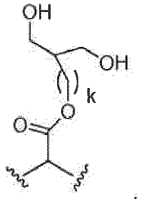

In some embodiments, W₁ and W₂ are each NH, m is 0, k is 1, o and p are each 1, and q is 0.

In some embodiments, W₁ and W₂ are each NH, m is 0, n, o, p, and q are each 1.

In some embodiments, W₁ and W₂ are each NH, m is 0, n and o are each 1, p and q are each 0, and each a is CH₃.

In some embodiments, W₁ and W₂ are each NH, m is 0, n and o arc each 1, p and q are each 0, and each b is CH₃.

In some embodiments, W₁ and W₂ are each NH, m is 1, n, o, p, and q are each 1, R₃ is H, and L is

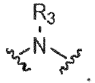

In some embodiments, W₁ and W₂ are each NH, m is 1, n, p and q are each 1, and o is 2, R₃ is H, and L is

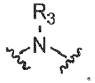

In some embodiments, W₁ and W₂ are each NH, m is 1, n, o, p are each 1, and q is 2, and L is

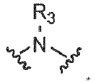

In some embodiments, W₁ and W₂ are each NH, m is 1, n, o, p, and q are each 1, and L is

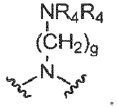

In some embodiments, W₁ and W₂ are each NH, m is 1, n and p are each 1, and o and q are each 0, and L is -C(O)-.

In some embodiments, W₁ and W₂ are each NH, m is 1, n and p are each 1, and o, and q are each 0, and L is

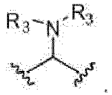

In some embodiments, W₁ and W₂ are each NH, m is 1, n, o, p, q are each 1, and L is

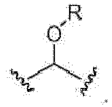

In some embodiments, W₁ and W₂ are each NH, m is 1, n, o, p , and q are each 1, h is 1, and L is

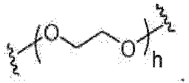

In some embodiments, W₁ and W₂ are each NH, m is 1, n, o, p , and q are each 1, and L is-S-.

In some embodiments, W₁ and W₂ are each NH, m is 1, n, o, p are each 0, q is 1, one d is -CH₃, and L is

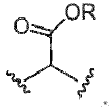

In some embodiments, W₁ and W₂ are each NH, m is 2, n, o, p, and q are each 0, one L is 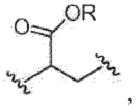 and one L is

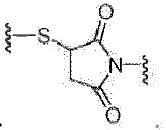

In some embodiments, m is 0, n, o, p, and q are each 0, and W₁ and W₂ are taken together to form an optionally substituted piperazine group.

In some embodiments, m is 1, n, o, p, and q are each 0, W₁ and W₂ are each null, and L is

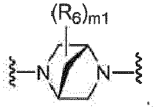

In some embodiments, m is 1, n and p are each 1, o and q are each 0, W₁ and W₂ are each NH, and L is C₃-C₆ cycloalkyl.

In some embodiments, m is 1, n is 1, o, p, and q are each 0, W₁ and W₂ are each NH, and L is C₃-C₆ cycloalkyl.

In some embodiments, m is 1, n, o, p, are each 0, q is 1, W₁ and W₂ are each NH, and L is C₃-C₆ cycloalkyl.

In some embodiments, m is 1, n, o, p, and q are each 0, W₁ is NH, W₂ is null, and L is

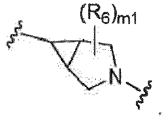

In some embodiments, m is 1, n o, p, and q are each 0, W₁ is null, W₂ is NH, and L is

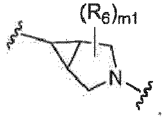

In some embodiments, m is 1, n o, p, and q are each 0, W₁ is NH, W₂ is null, and L is

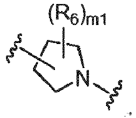

In some embodiments, m is 1, n o, p, and q are each 0, W₁ is null, W₂ is NH, and L is

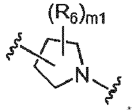

In some embodiments, m is 1, n is 1, o, p, and q are each 0, W₁ is NH, W₂ is null, and L is

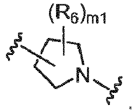

In some embodiments, m is 1, n, o, p, are each 0, q is 1, W₁ is null, W₂ is NH, and L is

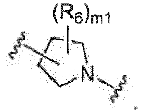

In some embodiments, m is 1, n, o, p, and q are each 0, W₁ is NH, W₂ is null, and L is

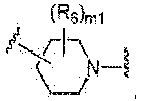

In some embodiments, m is 1, n, o, p, and q are each 0, W₁ is null, W₂ is NH, and L is

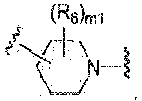

In some embodiments, m is 1, n is 1, o, p, and q are each 0, W₁ is NH, W₂ is null, and L is

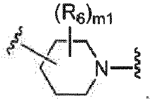

In some embodiments, m is 1, n, o, p, are each 0, q is 1, W₁ is null, W₂ is NH, and L is

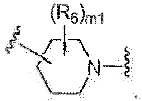

In some embodiments, m is 1, n is 1, o, p, and q are each 0, W₁ is NH, W₂ is null, and L is

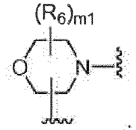

In some embodiments, m is 1, n, o, p, are each 0, q is 1, W₁ is null, W₂ is NH, and L is

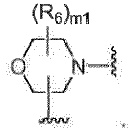

In some embodiments, m is 1, n, o, p, q are each 0, W₁ and W₂ is null, and L is

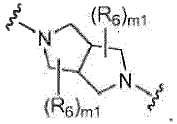

In some embodiments, m is 1, n, o, p, q are each 0, W₁ and W₂ is null, and L is

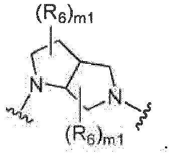

In some embodiments, m is 1, n, o, p, q arc each 0, W₁ is NH, W₂ is null, and L is

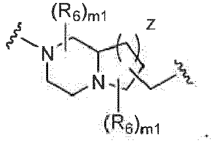

In some embodiments, m is 1, n, o, p, q are each 0, W₁ is null, W₂ is NH, and L is

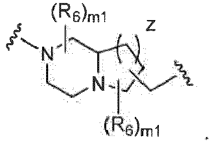

In some embodiments, m is 1, n, o, p, are each 0, q is 1, W₁ and W₂ are each and NH, is null, L is

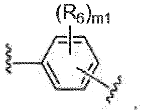

In some embodiments, m is 1, n, o, p, are each 0, q is 1, W₁ and W₂ are each NH, is null, and L is a heteroaryl.

In some of the foregoing embodiments, r is 2, s is 6 and t is 1.

In some of the foregoing embodiments, r is 3, s is 5 and t is 1.

In Formula I, II, II', **III**, IV, V, VI and VII, any one or more of H may be substituted with a deuterium. It is also understood in Formula I, II, **II**', **III**, IV, V, **VI** and VII that a methyl substituent can be substituted with a C₁-C₆ alkyl.

In other illustrative embodiments, compounds of Formula I, **II**, **II'**, **III**, IV, V, VI and VII used in the treatment of metabolic diseases described herein are as set forth below: 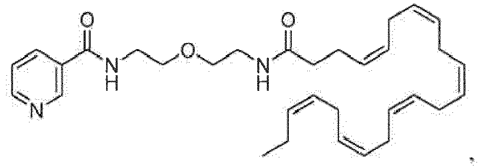
N-(2-(2-((4Z,7Z,10Z,13Z,16Z,19Z)-docosa-4,7,10,13,16,19-hexaenamido)ethoxy)ethyl)nicotinamide **(I-1)** 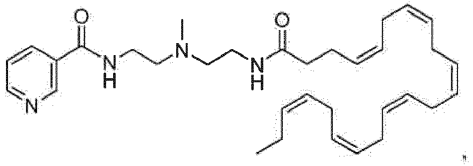
N-(2-((2-((4Z,7Z,10Z,13Z,16Z,19Z)-docosa-4,7,10,13,16,19-hexaenamido)ethyl)(methyl)amino)ethyl)nicotinamide **(I-2)** 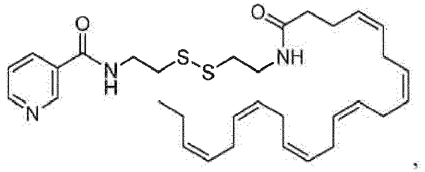
N-(2-((2-((4Z,7Z,10Z,13Z,16Z,19Z)-docosa-4,7,10,13,16,19-hexaenamido)ethyl)disulfanyl)ethyl)nicotinamide **(I-3)** 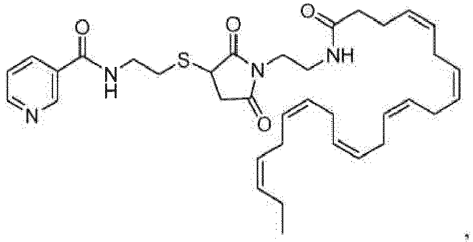
N-(2-((1-(2-((4Z,7Z,10Z,13Z,16Z,19Z)-docosa-4,7,10,13,16,19-hexaenamido)ethyl)-2,5-dioxopyrrolidin-3-yl)thio)ethyl)nicotinamide **(I-4)** 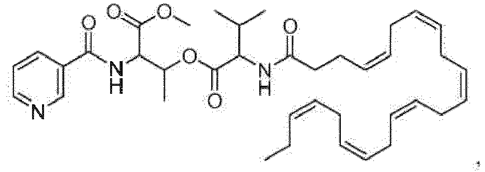
4-methoxy-3-(nicotinamido)-4-oxobutan-2-yl 2-((4Z,7Z,10Z,13Z,16Z,19Z)-docosa-4,7,10,13,16,19-hexaenamido)-3-methylbutanoate **(I-5)** 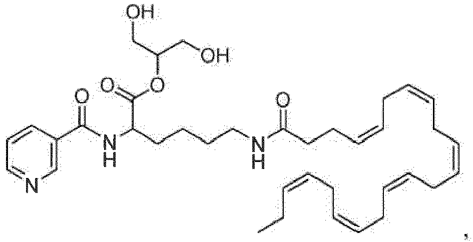
1,3-dihydroxypropan-2-yl 6-((4Z,7Z,10Z,13Z,16Z,19Z)-docosa-4,7,10,13,16,19-hexaenamido)-2-(nicotinamido)hexanoate **(I-6)** 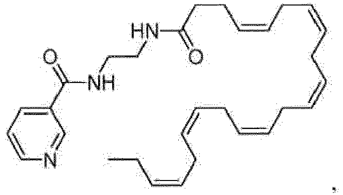
N-(2-((4Z,7Z,10Z,13Z,16Z,19Z)-docosa-4,7,10,13,16,19-hexaenamido)ethyl)nicotinamide (1-7) 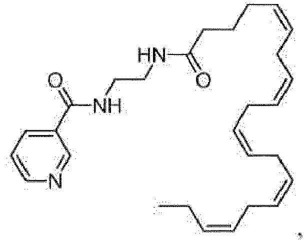
N-(2-((5Z,8Z,11Z,14Z,17Z)-icosa-5,8,11,14,17-pentaenamido)ethyl)nicotinamide **(I-8)** 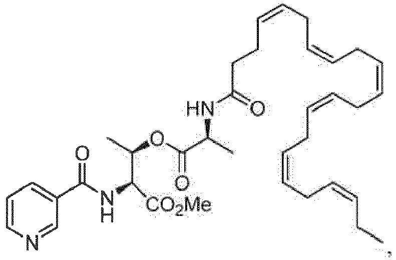
(2S,3R)-methyl 3-(((S)-2-((4Z,7Z,10Z,13Z,16Z,19Z)-docosa-4,7,10,13,16,19-hexaenamido)propanoyl)oxy)-2-(nicotinamido)butanoate **(I-9)** 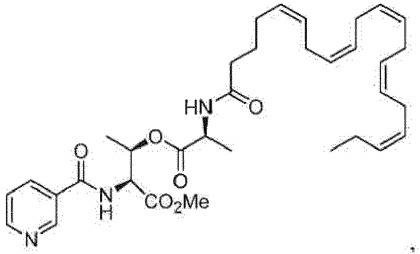
(2S,3R)-methyl 3-(((S)-2-((5Z,8Z,11Z,14Z,17Z)-icosa-5,8,11,14,17-pentaenamido)propanoyl)oxy)-2-(nicotinamido)butanoate **(I-10)** 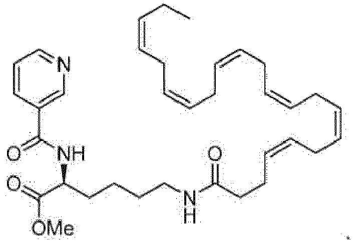
(S)-methyl 6-((4Z,7Z,10Z,13Z,16Z,19Z)-docosa-4,7,10,13,16,19-hexaenamido)-2-(nicotinamido)hexanoate **(I-11)** 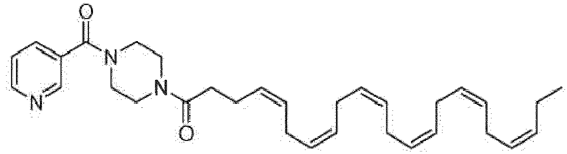
(4Z,7Z,10Z,13Z,16Z,19Z)-1-(4-nicotinoylpiperazin-1-yl)docosa-4,7,10,13,16,19-hexaen-1-one **(I-12)** 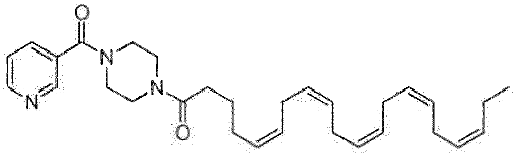
(5Z,8Z,11Z,14Z,17Z)-1-(4-nicotinoylpiperazin-1-yl)icosa-5,8,11,14,17-pentaen-1-one **(I-13)** 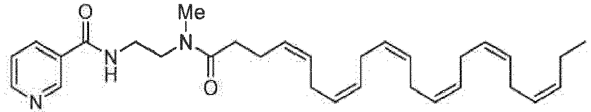
N-(2-((4Z,7Z,10Z,13Z,16Z,19Z)-N-methyldocosa-4,7,10,13,16,19-hexaenamido)ethyl)nicotinamide **(I-14)** 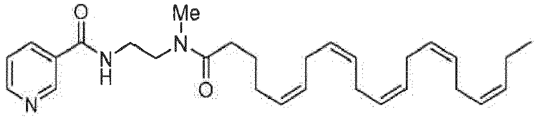
N-(2-((5Z,8Z,11Z,14Z,17Z)-N-methylicosa-5,8,11,14,17-pentaenamido)ethyl)nicotinamide **(I-15)** 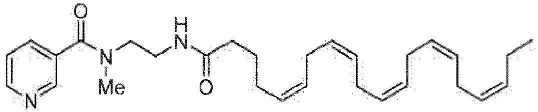
N-(2-((5Z,8Z,11Z,14Z,17Z)-icosa-5,8,11,14,17-pentaenamido)ethyl)-N-methylnicotinamide (**I**-**16**) 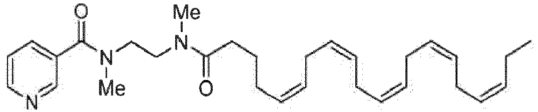
N-methyl-N-(2-((5Z,8Z,11Z,14Z,17Z)-N-methylicosa-5,8,11,14,17-pentaenamido)ethyl)nicotinamide **(I-17)** 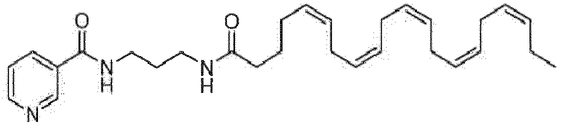
N-(3-((5Z,8Z,11Z,14Z,17Z)-icosa-5,8,11,14,17-pentaenamido)propyl)nicotinamide **(I-18)** 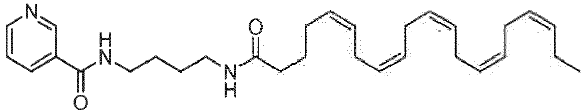
N-(4-((5Z,8Z,11Z,14Z,17Z)-icosa-5,8,11,14,17-pentaenamido)butyl)nicotinamide **(I-19)** 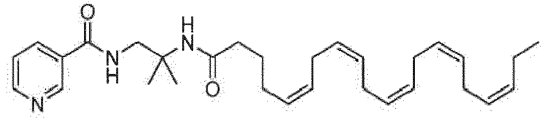
N-(2-((5Z,8Z,11Z,14Z,17Z)-icosa-5,8,11,14,17-pentaenamido)-2-methylpropyl)nicotinamide **(I-20)** 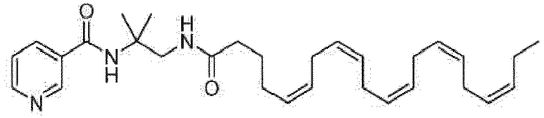
N-(1-((5Z,8Z,11Z,14Z,17Z)-icosa-5,8,11,14,17-pentaenamido)-2-methylpropan-2-yl)nicotinamide **(I-21)** 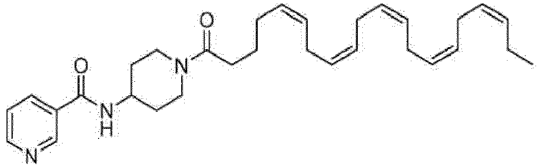
N-(1-((5Z,8Z,11Z,14Z,17Z)-icosa-5,8,11,14,17-pentaenoyl)piperidin-4-yl)nicotinamide **(I-22)** 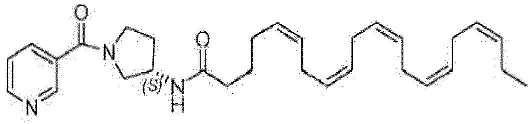
(5Z,8Z,11Z,14Z,17Z)-N-((S)-1-nicotinoylpyrrolidin-3-yl)icosa-5,8,11,14,17-pentaenamide **(I-23)** 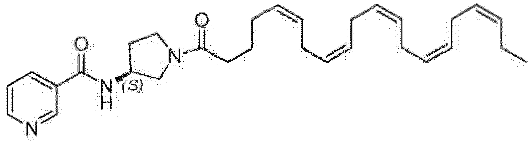
N-((S)-1-((5Z,8Z,11Z,14Z,17Z)-icosa-5,8,11,14,17-pentaenoyl)pyrrolidin-3-yl)nicotinamide **(I-24)** 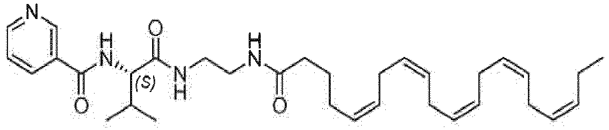
N-((S)-1-((2-((5Z,8Z,11Z,14Z,17Z)-icosa-5,8,11,14,17-pentaenamido)ethyl)amino)-3-methyl-1-oxobutan-2-yl)nicotinamide **(I-25)** 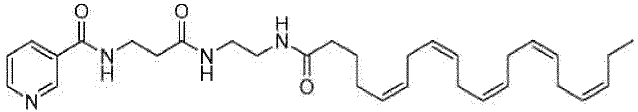
N-(3-((2-((5Z,8Z,11Z,14Z,17Z)-icosa-5,8,11,14,17-pentaenamido)ethyl)amino)-3-oxopropyl)nicotinamide **(I-26)** 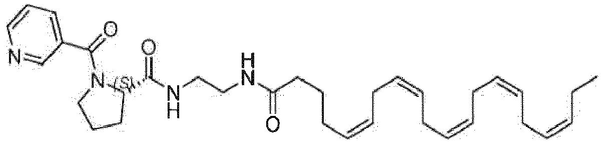
(S)-N-(2-((5Z,8Z,11Z,14Z,17Z)-icosa-5,8,11,14,17-pentaenamido)ethyl)-1-nicotinoylpyrrolidine-2-carboxamide **(I-27)** 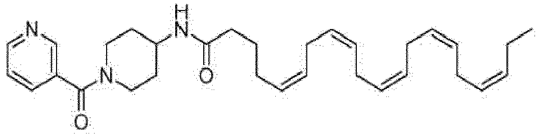
(5Z,8Z,11Z,14Z,17Z)-N-(1-nicotinoylpiperidin-4-yl)icosa-5,8,11,14,17-pentaenamide **(I-28)** 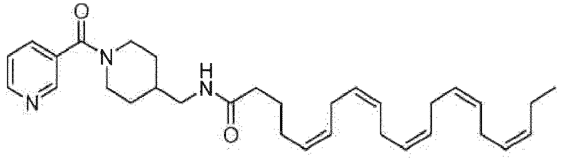
(5Z,8Z,11Z,14Z,17Z)-N-((1-nicotinoylpiperidin-4-yl)methyl)icosa-5,8,11,14,17-pentaenamide **(I-29)** 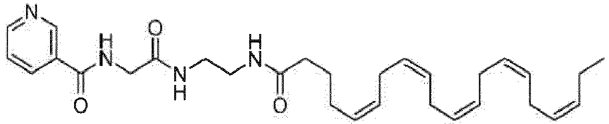
N-(2-((2-((5Z,8Z,11Z,14Z,17Z)-icosa-5,8,11,14,17-pentaenamido)ethyl)amino)-2-oxoethyl)nicotinamide **(I-30)** 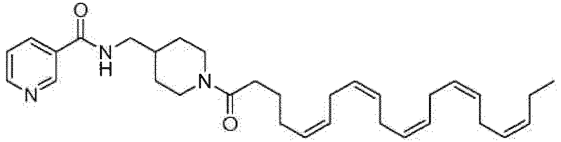
N-((1-((5Z,8Z,11Z,14Z,17Z)-icosa-5,8,11,14,17-pentaenoyl)piperidin-4-yl)methyl)nicotinamide (**I**-**31**) 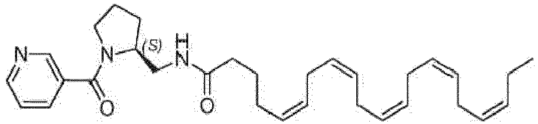
(5Z,8Z,11Z,14Z,17Z)-N-(((S)-1-nicotinoylpyrrolidin-2-yl)methyl)icosa-5,8,11,14,17-pentaenamide **(I-32)** 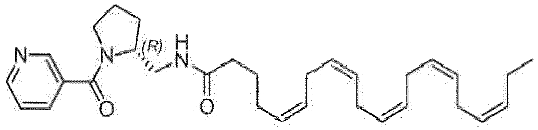
(5Z,8Z,11Z,14Z,17Z)-N-(((R)-1-nicotinoylpyrrolidin-2-yl)methyl)icosa-5,8,11,14,17-pentaenamide **(I-33)** 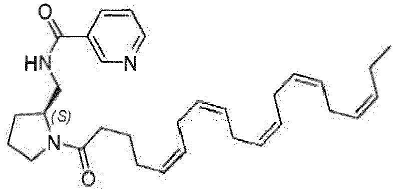
N-(((S)-1-((5Z,8Z,11Z,14Z,17Z)-icosa-5,8,11,14,17-pentaenoyl)pyrrolidin-2-yl)methyl)nicotinamide **(I-34)** 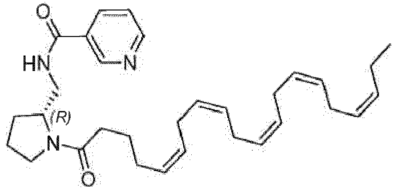
N-(((R)-1-((5Z,8Z,11Z,14Z,17Z)-icosa-5,8,11,14,17-pentaenoyl)pyrrolidin-2-yl)methyl)nicotinamide **(I-35)** 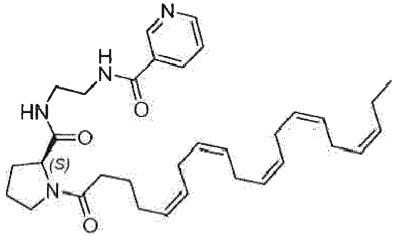
N-(2-((S)-1-((5Z,8Z,11Z,14Z,17Z)-icosa-5,8,11,14,17-pentaenoyl)pyrrolidine-2-carboxamido)ethyl)nicotinamide **(I-36)** 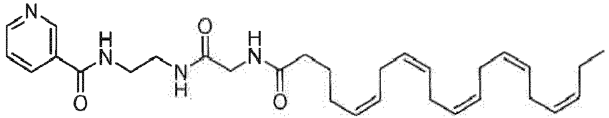
N-(2-(2-((5Z,8Z,11Z,14Z,17Z)-icosa-5,8,11,14,17-pentaenamido)acetamido)ethyl)nicotinamide **(I-37)** 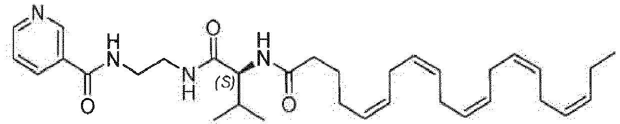
N-(2-((S)-2-((5Z,8Z,11Z,14Z,17Z)-icosa-5,8,11,14,17-pentaenamido)-3-methylbutanamido)ethyl)nicotinamide **(I-38)** 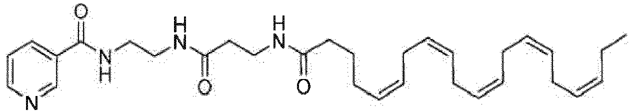
N-(2-(3-((5Z,8Z,11Z,14Z,17Z)-icosa-5,8,11,14,17-pentaenamido)propanamido)ethyl)nicotinamide **(I-39)** 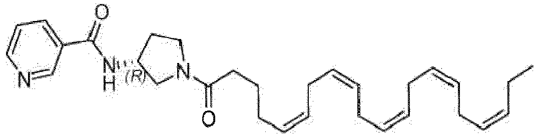
N-((R)-1-((5Z,8Z,11Z,14Z,17Z)-icosa-5,8,11,14,17-pentaenoyl)pyrrolidin-3-yl)nicotinamide **(I-40)** 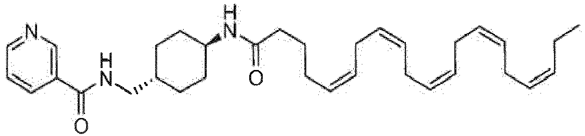
N-(((1R,4R)-4-((5Z,8Z,11Z,14Z,17Z)-icosa-5,8,11,14,17-pentaenamido)cyclohexyl)methyl)nicotinamide **(I-41)** 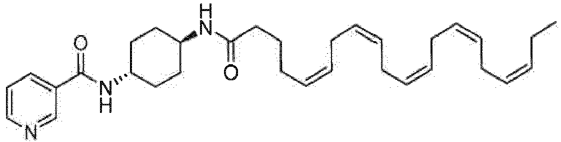
N-((1R,4R)-4-((5Z,8Z,11Z,14Z,17Z)-icosa-5,8,11,14,17-pentaenamido)cyclohexyl)nicotinamide **(I-42)** 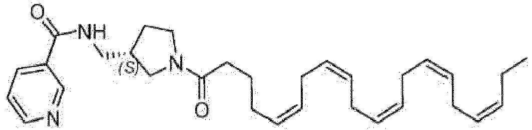
N-(((S)-1-((5Z,8Z,11Z,14Z,17Z)-icosa-5,8,11,14,17-pentaenoyl)pyrrolidin-3-yl)methyl)nicotinamide **(I-43)** 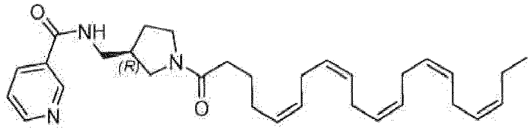
N-(((R)-1-((5Z,8Z,11Z,14Z,17Z)-icosa-5,8,11,14,17-pentaenoyl)pyrrolidin-3-yl)methyl)nicotinamide **(I-44)** 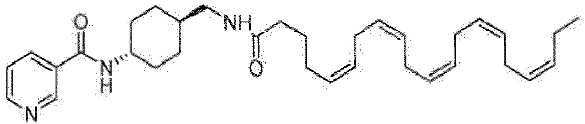
N-((1R,4R)-4-((5Z,8Z,11Z,14Z,17Z)-icosa-5,8,11,14,17-pentaenamidomethyl)cyclohexyl)nicotinamide **(I-45)** 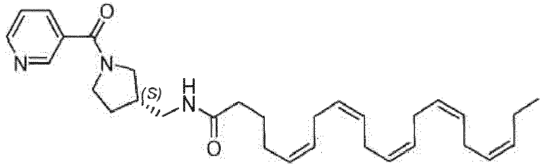
(5Z,8Z,11Z,14Z,17Z)-N-(((S)-1-nicotinoylpyrrolidin-3-yl)methyl)icosa-5,8,11,14,17-pentaenamide **(I-46)** 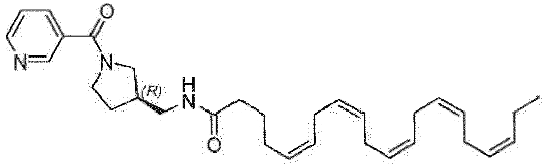
(5Z,8Z,11Z,14Z,17Z)-N-(((R)-1-nicotinoylpyrrolidin-3-yl)methyl)icosa-5,8,11,14,17-pentaenamide **(I-47)** 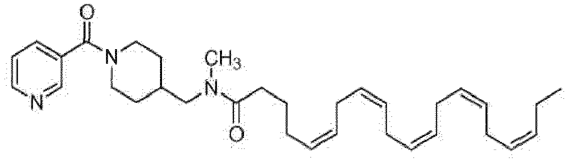
(5Z,8Z,11Z,14Z,17Z)-N-methyl-N-((1-nicotinoylpiperidin-4-yl)methyl)icosa-5,8,11,14,17-pentaenamide **(I-48)** 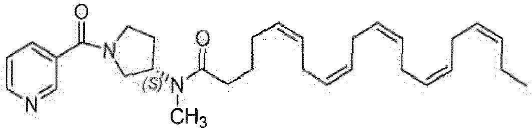
(5Z,8Z,11Z,14Z,17Z)-N-methyl-N-((S)-1-nicotinoylpyrrolidin-3-yl)icosa-5,8,11,14,17-pentaenamide **(I-49)** 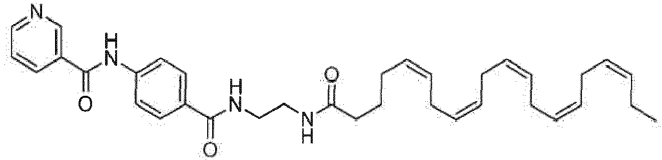
N-(4-((2-((5Z,8Z,11Z,14Z,17Z)-icosa-5,8,11,14,17-pentaenamido)ethyl)carbamoyl)phenyl)nicotinamide **(I-50)** 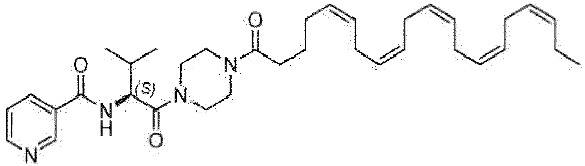
N-((S)-1-(4-((5Z,8Z,11Z,14Z,17Z)-icosa-5,8,11,14,17-pentaenoyl)piperazin-1-yl)-3-methyl-1-oxobutan-2-yl)nicotinamide **(I-51)** 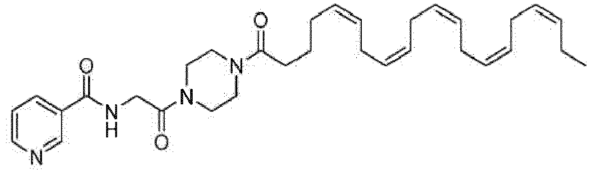
N-(2-(4-((5Z,8Z,11Z,14Z,17Z)-icosa-5,8,11,14,17-pentaenoyl)piperazin-1-yl)-2-oxoethyl)nicotinamide **(I-52)** 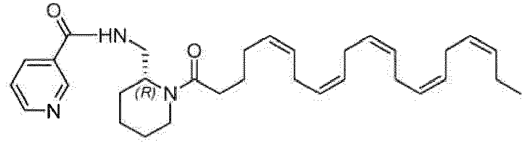
N-(((R)-1-((5Z,8Z,11Z,14Z,17Z)-icosa-5,8,11,14,17-pentaenoyl)piperidin-2-yl)mcthyl)nicotinamidc **(I-53)** 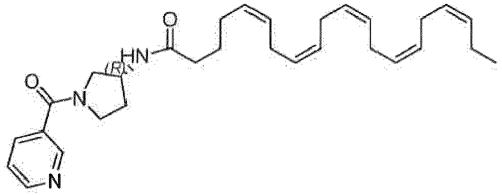
(5Z,8Z,11Z,14Z,17Z)-N-((R)-1-nicotinoylpyrrolidin-3-yl)icosa-5,8,11,14,17-pentaenamide **(I-54)** 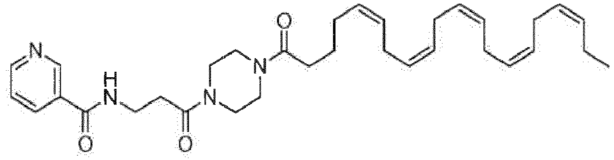
N-(3-(4-((5Z,8Z,11Z,14Z,17Z)-icosa-5,8,11,14,17-pentaenoyl)piperazin-1-yl)-3-oxopropyl)nicotinamide **(I-55)** 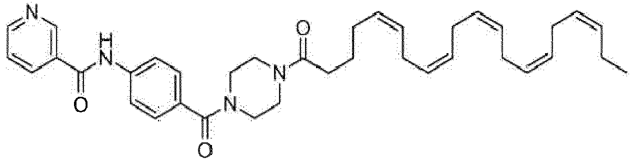
N-(4-(4-((5Z,8Z,11Z,14Z,17Z)-icosa-5,8,11,14,17-pentaenoyl)piperazine-1-carbonyl)phenyl)nicotinamide **(I-56)** 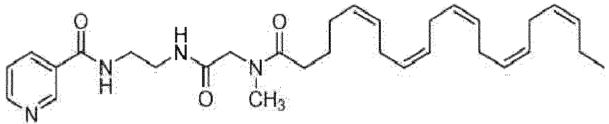
N-(2-(2-((5Z,8Z,11Z,14Z,17Z)-N-methylicosa-5,8,11,14,17-pentaenamido)acetamido)ethyl)nicotinamide **(I-57)** 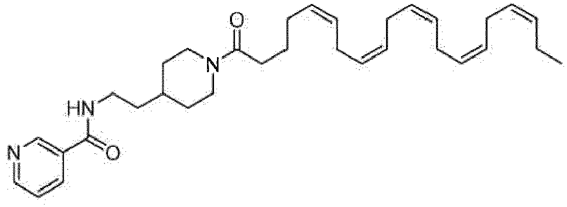
N-(2-(1-((5Z,8Z,11Z,14Z,17Z)-icosa-5,8,11,14,17-pentaenoyl)piperidin-4-yl)ethyl)nicotinamide **(I-58)** 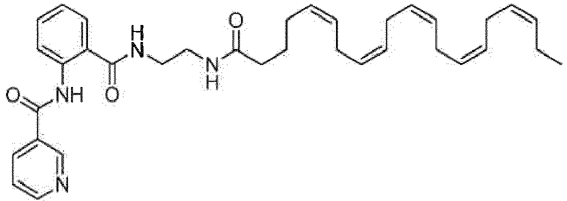
N-(2-((2-((3Z,8Z,11Z,14Z,17Z)-icosa-5,8,11,14,17-pentaenamido)ethyl)carbamoyl)phenyl)nicotinamide **(I-59)** 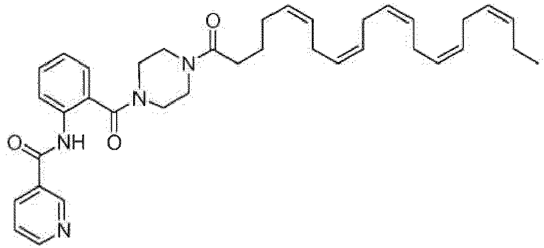
N-(2-(4-((5Z,8Z,11Z,14Z,17Z)-icosa-5,8,11,14,17-pentaenoyl)piperazine-1-carbonyl)phenyl)nicotinamide **(I-60)** 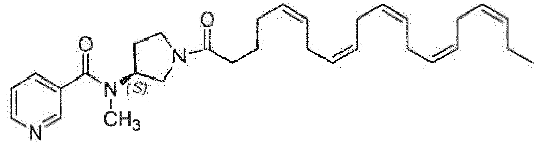
N-((S)-1-((5Z,8Z,11Z,14Z,17Z)-icosa-5,8,11,14,17-pentaenoyl)pyrrolidin-3-yl)-N-methylnicotinamide **(I-61)** 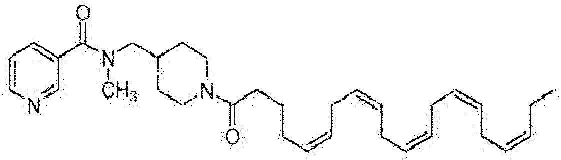
N-((1-((5Z,8Z,11Z,14Z,17Z)-icosa-5,8,11,14,17-pentaenoyl)piperidin-4-yl)methyl)-N-methylnicotinamide **(I-62)** 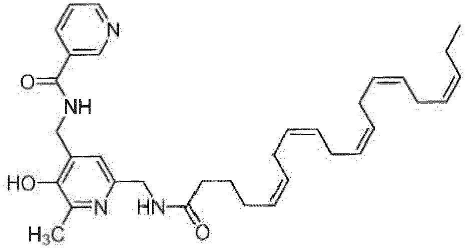
N-((3-hydroxy-6-((5Z,8Z,11Z,14Z,17Z)-icosa-5,8,11,14,17-pentaenamidomethyl)-2-methylpyridin-4-yl)methyl)nicotinamide **(I-63)** 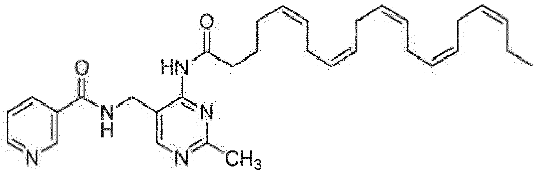
N-((4-((5Z,8Z,11Z,14Z,17Z)-icosa-5,8,11,14,17-pentaenamido)-2-methylpyrimidin-5-yl)methyl)nicotinamide **(I-64)** 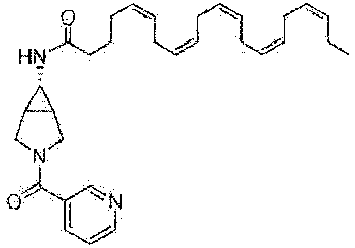
(5Z,8Z,11Z,14Z,17Z)-N-(3-nicotinoyl-3-azabicyclo[3.1.0]hexan-6-yl)icosa-5,8,11,14,17-pentaenamide **(I-65)** 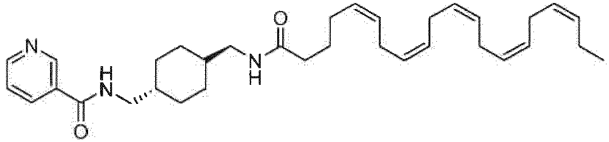
N-(((1S,4S)-4-((5Z,8Z,11Z,14Z,17Z)-icosa-5,8,11,14,17-pentaenamidomethyl)cyclohexyl)methyl)nicotinamide **(I-66)** 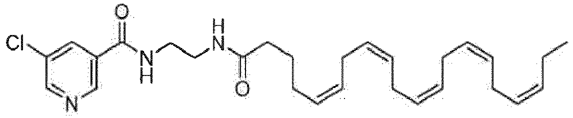
5-chloro-N-(2-((5Z,8Z,11Z,14Z,17Z)-icosa-5,8,11,14,17-pentaenamido)ethyl)nicotinamide **(I-67)** 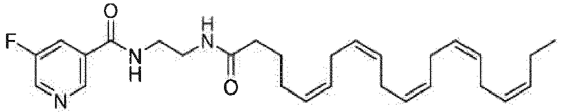
5-fluoro-N-(2-((5Z,8Z,11Z,14Z,17Z)-icosa-5,8,11,14,17-pentaenamido)ethyl)nicotinamide **(I**-**68**) 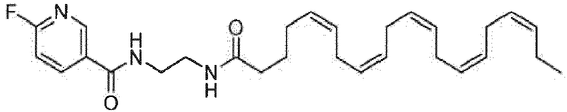
6-fluoro-N-(2-((5Z,8Z,11Z,14Z,17Z)-icosa-5,8,11,14,17-pentaenamido)ethyl)nicotinamide **(I-69)** 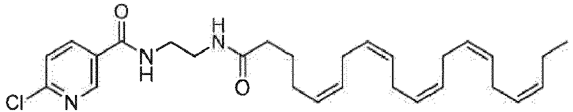
6-chloro-N-(2-((5Z,8Z,11Z,14Z,17Z)-icosa-5,8,11,14,17-pentaenamido)ethyl)nicotinamide **(I-70)** 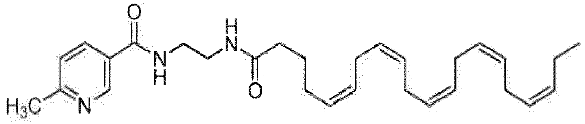
N-(2-((5Z,8Z,11Z,14Z,17Z)-icosa-5,8,11,14,17-pentaenamido)ethyl)-6-methylnicotinamide **(I-71)** 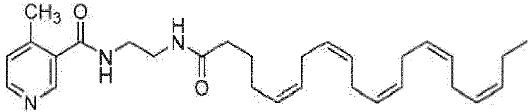
N-(2-((5Z,8Z,11Z,14Z,17Z)-icosa-5,8,11,14,17-pentaenamido)ethyl)-4-methylnicotinamide **(I-72)** 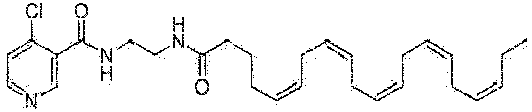
4-chloro-N-(2-((5Z,8Z,11Z,14Z,17Z)-icosa-5,8,11,14,17-pentaenamido)ethyl)nicotinamide **(I-73)** 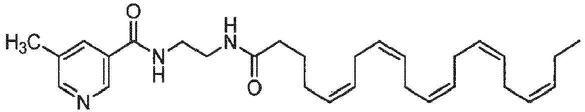
N-(2-((5Z,8Z,11Z,14Z,17Z)-icosa-5,8,11,14,17-pentaenamido)ethyl)-5-methylnicotinamide **(I-74)** 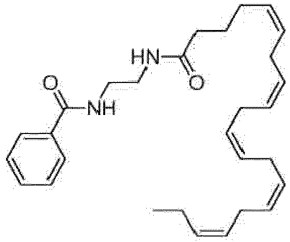
N-(2-((5Z,8Z,11Z,14Z,17Z)-icosa-5,8,11,14,17-pentaenamido)ethyl)benzamide **(II-1)** 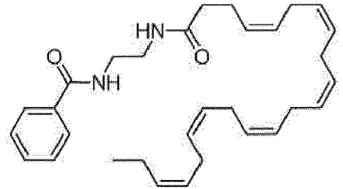
N-(2-((4Z,7Z,10Z,13Z,16Z,19Z)-docosa-4,7,10,13,16,19-hexaenamido)ethyl)benzamide **(II-2)** 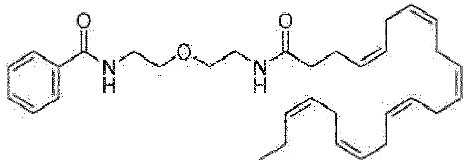
N-(2-(2-((4Z,7Z,10Z,13Z,16Z,19Z)-docosa-4,7,10,13,16,19-hexaenamido)ethoxy)ethyl)benzamide **(II-3)** 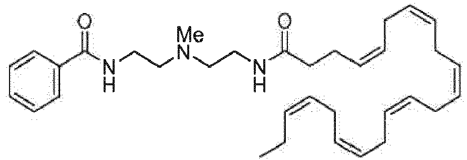
N-(2-((2-((4Z,7Z,10Z,13Z,16Z,19Z)-docosa-4,7,10,13,16,19-hexaenamido)ethyl)(methyl)amino)ethyl)benzamide **(II-4)** 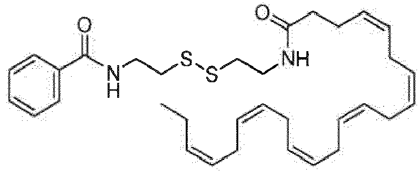
N-(2-((2-((4Z,7Z,10Z,13Z,16Z,19Z)-docosa-4,7,10,13,16,19-hexaenamido)ethyl)disulfanyl)ethyl)benzamide **(II-5)** 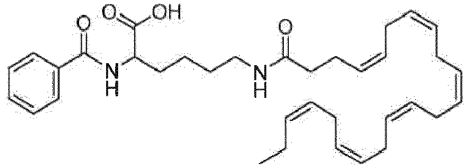
2-benzamido-6-((4Z,7Z,10Z,13Z,16Z,19Z)-docosa-4,7,10,13,16,19-hexaenamido)hexanoic acid **(II-6)** 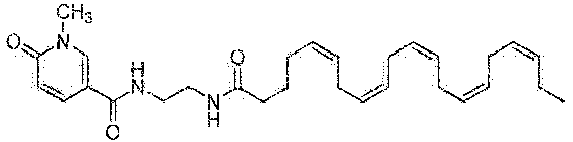
N-(2-((5Z,8Z,11Z,14Z,17Z)-icosa-5,8,11,14,17-pentaenamido)ethyl)-1-methyl-6-oxo-1,6-dihydropyridine-3-carboxamide **(II-7)** 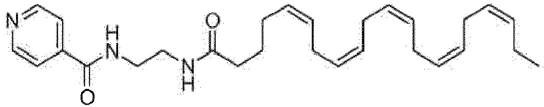
N-(2-((5Z,8Z,11Z,14Z,17Z)-icosa-5,8,11,14,17-pentaenamido)ethyl)isonicotinamide **(II-8)** 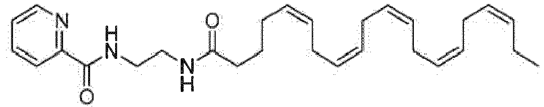
N-(2-((5Z,8Z,11Z,14Z,17Z)-icosa-5,8,11,14,17-pentaenamido)ethyl)picolinamide **(II-9)** 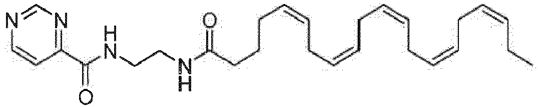
N-(2-((5Z,8Z,11Z,14Z,17Z)-icosa-5,8,11,14,17-pentaenamido)ethyl)pyrimidine-4-carboxamide **(II-10)** 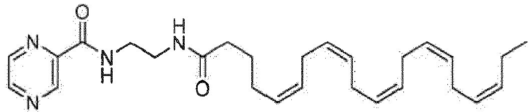
N-(2-((5Z,8Z,11Z,14Z,17Z)-icosa-5,8,11,14,17-pentaenamido)ethyl)pyrazine-2-carboxamide **(II-11)** 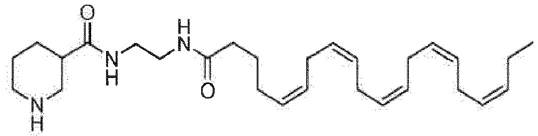
N-(2-((5Z,8Z,11Z,14Z,17Z)-icosa-5,8,11,14,17-pentaenamido)ethyl)piperidine-3-carboxamide **(II-12)** 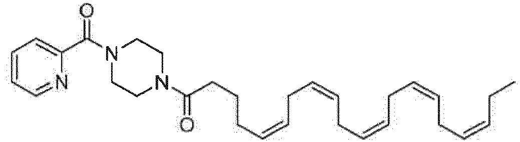
(5Z,8Z,11Z,14Z,17Z)-1-(4-picolinoylpiperazin-1-yl)icosa-5,8,11,14,17-pentaen-1-one **(II-13)** 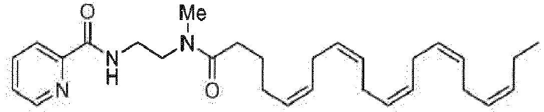
N-(2-((5Z,8Z,11Z,14Z,17Z)-N-methylicosa-5,8,11,14,17-pentaenamido)ethyl)picolinamide **(II-14)** 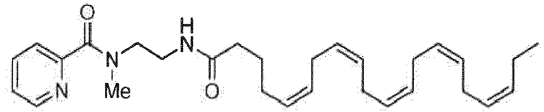
N-(2-((5Z,8Z,11Z,14Z,17Z)-icosa-5,8,11,14,17-pentaenamido)ethyl)-N-methylpicolinamide **(II-15)** 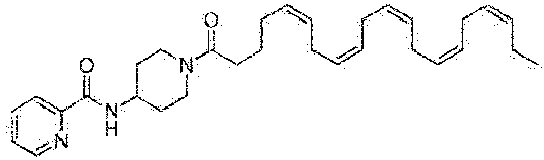
N-(1-((5Z,8Z,11Z,14Z,17Z)-icosa-5,8,11,14,17-pentaenoyl)piperidin-4-yl)picolinamide (**II-16)** 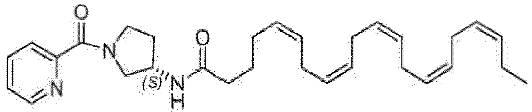
(5Z,8Z,11Z,14Z,17Z)-N-((S)-1-picolinoylpyrrolidin-3-yl)icosa-5,8,11,14,17-pentaenamide **(II-17)** 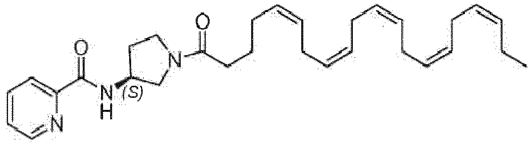
N-((S)-1-((5Z,8Z,11Z,14Z,17Z)-icosa-5,8,11,14,17-pentaenoyl)pyrrolidin-3-yl)picolinamide **(II-18)** 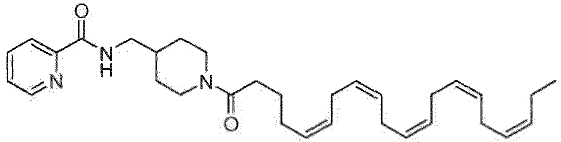
N-((1-((5Z,8Z,11Z,14Z,17Z)-icosa-5,8,11,14,17-pentaenoyl)piperidin-4-yl)methyl)picolinamide **(II-19)** 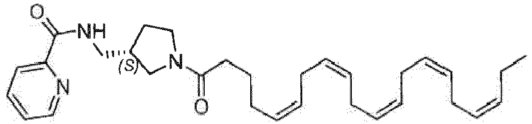
N-(((S)-1-((5Z,8Z,11Z,14Z,17Z)-icosa-5,8,11,14,17-pentaenoyl)pyrrolidin-3-yl)methyl)picolinamide **(II-20)** 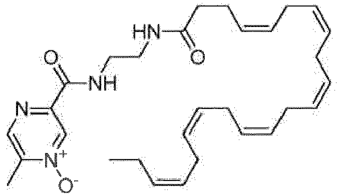
5-((2-((4Z,7Z,10Z,13Z,16Z,19Z)-docosa-4,7,10,13,16,19-hexaenamido)ethyl)carbamoyl)-2-methylpyrazine 1-oxide **(II-21)** 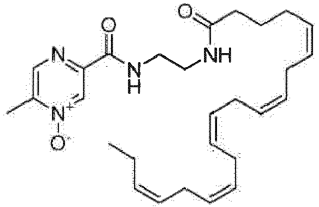
5-((2-((5Z,8Z,11Z,14Z,17Z)-icosa-5,8,11,14,17-pentaenamido)ethyl)carbamoyl)-2-mcthylpyrazine 1-oxide **(II-22)** 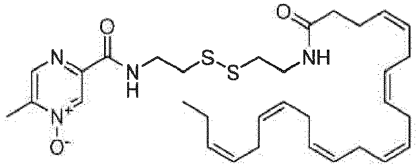
5-((2-((2-((4Z,7Z,10Z,13Z,16Z,19Z)-docosa-4,7,10,13,16,19-hexaenamido)ethyl)disulfanyl)ethyl)carbamoyl)-2-methylpyrazine 1-oxide **(II-23)** 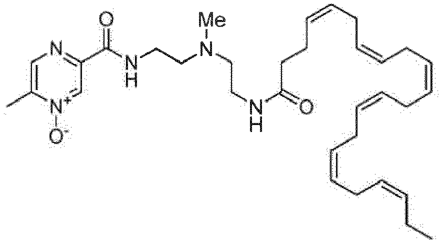
5-((2-((2-((4Z,7Z,10Z,13Z,16Z,19Z)-docosa-4,7,10,13,16,19-hexaenamido)ethyl)(methyl)amino)ethyl)carbamoyl)-2-methylpyrazine 1-oxide **(II-24)** 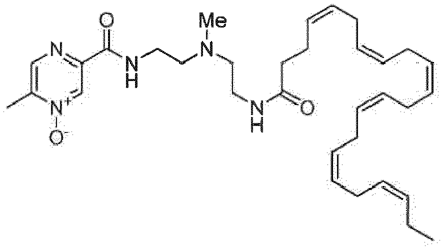
5-((2-((2-((4Z,7Z,10Z,13Z,16Z,19Z)-docosa-4,7,10,13,16,19-hexaenamido)ethyl)(methyl)amino)ethyl)carbamoyl)-2-methylpyrazine 1-oxide **(II-25)** 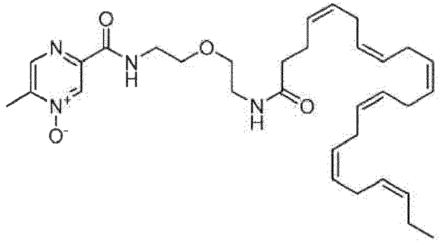
5-((2-(2-((4Z,7Z,10Z,13Z,16Z,19Z)-docosa-4,7,10,13,16,19-hexaenamido)ethoxy)ethyl)carbamoyl)-2-methylpyrazine 1-oxide **(II-26)** 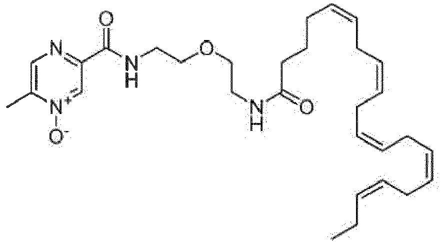
5-((2-(2-((5Z,8Z,11Z,14Z,17Z)-icosa-5,8,11,14,17-pentaenamido)ethoxy)ethyl)carbamoyl)-2-methylpyrazine 1-oxide **(II-27)** 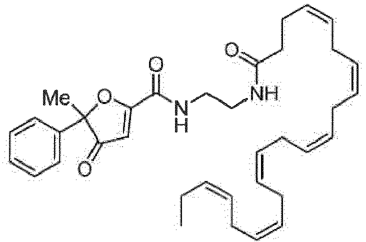
N-(2-((4Z,7Z,10Z,13Z,16Z,19Z)-docosa-4,7,10,13,16,19-hexaenamido)ethyl)-5-methyl-4-oxo-5-phenyl-4,5-dihydrofuran-2-carboxamide **(II-28)** 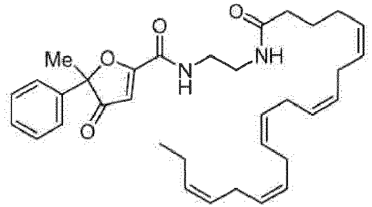
N-(2-((5Z,8Z,11Z,14Z,17Z)-icosa-5,8,11,14,17-pentaenamido)ethyl)-5-methyl-4-oxo-5-phenyl-4,5-dihydrofuran-2-carboxamide **(II-29)** 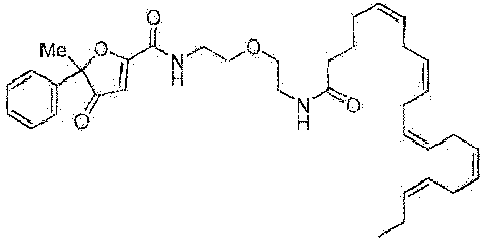
N-(2-(2-((5Z,8Z,11Z,14Z,17Z)-icosa-5,8,11,14,17-pentaenamido)ethoxy)ethyl)-5-methyl-4-oxo-5-phenyl-4,5-dihydrofuran-2-carboxamide **(II-30)** 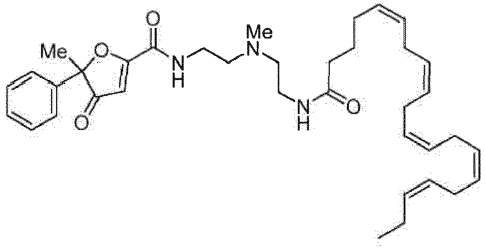
N-(2-((2-((5Z,8Z,11Z,14Z,17Z)-icosa-5,8,11,14,17-pentaenamido)ethyl)(methyl)amino)ethyl)-5-methyl-4-oxo-5-phenyl-4,5-dihydrofuran-2-carboxamide **(II-31)** 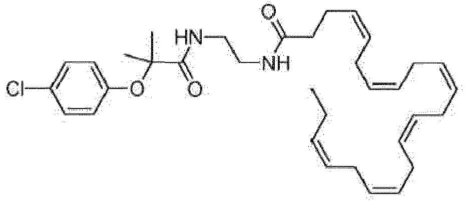
(4Z,7Z,10Z,13Z,16Z,19Z)-N-(2-(2-(4-chlorophenoxy)-2-methylpropanamido)ethyl)docosa-4,7,10,13,16,19-hexaenamide **(II-32)** 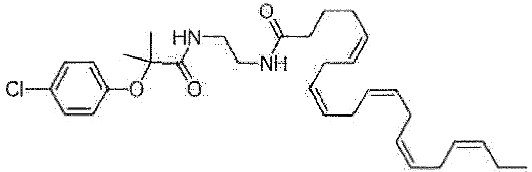
(5Z,8Z,11Z,14Z,17Z)-N-(2-(2-(4-chlorophenoxy)-2-methylpropanamido)ethyl)icosa-5,8,11,14,17-pentaenamide **(II-33)** 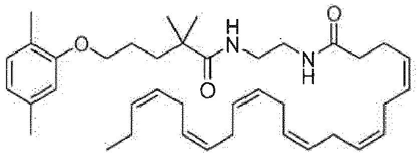
(4Z,7Z,10Z,13Z,16Z,19Z)-N-(2-(5-(2,5-dimethylphenoxy)-2,2-dimethylpentanamido)ethyl)docosa-4,7,10,13,16,19-hexaenamide **(II-34)** 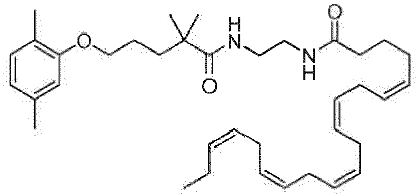
(5Z,8Z,11Z,14Z,17Z)-N-(2-(5-(2,5-dimethylphenoxy)-2,2-dimethylpentanamido)ethyl)icosa-5,8,11,14,17-pcntacnamidc **(II-35)** 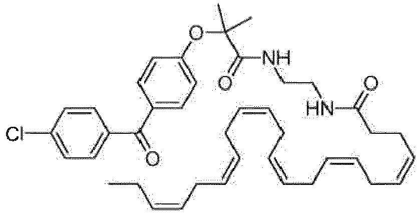
(4Z,7Z,10Z,13Z,16Z,19Z)-N-(2-(2-(4-(4-chlorobenzoyl)phenoxy)-2-methylpropanamido)ethyl)docosa-4,7,10,13,16,19-hexaenamide **(II-36)** 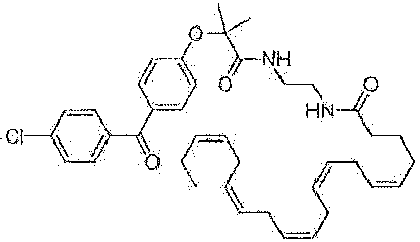
(5Z,8Z,11Z,14Z,17Z)-N-(2-(2-(4-(4-chlorobenzoyl)phenoxy)-2-methylpropanamido)ethyl)icosa-5,8,11,14,17-pentaenamide **(II-37)** 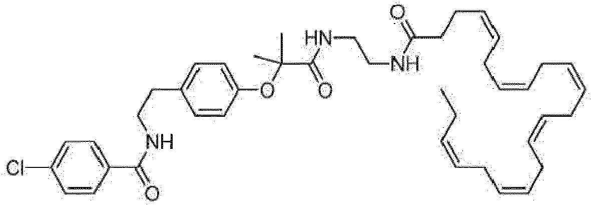
4-chloro-N-(4-((1-((2-((4Z,7Z,10Z,13Z,16Z,19Z)-docosa-4,7,10,13,16,19-hexaenamido)ethyl)amino)-2-methyl-1-oxopropan-2-yl)oxy)phenethyl)benzamide **(II-38)** 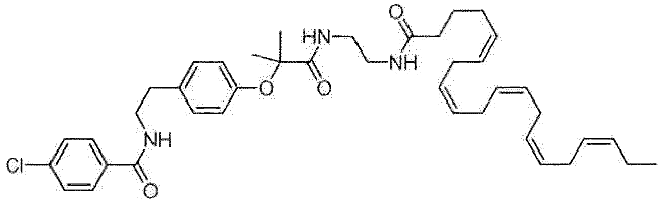
4-chloro-N-(4-((1-((2-((5Z,8Z,11Z,14Z,17Z)-icosa-5,8,11,14,17-pentaenamido)ethyl)amino)-2-methyl-1-oxopropan-2-yl)oxy)phenethyl)benzamide **(II-39)** 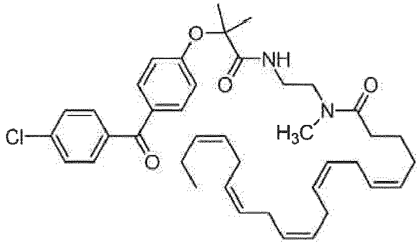
(5Z,8Z,11Z,14Z,17Z)-N-(2-(2-(4-(4-chlorobenzoyl)phenoxy)-2-methylpropanamido)ethyl)-N-methylicosa-5,8,11,14,17-pentaenamide **(II-40)** 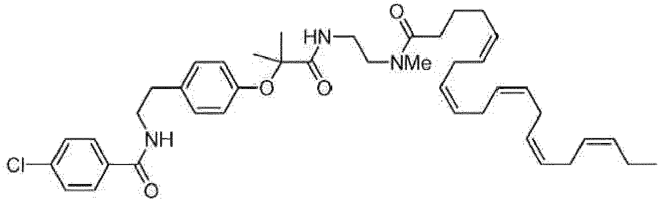
4-chloro-N-(4-((2-methyl-1-((2-((5Z,8Z,11Z,14Z,17Z)-N-methylicosa-5,8,11,14,17-pentaenamido)ethyl)amino)-1-oxopropan-2-yl)oxy)phenethyl)benzamide **(II-41)** 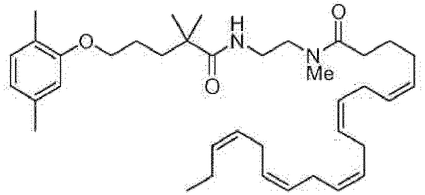
(5Z,8Z,11Z,14Z,17Z)-N-(2-(5-(2,5-dimethylphenoxy)-2,2-dimethylpentanamido)ethyl)-N-methylicosa-5,8,11,14,17-pentaenamide **(II-42)** 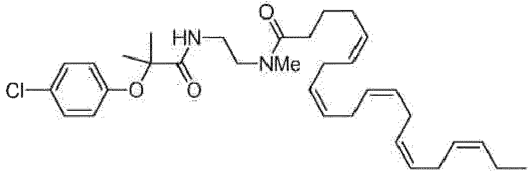
(5Z,8Z, 11Z, 14Z, 17Z)-N-(2-(2-(4-chlorophenoxy)-2-methylpropanamido)ethyl)-N-mcthylicosa-5,8,11,14,17-pcntacnamidc **(II-43)** 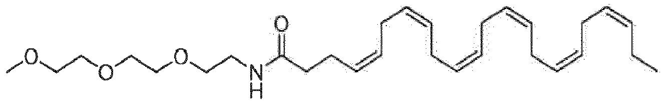
(4Z,7Z,10Z,13Z,16Z,19Z)-N-(2-(2-(2-methoxyethoxy)ethoxy)ethyl)docosa-4,7,10,13,16,19-hexaenamide **(III-1)** 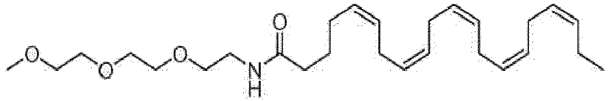
(5Z,8Z,11Z,14Z,17Z)-N-(2-(2-(2-methoxyethoxy)ethoxy)ethyl)icosa-5,8,11,14,17-pentaenamide **(III-2)** 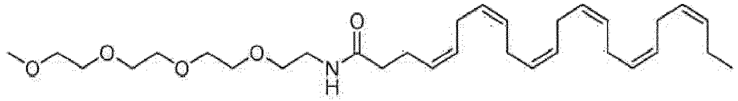
(4Z,7Z,10Z,13Z,16Z,19Z)-N-(2,5,8,11-tetraoxatridecan-13-yl)docosa-4,7,10,13,16,19-hexaenamide **(III-3)** 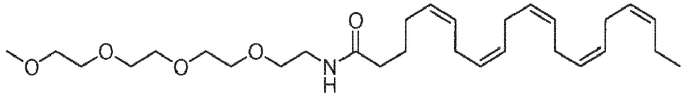
(5Z,8Z,11Z,14Z,17Z)-N-(2,5,8,11-tetraoxatridecan-13-yl)icosa-5,8,11,14,17-pentaenamide **(III-4)** 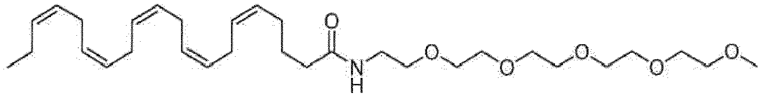
(5Z,8Z,11Z,14Z,17Z)-N-(2-(2-(2-(2-(2-methoxyethoxy)ethoxy)ethoxy)ethoxy)ethyl)icosa-5,8,11,14,17-pentaenamide **(III-5);** 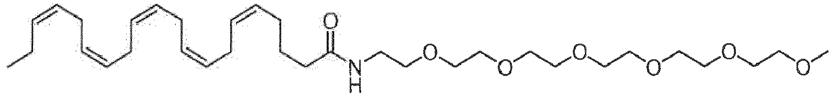
(5Z,8Z,11Z,14Z,17Z)-N-(2-(2-(2-(2-(2-(2-methoxyethoxy)ethoxy)ethoxy)ethoxy)ethoxy)ethyl)icosa-5,8,11,14,17-pentaenamide **(III-6).** 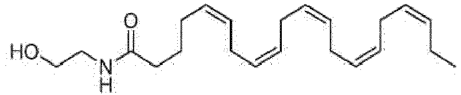
(5Z,8Z,11Z,14Z,17Z)-N-(2-hydroxyethyl)icosa-5,8,11,14,17-pentaenamide **(III-7)** 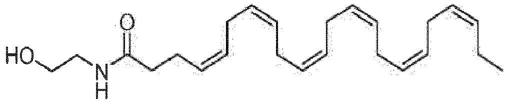
(4Z,7Z,10Z,13Z,16Z,19Z)-N-(2-hydroxyethyl)docosa-4,7,10,13,16,19-hexaenamide (**III**-**8**) 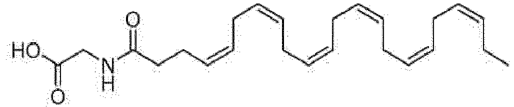
2-((4Z,7Z,10Z,13Z,16Z,19Z)-docosa-4,7,10,13,16,19-hexaenamido)acetic acid (**III**-**9**) 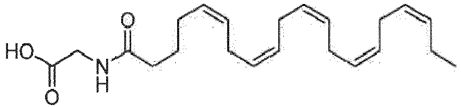
2-((5Z,8Z,11Z,14Z,17Z)-icosa-5,8,11,14,17-pentaenamido)acetic acid **(III-10)** 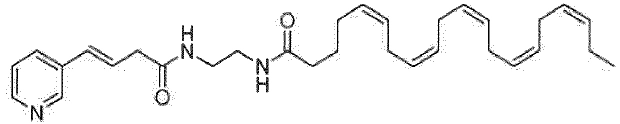
(5Z,8Z,11Z,14Z,17Z)-N-(2-((E)-4-(pyridin-3-yl)but-3-enamido)ethyl)icosa-5,8,11,14,17-pentaenamide **(IV-1)** 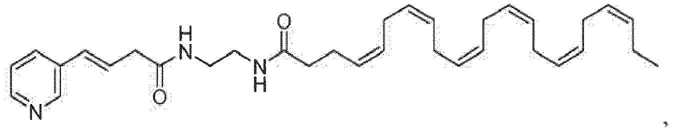
(4Z,7Z,10Z,13Z,16Z,19Z)-N-(2-((E)-4-(pyridin-3-yl)but-3-enamido)ethyl)docosa-4,7,10,13,16,19-hexaenamide **(IV-2)** 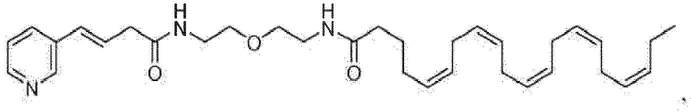
(5Z,8Z,11Z,14Z,17Z)-N-(2-(2-((E)-4-(pyridin-3-yl)but-3-enamido)ethoxy)ethyl)icosa-5,8,11,14,17-pentaenamide **(IV-3)** 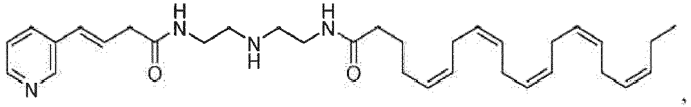
(5Z,8Z,11Z,14Z,17Z)-N-(2-(2-((E)-4-(pyridin-3-yl)but-3-enamido)ethylamino)ethyl)icosa-5,8,11,14,17-pentaenamide **(IV-4)** 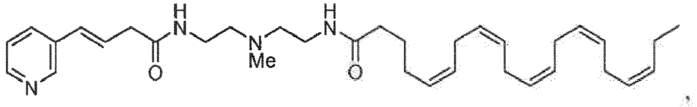
(5Z,8Z,11Z,14Z,17Z)-N-(2-(methyl(2-((E)-4-(pyridin-3-yl)but-3-enamido)ethyl)amino)ethyl)icosa-5,8,11,14,17-pentaenamide **(IV-5)** 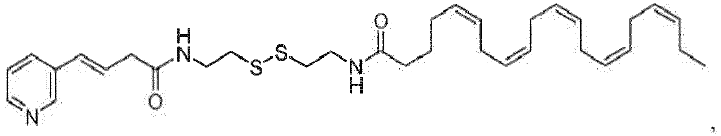
(5Z,8Z,11Z,14Z,17Z)-N-(2-(2-(2-((E)-4-(pyridin-3-yl)but-3-enamido)ethyl)disulfanyl)ethyl)icosa-5,8,11,14,17-pentaenamide **(IV-6)** 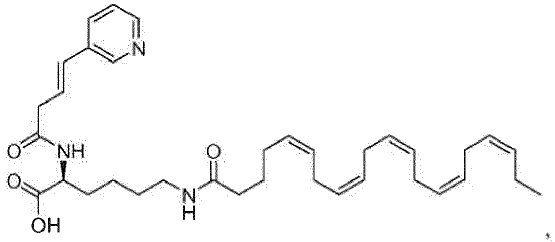
(S)-6-((5Z,8Z,11Z,14Z,17Z)-icosa-5,8,11,14,17-pentaenamido)-2-((E)-4-(pyridin-3-yl)but-3-enamido)hexanoic acid **(IV-7)** 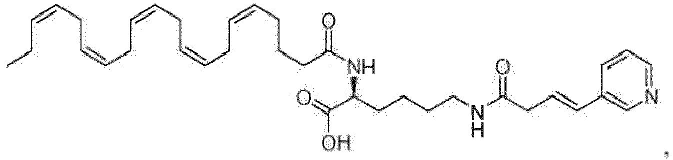
(S)-2-((5Z,8Z,11Z,14Z,17Z)-icosa-5,8,11,14,17-pentaenamido)-6-((E)-4-(pyridin-3-yl)but-3-enamido)hexanoic acid **(IV-8)** 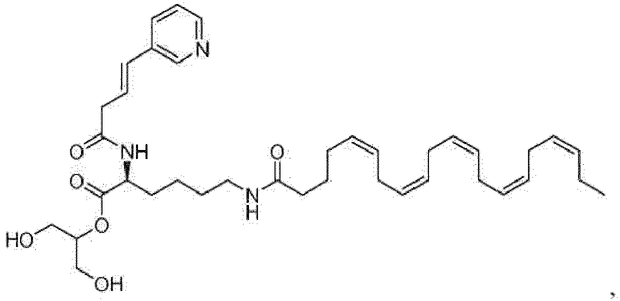
(S)-1,3-dihydroxypropan-2-yl 6-((5Z,8Z,11Z,14Z,17Z)-icosa-5,8,11,14,17-pentaenamido)-2-((E)-4-(pyridin-3-yl)but-3-enamido)hexanoate **(IV-9)** 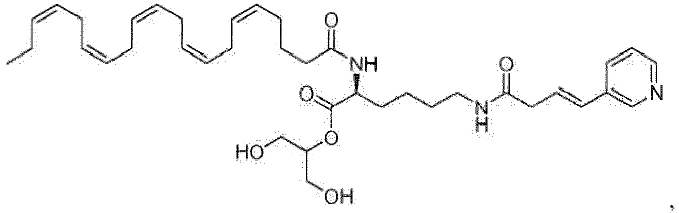
(S)-1,3-dihydroxypropan-2-yl 2-((5Z,8Z,11Z,14Z,17Z)-icosa-5,8,11,14,17-pentaenamido)-6-((E)-4-(pyridin-3-yl)but-3-enamido)hexanoate **(IV-10)** 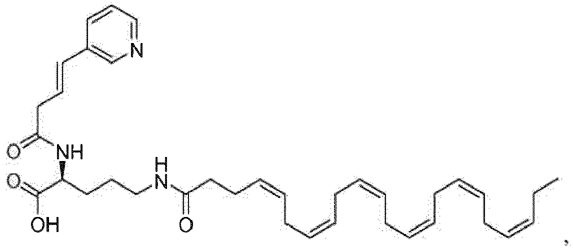
(S)-5-((4Z,7Z,10Z,13Z,16Z,19Z)-docosa-4,7,10,13,16,19-hexaenamido)-2-((E)-4-(pyridin-3-yl)but-3-enamido)pentanoic acid **(IV-11)** 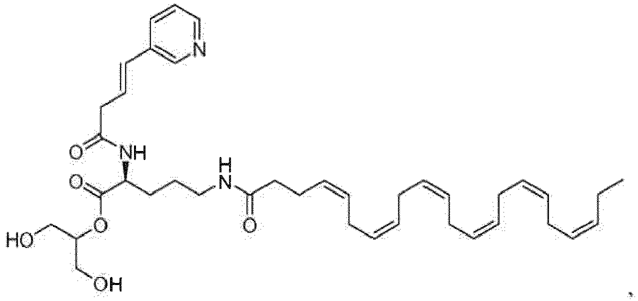
(S)-1,3-dihydroxypropan-2-yl 5-((4Z,7Z,10Z,13Z,16Z,19Z)-docosa-4,7,10,13,16,19-hexaenamido)-2-((E)-4-(pyridin-3-yl)but-3-enamido)pentanoate **(IV-12)** 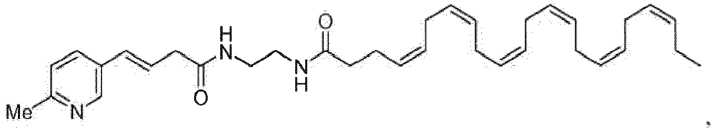
(4Z,7Z,10Z,13Z,16Z,19Z)-N-(2-((E)-4-(6-methylpyridin-3-yl)but-3-enamido)ethyl)docosa-4,7,10,13,16,19-hexaenamide **(IV-13)** 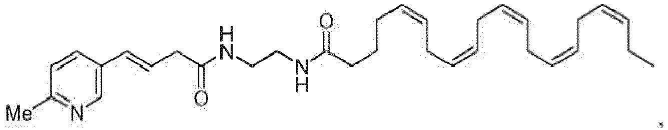
(5Z,8Z,11Z,14Z,17Z)-N-(2-((E)-4-(6-methylpyridin-3-yl)but-3-enamido)ethyl)icosa-5,8,11,14,17-pentaenamide **(IV-14)** 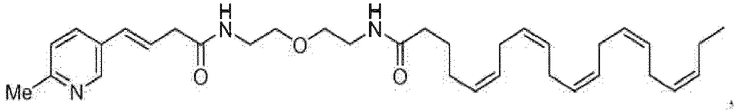
(5Z,8Z,11Z,14Z,17Z)-N-(2-(2-((E)-4-(6-methylpyridin-3-yl)but-3-enamido)ethoxy)ethyl)icosa-5,8,11,14,17-pentaenamide **(IV-15)** 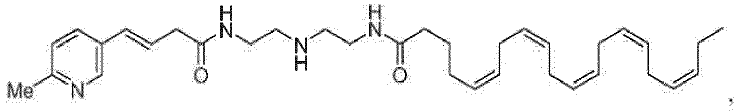
(5Z,8Z,11Z,14Z,17Z)-N-(2-(2-((E)-4-(6-methylpyridin-3-yl)but-3-enamido)ethylamino)ethyl)icosa-5,8,11,14,17-pentaenamide **(IV-16)** 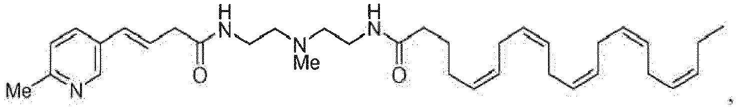
(5Z,8Z,11Z,14Z,17Z)-N-(2-(methyl(2-((E)-4-(6-methylpyridin-3-yl)but-3-enamido)ethyl)amino)ethyl)icosa-5,8,11,14,17-pentaenamide (IV-17) 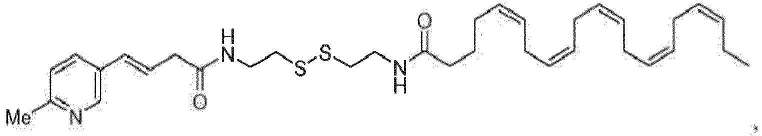
(5Z,8Z,11Z,14Z,17Z)-N-(2-(2-(2-((E)-4-(6-methylpyridin-3-yl)but-3-enamido)ethyl)disulfanyl)ethyl)icosa-5,8,11,14,17-pentaenamide **(IV-18)** 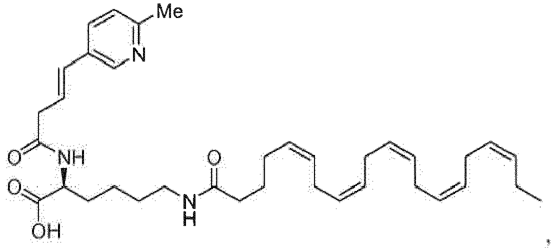
(S)-6-((5Z,8Z,11Z,14Z,17Z)-icosa-5,8,11,14,17-pentaenamido)-2-((E)-4-(6-methylpyridin-3-yl)but-3-cnamido)hcxanoic acid **(IV-19)** 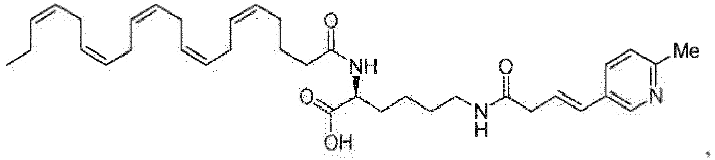
(S)-2-((5Z,8Z,11Z,14Z,17Z)-icosa-5,8,11,14,17-pentaenamido)-6-((E)-4-(6-methylpyridin-3-yl)but-3-enamido)hexanoic acid **(IV-20)** 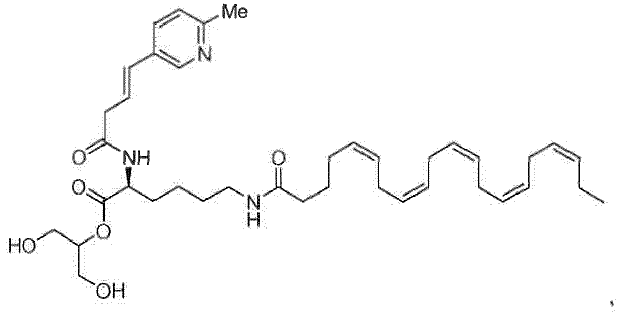
(S)-1,3-dihydroxypropan-2-yl 6-((5Z,8Z,11Z,14Z,17Z)-icosa-5,8,11,14,17-pentaenamido)-2-((E)-4-(6-methylpyridin-3-yl)but-3-enamido)hexanoate **(IV-21)** 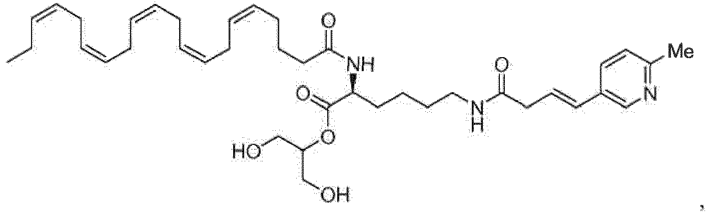
(S)-1,3-dihydroxypropan-2-yl 2-((5Z,8Z,11Z,14Z,17Z)-icosa-5,8,11,14,17-pentaenamido)-6-((E)-4-(6-methylpyridin-3-yl)but-3-enamido)hexanoate **(IV-22)** 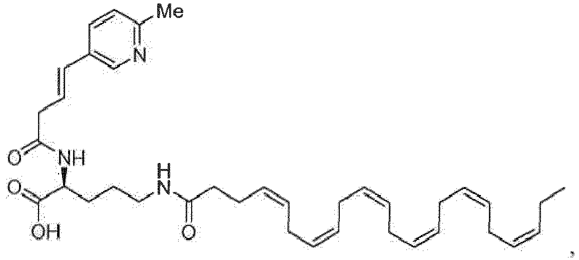
(S)-5-((4Z,7Z,10Z,13Z,16Z,19Z)-docosa-4,7,10,13,16,19-hexaenamido)-2-((E)-4-(6-methylpyridin-3-yl)but-3-enamido)pentanoic acid **(IV-23)** 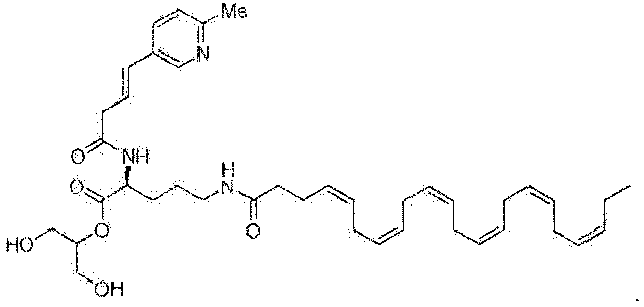
(S)-1,3-dihydroxypropan-2-yl 5-((4Z,7Z,10Z,13Z,16Z,19Z)-docosa-4,7,10,13,16,19-hexaenamido)-2-((E)-4-(6-methylpyridin-3-yl)but-3-enamido)pentanoate **(IV-24)** 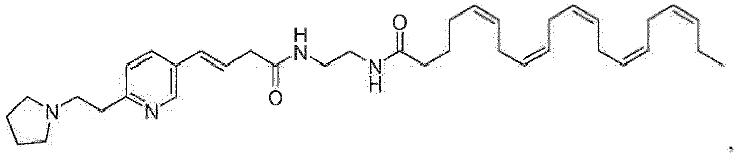
(5Z,8Z,11Z,14Z,17Z)-N-(2-((E)-4-(6-(2-(pyrrolidin-1-yl)ethyl)pyridin-3-yl)but-3-enamido)ethyl)icosa-5,8,11,14,17-pentaenamide (**IV**-**25**) 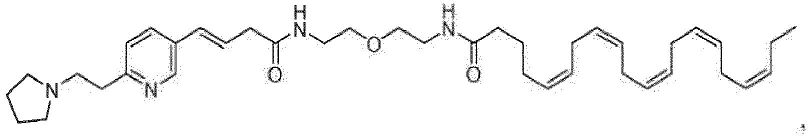
(5Z,8Z,11Z,14Z,17Z)-N-(2-(2-((E)-4-(6-(2-(pyrrolidin-1-yl)ethyl)pyridin-3-yl)but-3-enamido)ethoxy)ethyl)icosa-5,8,11,14,17-pentaenamide **(IV-26)** 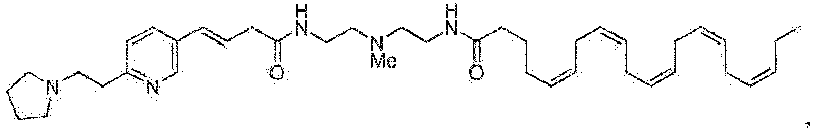
(5Z,8Z,11Z,14Z,17Z)-N-(2-(methyl(2-((E)-4-(6-(2-(pyrrolidin-1-yl)ethyl)pyridin-3-yl)but-3-enamido)ethyl)amino)ethyl)icosa-5,8,11,14,17-pentaenamide **(IV-27)** 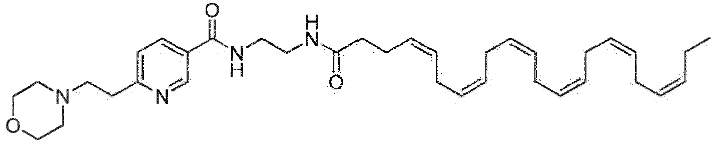
N-(2-((4Z,7Z,10Z,13Z,16Z,19Z)-docosa-4,7,10,13,16,19-hexaenamido)ethyl)-6-(2-morpholinoethyl)nicotinamide **(V-1)** 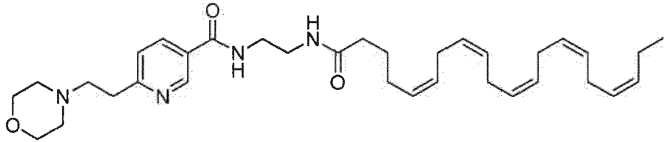
N-(2-((5Z,8Z,11Z,14Z,17Z)-icosa-5,8,11,14,17-pentaenamido)ethyl)-6-(2-morpholinoethyl)nicotinamide **(V-2)** 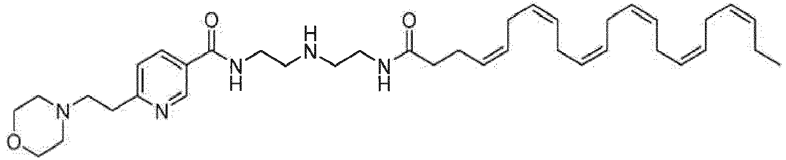
N-(2-((2-((4Z,7Z,10Z,13Z, 16Z,19Z)-docosa-4,7,10,13,16,19-hexaenamido)ethyl)amino)ethyl)-6-(2-morpholinoethyl)nicotinamide **(V-3)** 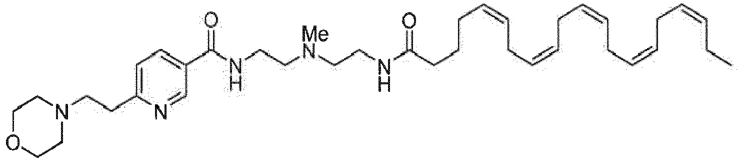
N-(2-((2-((5Z,8Z,11Z,14Z,17Z)-icosa-5,8,11,14,17-pentaenamido)ethyl)(methyl)amino)ethyl)-6-(2-morpholinoethyl)nicotinamide **(V-4)** 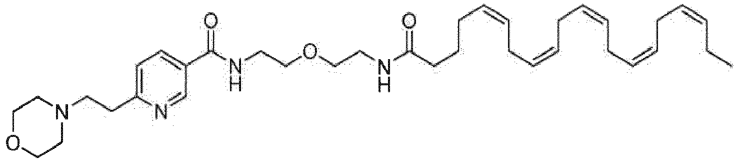
N-(2-(2-((5Z,8Z,11Z,14Z,17Z)-icosa-5,8,11,14,17-pentaenamido)ethoxy)ethyl)-6-(2-morpholinoethyl)nicotinamide **(V-5)** 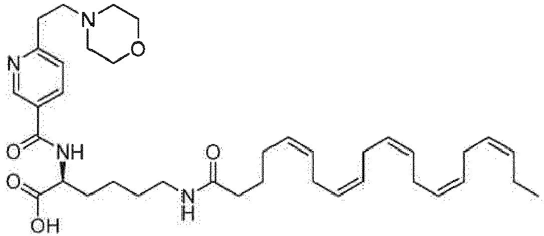
(S)-6-((5Z,8Z,11Z,14Z,17Z)-icosa-5,8,11,14,17-pentaenamido)-2-(6-(2-morpholinoethyl)nicotinamido)hexanoic acid **(V-6)** 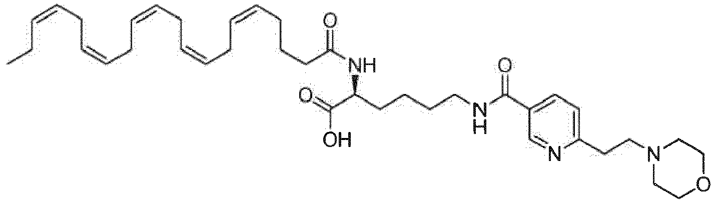
(S)-2-((5Z,8Z,11Z,14Z,17Z)-icosa-5,8,11,14,17-pentaenamido)-6-(6-(2-morpholinoethyl)nicotinamido)hexanoic acid **(V-7)** 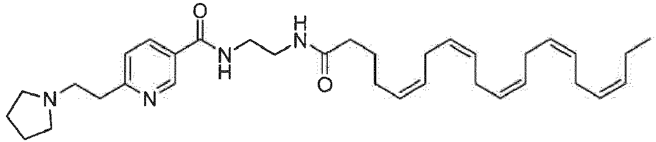
N-(2-((5Z,8Z,11Z,14Z,17Z)-icosa-5,8,11,14,17-pentaenamido)ethyl)-6-(2-(pyrrolidin-1-yl)ethyl)nicotinamide **(V-8)** 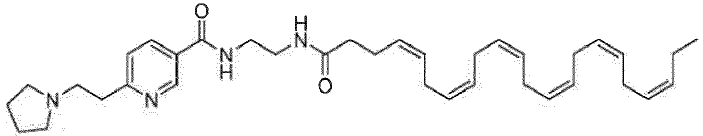
N-(2-((4Z,7Z,10Z,13Z,16Z,19Z)-docosa-4,7,10,13,16,19-hexaenamido)cthyl)-6-(2-(pyrrolidin-1-yl)ethyl)nlcotinamide **(V-9)** 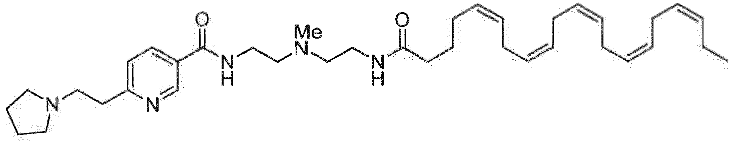
N-(2-((2-((5Z,8Z,11Z,14Z,17Z)-icosa-5,8,11,14,17-pentaenamido)ethyl)(methyl)amino)ethyl)-6-(2-(pyrrolidin-1-yl)ethyl)nicotinamide **(V-10)** 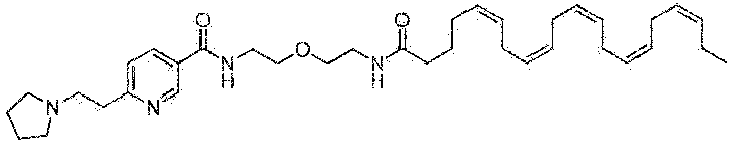
N-(2-(2-((5Z,8Z,11Z,14Z,17Z)-icosa-5,8,11,14,17-pentaenamido)ethoxy)ethyl)-6-(2-(pyrrolidin-1-yl)ethyl)nicotinamide **(V-11)** 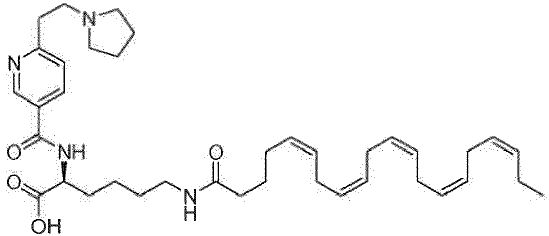
(S)-6-((5Z,8Z,11Z,14Z,17Z)-icosa-5,8,11,14,17-pentaenamido)-2-(6-(2-(pyrrolidin-1-yl)ethyl)nicotinamido)hexanoic acid **(V-12)** 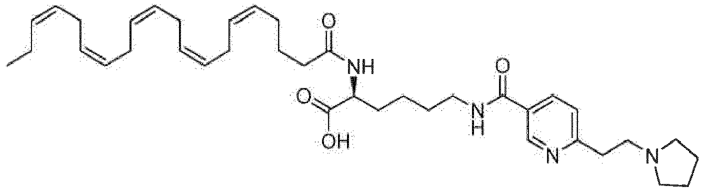
(S)-2-((5Z,8Z,11Z,14Z,17Z)-icosa-5,8,11,14,17-pentaenamido)-6-(6-(2-(pyrrolidin-1-yl)ethyl)nicotinamido)hexanoic acid **(V-13)** 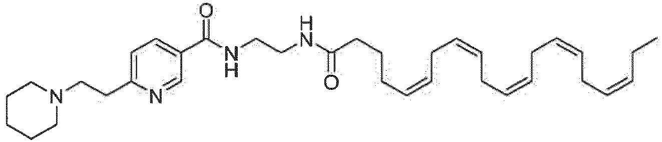
N-(2-((5Z,8Z,11Z,14Z,17Z)-icosa-5,8,11,14,17-pentaenamido)ethyl)-6-(2-(piperidtn-1-yl)ethyl)nicotinamide **(V-14)** 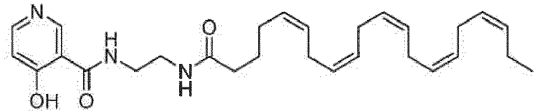
4-hydroxy-N-(2-((5Z,8Z,11Z,14Z,17Z)-icosa-5,8,11,14,17-pentaenamido)ethyl)nicotinamide **(VI-1)** 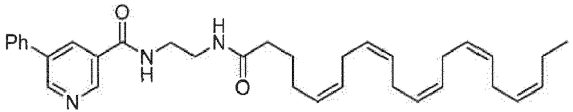
N-(2-((5Z,8Z,11Z,14Z,17Z)-icosa-5,8,11,14,17-pentaenamido)ethyl)-5-phenylnicotinamide **(VI-2)** 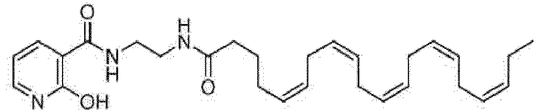
2-hydroxy-N-(2-((5Z,8Z,11Z,14Z,17Z)-icosa-5,8,11,14,17-pentaenamido)ethyl)nicotinamide **(VI-3)** 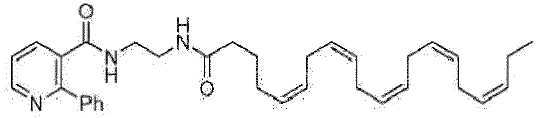
N-(2-((5Z,8Z,11Z,14Z,17Z)-icosa-5,8,11,14,17-pentaenamido)ethyl)-2-phenylnicotinamide **(VI-4)** 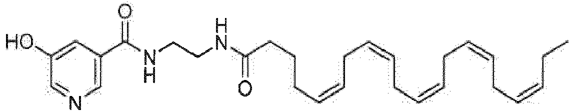
5-hydroxy-N-(2-((5Z,8Z,11Z,14Z,17Z)-icosa-5,8,11,14,17-pentaenamido)ethyl)nicotinamide **(VI-5)** 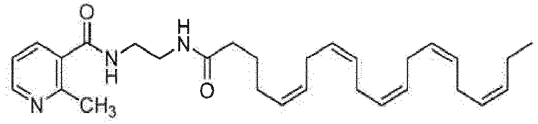
N-(2-((5Z,8Z,11Z,14Z,17Z)-icosa-5,8,11,14,17-pentaenamido)ethyl)-2-methylnicotinamide **(VI-6)** 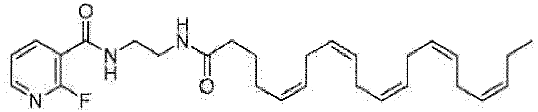
2-fluoro-N-(2-((5Z,8Z,11Z,14Z,17Z)-icosa-5,8,11,14,17-pentaenamido)ethyl)nicotinamide **(VI-7)** 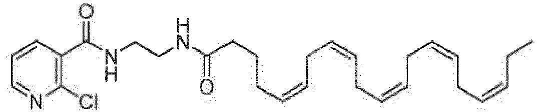
2-chloro-N-(2-((5Z,8Z,11Z,14Z,17Z)-icosa-5,8,11,14,17-pentaenamido)ethyl)nicotinamide **(VI-8)** 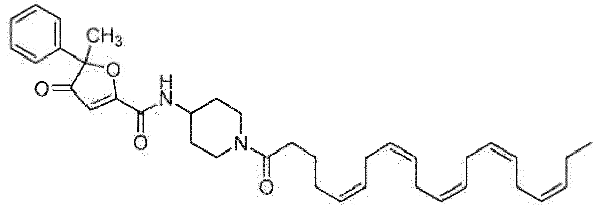
N-(1-((5Z,8Z,11Z,14Z,17Z)-icosa-5,8,11,14,17-pentaenoyl)piperidin-4-yl)-5-methyl-4-oxo-5-phenyl-4,5-dihydrofuran-2-carboxamide **(VII-1)** 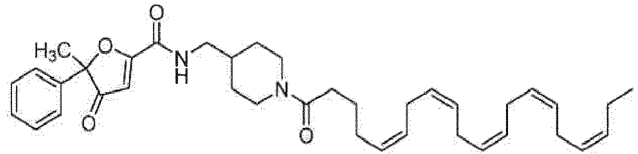
N-((1-((5Z,8Z,11Z,14Z,17Z)-icosa-5,8,11,14,17-pentaenoyl)piperidin-4-yl)methyl)-5-methyl-4-oxo-5-phenyl-4,5-dihydrofuran-2-carboxamide **(VII-2)** 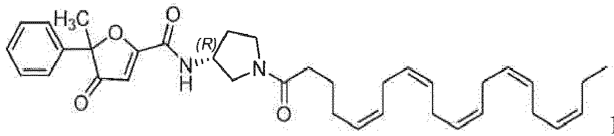
N-((R)-1-((5Z,8Z,11Z,14Z,17Z)-icosa-5,8,11,14,17-pentaenoyl)pyrrolidin-3-yl)-5-methyl-4-oxo-5-phenyl-4,5-dihydrofuran-2-carboxamide **(VII-3)** 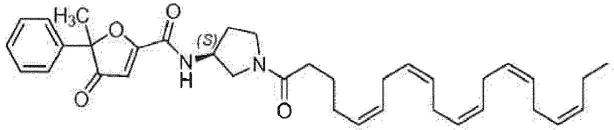
N-((S)-1-((5Z,8Z,11Z,14Z,17Z)-icosa-5,8,11,14,17-pentacnoyl)pyrrolidin-3-yl)-5-methyl-4-oxo-5-phenyl-4,5-dihydrofuran-2-carboxamide **(VII-4)** 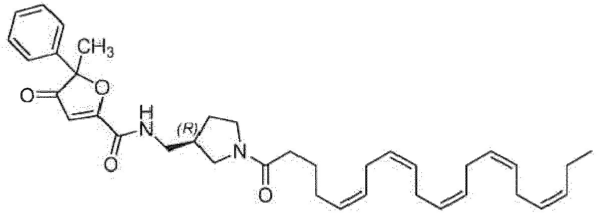
N-(((R)-1-((5Z,8Z,11Z,14Z,17Z)-icosa-5,8,11,14,17-pentaenoyl)pyrrolidin-3-yl)methyl)-5-methyl-4-oxo-5-phenyl-4,5-dihydrofuran-2-carboxamide **(VII-5)** 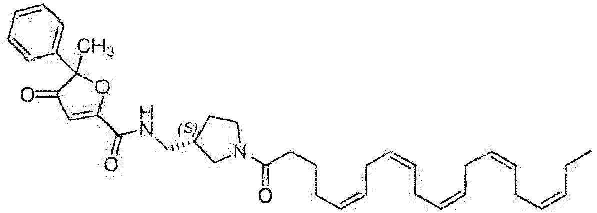
N-(((S)-1-((5Z,8Z,11 Z,14Z,17Z)-icosa-5,8,11,14,17-pentaenoyl)pyrrolidin-3-yl)methyl)-5-methyl-4-oxo-5-phenyl-4,5-dihydrofuran-2-carboxamide (VII-6) 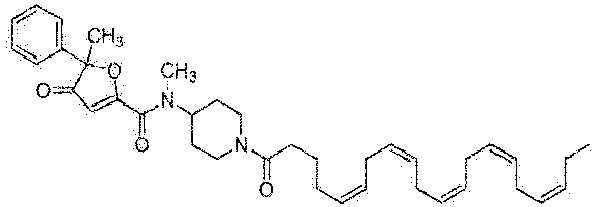
N-(1-((5Z,8Z,11Z,14Z,17Z)-icosa-5,8,11,14,17-pentacnoyl)pipcridin-4-yl)-N,5-dimethyl-4-oxo-5-phenyl-4,5-dihydrofuran-2-carboxamide **(VII-7)** 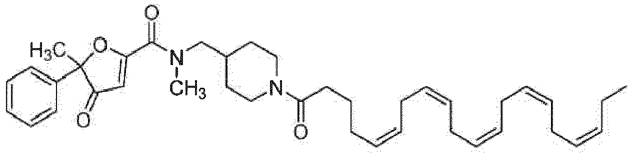
N-((1-((5Z,8Z,11Z,14Z,17Z)-icosa-5,8,11,14,17-pentaenoyl)pipendin-4-yl)methyl)-N,5-dimethyl-4-oxo-5-phenyl-4,5-dihydrofuran-2-carboxamide **(VII-8)** 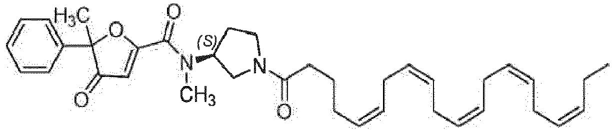
N-((S)-1-((5Z,8Z,11Z,14Z,17Z)-icosa-5,8,11,14,17-pentaenoyl)pyrrolidin-3-yl)-N,5-dimethyl-4-oxo-5-phenyl-4,5-dihydrofuran-2-carboxamide **(VII-9)** 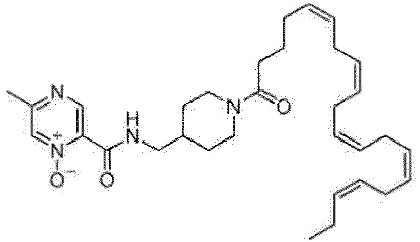
2-(((1-((5Z,8Z,11Z,14Z,17Z)-icosa-5,8,11,14,17-pentaenoyl)piperidin-4-yl)methyl)carbamoyl)-5-methylpyrazine 1-oxide **(VII-10)** 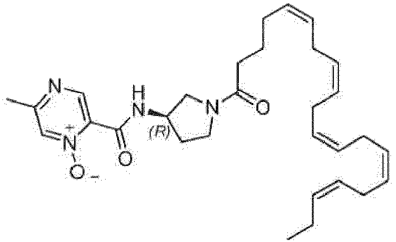
2-(((R)-1-((5Z,8Z,11Z,14Z,17Z)-icosa-5,8,11,14,17-pentaenoyl)pyrrolidin-3-yl)carbamoyl)-5-methylpyrazine 1-oxide **(VII-11)** 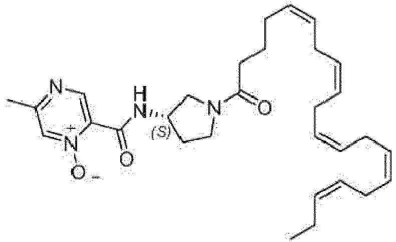
2-(((S)-1-((5Z,8Z,11Z,14Z,17Z)-icosa-5,8,11,14,17-pentaenoyl)pyrrolidin-3-yl)carbamoyl)-5-methylpyrazine 1-oxide **(VII-12)** 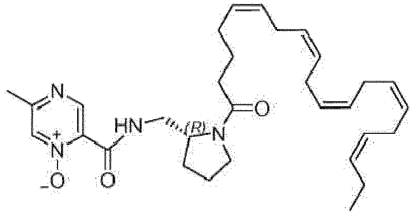
2-((((R)-1-((5Z,8Z,11Z,14Z,17Z)-icosa-5,8,11,14,17-pentaenoyl)pyrrolidin-2-yl)methyl)carbamoyl)-5-methylpyrazine 1-oxide **(VII-13)** 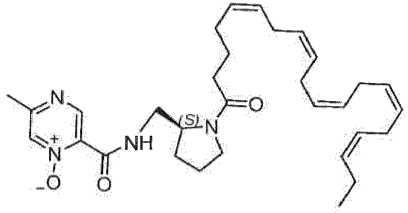
2-((((S)-1-((5Z,8Z,11Z,14Z,17Z)-icosa-5,8,11,14,17-pentacnoyl)pyrrolidin-2-yl)methyl)carbamoyl)-5-methylpyrazine 1-oxide **(VII-14)** 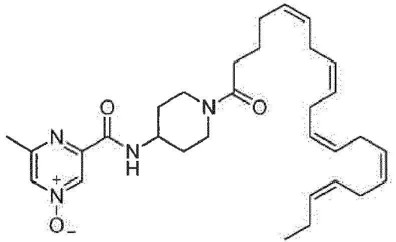
3-(1-((5Z,8Z,11Z,14Z,17Z)-icosa-5,8,11,14,17-pentaenoyl)piperidin-4-yl)carbamoyl)-5-methylpyrazine 1-oxide **(VII-15)** 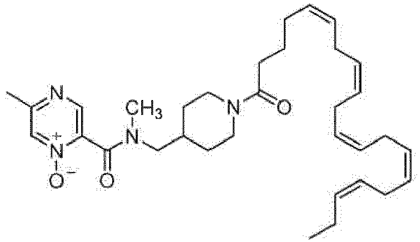
2-(((1-((5Z,8Z,11Z,14Z,17Z)-icosa-5,8,11,14,17-pentaenoyl)piperidin-4-yl)methyl)(methyl)carbamoyl)-5-methylpyrazine 1-oxide **(VII-16)** 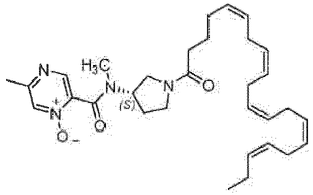
2-(((S)-1-((5Z,8Z,11Z,14Z,17Z)-icosa-5,8,11,14,17-pentaenoyl)pyrrolidin-3-yl)(methyl)carbamoyl)-5-methylpyrazine 1-oxide **(VII-17)** 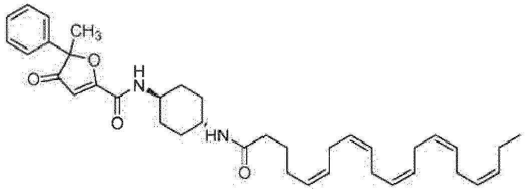
N-((1r,4r)-4-((5Z,8Z,11Z,14Z,17Z)-icosa-5,8,11,14,17-pentaenamido)cyclohexyl)-5-methyl-4-oxo-5-phenyl-4,5-dihydrofuran-2-carboxamide **(VII-18)** 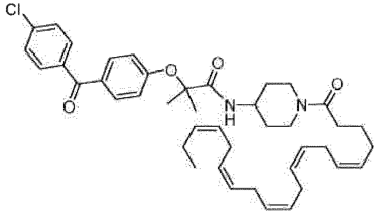
2-(4-(4-chlorobenzoyl)phenoxy)-N-(1-((5Z,8Z,11Z,14Z,17Z)-icosa-5,8,11,14,17-pentaenoyl)piperidin-4-yl)-2-methylpropanamide **(VII-19)** 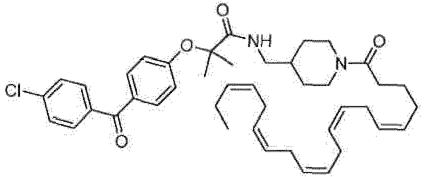
2-(4-(4-chlorobenzoyl)phenoxy)-N-((1-((5Z,8Z,11Z,14Z,17Z)-icosa-5,8,11,14,17-pentaenoyl)piperidin-4-yl)methyl)-2-methylpropanamide **(VII-20)** 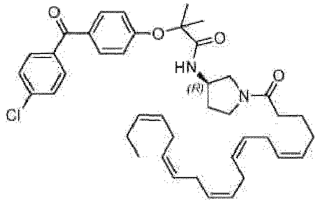
2-(4-(4-chlorobenzoyl)phenoxy)-N-((R)-1-((5Z,8Z,11Z,14Z,17Z)-icosa-5,8,11,14,17-pentaenoyl)pyrrolidin-3-yl)-2-methylpropanamide **(VII-21)** 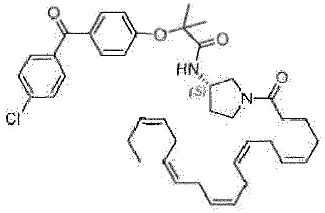
2-(4-(4-chlorobenzoyl)phenoxy)-N-((S)-1-((5Z,8Z,11Z,14Z,17Z)-icosa-5,8,11,14,17-pentaenoyl)pyrrolidin-3-yl)-2-methylpropanamide **(VII-22)** 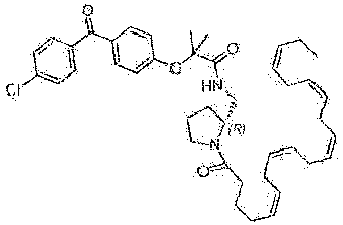
2-(4-(4-chlorobenzoyl)phenoxy)-N-(((R)-1-((5Z,8Z,11Z,14Z,17Z)-icosa-5,8,11,14,17-pentaenoyl)pyrrolidin-2-yl)methyl)-2-methylpropanamide **(VII-23)** 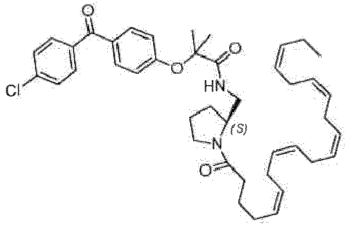
2-(4-(4-chlorobenzoyl)phenoxy)-N-(((S)-1-((5Z,8Z,11Z,14Z,17Z)-icosa-5,8,11,14,17-pentaenoyl)pyrrolidin-2-yl)methyl)-2-methylpropanamide **(VII-24)** 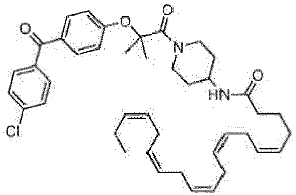
(5Z,8Z,11Z,14Z,17Z)-N-(1-(2-(4-(4-chlorobenzoyl)phenoxy)-2-methylpropanoyl)piperidin-4-yl)icosa-5,8,11,14,17-pentaenamide **(VII-25)** 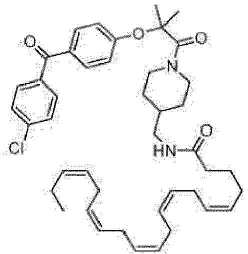
(5Z,8Z,11Z,14Z,17Z)-N-((1-(2-(4-(4-chlorobenzoyl)phenoxy)-2-methylpropanoyl)piperidin-4-yl)methyl)icosa-5,8,11,14,17-pentaenamide **(VII-26)** 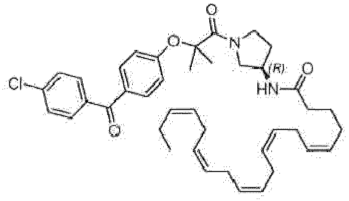
(5Z,8Z,11Z,14Z,17Z)-N-((R)-1-(2-(4-(4-chlorobenzoyl)phenoxy)-2-methylpropanoyl)pyrrolidin-3-yl)icosa-5,8,11,14,17-pentaenamide **(VII-27)** 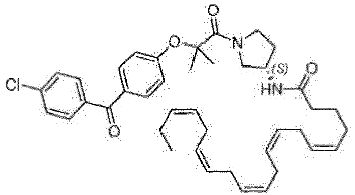
(5Z,8Z,11Z,14Z,17Z)-N-((S)-1-(2-(4-(4-chlorobenzoyl)phenoxy)-2-methylpropanoyl)pyrrolidin-3-yl)icosa-5,8,11,14,17-pentaenamide **(VII-28)** 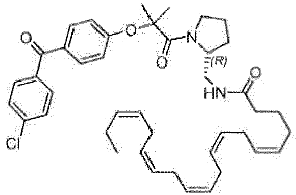
(5Z,8Z,11Z,14Z,17Z)-N-(((R)-1-(2-(4-(4-chlorobenzoyl)phenoxy)-2-methylpropanoyl)pyrrolidin-2-yl)methyl)icosa-5,8,11,14,17-pentaenamide **(VII-29)** 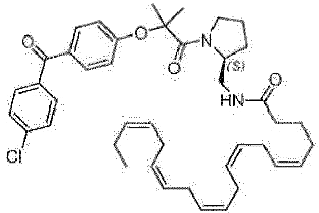
(5Z,8Z,11Z,14Z,17Z)-N-(((S)-1-(2-(4-(4-chlorobenzoyl)phenoxy)-2-methylpropanoyl)pyrrolidin-2-yl)methyl)icosa-5,8,11,14,17-pentaenamide **(VII-30)** 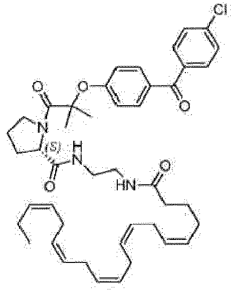
(S)-1-(2-(4-(4-chlorobenzoyl)phenoxy)-2-methylpropanoyl)-N-(2-((5Z,8Z,11Z,14Z,17Z)-icosa-5,8,11,14,17-pentaenamido)ethyl)pyrrolidine-2-carboxamide **(VII-31)** 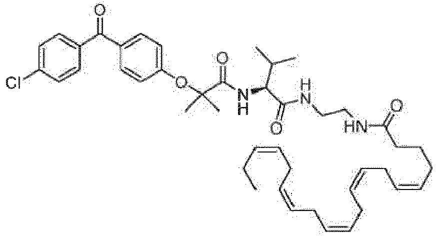
(5Z,8Z,11Z,14Z,17Z)-N-(2-((S)-2-(2-(4-(4-chlorobenzoyl)phenoxy)-2-methylpropanamido)-3-methylbutanamido)ethyl)icosa-5,8,11,14,17-pentaenamide **(VII-32)** 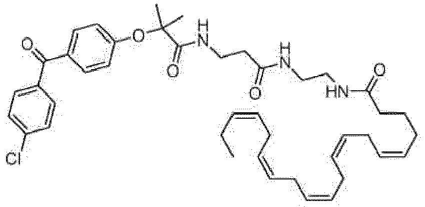
(SZ,8Z,11Z,14Z,17Z)-N-(2-(3-(2-(4-(4-chlorobenzoyl)phenoxy)-2-methylpropanamido)propanamido)ethyl)icosa-5,8,11,14,17-pentaenamide **(VII-33)** 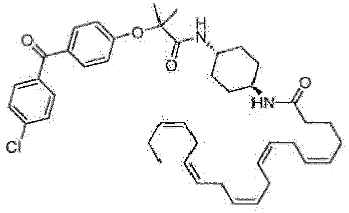
(5Z,8Z,11Z,14Z,17Z)-N-((1r,4r)-4-(2-(4-(4-chlorobenzoyl)phenoxy)-2-methylpropanamido)cyclohexyl)icosa-5,8,11,14,17-pentaenamide **(VII-34)** 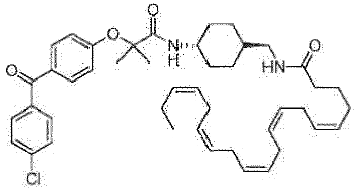
(5Z,8Z,11Z,14Z,17Z)-N-(((1s,4s)-4-(2-(4-(4-chlorobenzoyl)phenoxy)-2-methylpropanamido)cyclohexyl)methyl)icosa-5,8,11,14,17-pentaenamide **(VII-35)** 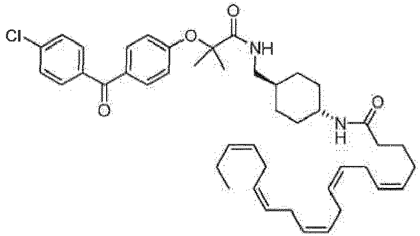
(5Z,8Z,11Z,14Z,17Z)-N-((1r,4r)-4-((2-(4-(4-chlorobenzoyl)phenoxy)-2-methylpropanamido)methyl)cyclohexyl)icosa-5,8,11,14,17-pentaenamide **(VII-36)** 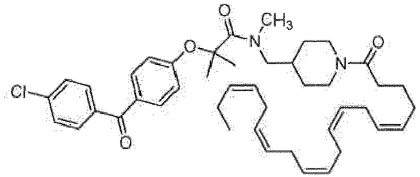
2-(4-(4-chlorobenzoyl)phenoxy)-N-((1-((5Z,8Z,11Z, 14Z,17Z)-icosa-5,8,11,14,17-pentaenoyl)piperidin-4-yl)methyl)-N,2-dimethylpropanamide **(VII-37)** 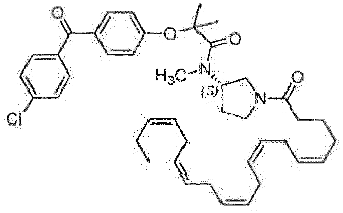
2-(4-(4-chlorobenzoyl)phenoxy)-N-((S)-1-((5Z,8Z,11Z,14Z,17Z)-icosa-3,8,11,14,17-pentaenoyl)pyrrolidin-3-yl)-N,2-dimethylpropanamide **(VII-38)** 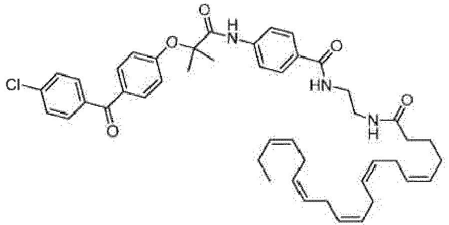
4-(2-(4-(4-chlorobenzoyl)phenoxy)-2-methylpropanamido)-N-(2-((5Z,8Z,11Z,14Z,17Z)-icosa-5,8,11,14,17-pentaenamido)ethyl)benzamide **(VII-39)** 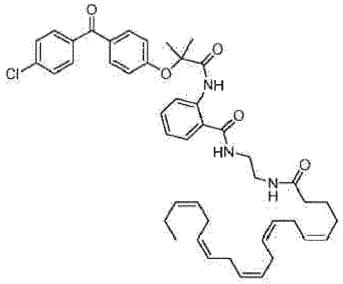
2-(2-(4-(4-chiorobenzoyl)phenoxy)-2-methylpropanamido)-N-(2-((5Z,8Z,11Z,14Z,17Z)-icosa-5,8,11,14,17-pentaenamido)ethyl)benzamide **(VII-40)** 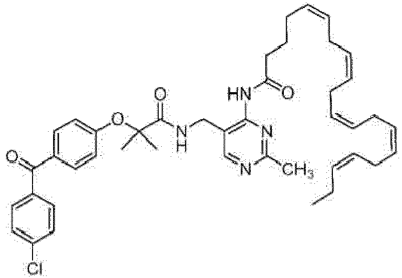
(5Z.8Z,11Z,14Z,17Z)-N-(5-((2-(4-(4-chlorobenzoyl)phenoxy)-2-methylpropanamido)methyl)-2-methylpyrimidin-4-yl)icosa-5,8,11,14,17-pentaenamide **(VII-41)** 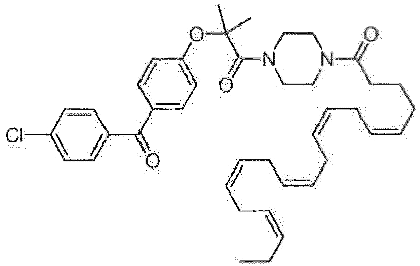
(5Z,8Z,11Z,14Z,17Z)-1-(4-(2-(4-(4-chlorobenzoyl)phenoxy)-2-methylpropanoyl)piperazin-1-yl)icosa-5,8,11,14,17-pentaen-1-one **(VII-42)**

### Methods for using the fatty acid bioactive derivatives

The invention also includes methods for treating metabolic diseases such as the treatment or prevention of metabolic diseases including atherosclerosis, dyslipidemia, coronary heart disease, hypercholesterolemia, Type 2 diabetes, elevated cholesterol, metabolic syndrome and cardiovascular disease.

In one embodiment, the method involves the inhibition of PCSK9 by fatty acid derivatives. Inhibition of PCSK9 will lead to a reduction in LDL-C.

In one embodiment, the method comprises contacting a cell with a fatty acid derivative in an amount sufficient to decrease the release of triglycerides or VLDL or LDL or cause an increase in reverse cholesterol transport or increase HDL concentrations.

Also provided in the invention is a method for inhibiting, preventing, or treating a metabolic disease, or symptoms of a metabolic disease, in a subject. Examples of such disorders include, but are not limited to atherosclerosis, dyslipidemia, hypertriglyceridemia, hypertension, heart failure, cardiac arrhythmias, low HDL levels, high LDL levels, sudden death, stable angina, coronary heart disease, acute myocardial infarction, secondary prevention of myocardial infarction, cardiomyopathy, endocarditis, type 2 diabetes, insulin resistance, impaired glucose tolerance, hypercholesterolemia, stroke, hyperlipidemia, hyperlipoproteinemia, chronic kidney disease, intermittent claudication, hyperphosphatemia, carotid atherosclerosis, peripheral arterial disease, diabetic nephropathy, hypercholesterolemia in HIV infection, acute coronary syndrome (ACS), non-alcoholic fatty liver disease, arterial occlusive diseases, cerebral arteriosclerosis, cerebrovascular disorders, myocardial ischemia, and diabetic autonomic neuropathy. Because of the ability of fatty acid niacin conjugates and other fatty acid conjugates used as PCSK9 inhibitors to lower cholesterol and triglycerides, they can also be used to treat diseases of the liver such as fatty liver disease, nonalcoholic fatty liver disease (NFLD), nonalcoholic steatohepatitis (NASH).

In some embodiments, the fatty acid niacin conjugates and other fatty acid conjugates used as PCSK9 inhibitors can be used to treat familial hyperlipidemia. Hyperlipidemia are classified according to which types of lipids are elevated, that is hypercholesterolemia, hypertriglyceridemia, or both in combined hyperlipidemia. Elevated levels of lipoprotein may also be classified as a form of hyperlipidemia. There are five types of hyperlipoproteinemia (types I through V) and these are further classified according to the Fredrikson classification, based on the pattern of lipoproteins on electrophoresis or ultracentrifugation. **Type I hyperlipoproteinemia** has three subtypes: Type Ia (also called Buerger-Gruetz syndrome or familial hyperchylomicronemia), Type Ib (also called familial apoprotein CII deficiency) and Type Ic. Due to defects in either decreased in lipoprotein lipase (LPL), altered ApoC2 or LPL inhibitor in blood, all three subtypes of Type I hyperlipoproteinemia share the same characteristic increase in chylomicrons. The frequency of occurrence for Type I hyperlipoproteinemia is 1 in 1,000,000 and thus far treatment has consisted mainly of diet. Because of the ability of fatty acid niacin conjugates in affecting postprandial lipids, it can be especially useful in treating Type I hyperlipoproteinemia. Type **II hyperlipoproteinemia** has two subtypes: Type IIa (also called familial hypercholesterolemia) is characterized by an elevated level of low-density lipoprotein (LDL); and Type IIb (also called familial combined hyperlipidemia) is characterized by an elevated level of LDL and very-low density lipoprotein (VLDL). **Type III hyperlipoproteinemia** (also called familial dysbetalipoproteinemia) is characterized by an elevated level of intermediate-density lipoprotein (IDL). **Type IV hyperlipoproteinemia** (also called familial hypertriglyceridemia) is characterized by an elevated level of VLDL. **Type V hyperlipoproteinemia** is characterized by an elevated level of VLDL and chylomicrons. Treatment for Type V hyperlipoproteinemia thus far has not been adequate with using just niacin or fibrate. Because of the ability of fatty acid niacin conjugates in affecting postprandial lipids, it can be especially useful in treating Type V hyperlipoproteinemia.

In some embodiments, the subject is administered an effective amount of a fatty acid derivative.

The invention also includes pharmaceutical compositions useful for treating or preventing a metabolic disease, or for inhibiting a metabolic disease, or more than one of these activities. The compositions can be suitable for internal use and comprise an effective amount of a fatty acid derivative and a pharmaceutically acceptable carrier. The fatty acid derivatives are especially useful in that they demonstrate very low peripheral toxicity or no peripheral toxicity.

The fatty acid derivatives can each be administered in amounts that are sufficient to treat or prevent a metabolic disease or prevent the development thereof in subjects.

Administration of the fatty acid derivatives can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, all using forms well known to those skilled in the pharmaceutical arts.

Illustrative pharmaceutical compositions are tablets and gelatin capsules comprising a fatty acid niacin derivative and a pharmaceutically acceptable carrier, such as: a) a diluent, *e.g*., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, *e.g*., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, *e.g*., starches, agar, methyl cellulose, bentonite, xanthan gum, alginic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion, *etc.* For example, the fatty acid niacin derivative is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the fatty acid niacin derivatives.

The fatty acid derivatives can be also formulated as a suppository that can be prepared from fatty emulsions or suspensions; using polyalkylene glycols such as propylene glycol, as the carrier.

The fatty acid derivatives can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in United States Patent No. 5,262,564, the contents of which are herein incorporated by reference in their entirety.

Fatty acid derivatives can also be delivered by the use of monoclonal antibodies as individual carriers to which the fatty acid derivatives are coupled. The fatty acid derivatives can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the fatty acid derivatives can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels. In one embodiment, fatty acid *derivatives* arc not covalently bound to a polymer, e.g., a polycarboxylic acid polymer, or a polyacrylate.

Parenteral injectable administration is generally used for subcutaneous, intramuscular or intravenous injections and infusions. Injectables can be prepared in conventional forms, either as liquid solutions or suspensions or solid forms suitable for dissolving in liquid prior to injection.

Compositions can be prepared according to conventional mixing, granulating or coating methods, respectively, and the present pharmaceutical compositions can contain from about 0.1 % to about 90 %, from about 10 % to about 90 %, or from about 30 % to about 90 % of the fatty acid derivative by weight or volume.

The dosage regimen utilizing the fatty acid derivative is selected in accordance with a variety of factors including type, species, age, weight, sex and medical condition of the patient; the severity of the condition to be treated; the route of administration; the renal or hepatic function of the patient; and the particular fatty acid niacin derivative employed. A physician or veterinarian of ordinary skill in the art can readily determine and prescribe the effective amount of the drug required to prevent, counter or arrest the progress of the condition.

Effective dosage amounts of the present invention, when used for the indicated effects, range from about 20 mg to about 5,000 mg of the fatty acid derivative per day. Compositions for *in vivo* or *in vitro* use can contain about 20, 50, 75, 100, 150, 250, 500, 750, 1,000, 1,250, 2,500, 3,500, or 5,000 mg of the fatty acid derivative. In one embodiment, the compositions are in the form of a tablet that can be scored. Effective plasma levels of the fatty acid niacin derivative can range from 5 ng/mL to 5000 ng/mL. Appropriate dosages of the fatty acid derivatives can be determined as set forth in Goodman, L. S.; Gilman, A. The Pharmacological Basis of Therapeutics. 5th ed.; MacMillan: New York, 1975, pp. 201-226.

Fatty acid derivatives can be administered in a single daily dose, or the total daily dosage can be administered in divided doses of two, three or four times daily. Furthermore, fatty acid derivatives can be administered in intranasal form via topical use of suitable intranasal vehicles, or via transdermal routes, using those forms of transdermal skin patches well known to those of ordinary skill in that art. To be administered in the form of a transdermal delivery system, the dosage administration can be continuous rather than intermittent throughout the dosage regimen. Other illustrative topical preparations include creams, ointments, lotions, aerosol sprays and gels, wherein the concentration of the fatty acid derivative ranges from about 0.1 % to about 15 %, w/w or w/v.

### Combination therapies

Fatty acid derivatives may also be administered with other therapeutic agents such as cholesterol-lowering agents, fibrates and hypolipidemic agents, anti-diabetic agents, anti-diabetic agents, antihypertensive agents and anti-inflammatory agents.

In some embodiments, the other therapeutic agent is a cholesterol-lowering agents. Non limiting examples of cholesterol-lowering agents are atorvastatin, cerivastatin, fluvastatin, lovastatin, pitavastatin, pravastatin, rosuvastatin, simvastatin, ezetimibe, and the combination of ezetimibe/simvastatin (Vytorin®).

In some embodiments, the other therapeutic agent is a fibrate or hypolipidemic agent. Non-limiting examples of fibrates or hypolipidemic agents are acifran, acipimox, beclobrate, bezafibrate, binifibrate, ciprofibrate, clofibrate, colesevelam, gemfibrozil, fenofibrate, melinamide, niacin, and ronafibrate.

In some embodiments, the other therapeutic agent is a DPP-IV inhibitor as anti-diabetic agent. Non-limiting examples of DPP-IV inhibitors as anti-diabetic agents are sitagliptin, saxagliptin, vildagliptin, linagliptin, dutogliptin, gemigliptin and alogliptin.

In some embodiments, the other therapeutic agent is an Anti-diabetic agent. Non-limiting examples of anti-diabetic agents are acarbose, epalrestat, exenatide, glimepiride, liraglutide, metformin, miglitol, mitiglinide, nateglinide, pioglitazone, pramlintide, repaglinide, rosiglitazone, tolrestat, troglitazone, and voglibose.

In some embodiments, the other therapeutic agent is an antihypertensive agents. Non-limiting examples of antihypertensive agents include alacepril, alfuzosin, aliskiren, amlodipine besylate, amosulalol, aranidipine, arotinolol HCl, azelnidipine, barnidipine hydrochloride, benazepril hydrochloride, benidipine hydrochloride, betaxolol HCl, bevantolol HCl, bisoprolol fumarate, bopindolol, bosentan, budralazine, bunazosin HCl, candesartan cilexetil, captopril, carvedilol, celiprolol HCl, cicletanine, cilazapril, cinildipine, clevidipine, delapril, dilevalol, doxazosin mesylate, efonidipine, enalapril maleate, enalaprilat, eplerenone, eprosartan, felodipine, fenoldopam mesylate, fosinopril sodium, guanadrel sulfate, imidapril HCl, irbesartan, isradipine, ketanserin, lacidipine, lercanidipine, lisinopril, losartan, manidipine hydrochloride, mebefradil hydrochloride, moxonidine, nebivolol, nilvadipine, nipradilol, nisoldipine, olmesartan medoxomil, perindopril, pinacidil, quinapril, ramipril, rilmedidine, spirapril HCl, telmisartan, temocarpil, terazosin HCl, tertatolol HCl, tiamenidine HCl, tilisolol hydrochloride, trandolapril, treprostinil sodium, trimazosin HCl, valsartan, and zofenopril calcium.

In other embodiments, suitable angiotensin-converting-enzyme (ACE) inhibitors used in the above-described combination therapies include, without limitation, enalapril, ramipril, quinapril, perindopril, lisinopril, imidapril, zofenopril, trandolapril, fosinopril, and captopril.

### METHODS OF MAKING

### Methods for making the fatty acid bioactive derivatives

Examples of synthetic pathways useful for making fatty acid derivatives described herein are described, for example, in US 2010/0041748 and US 2011/0053990, and specifically for compounds of Formula II are set forth in the Examples below and generalized in **Schemes 1-9.** 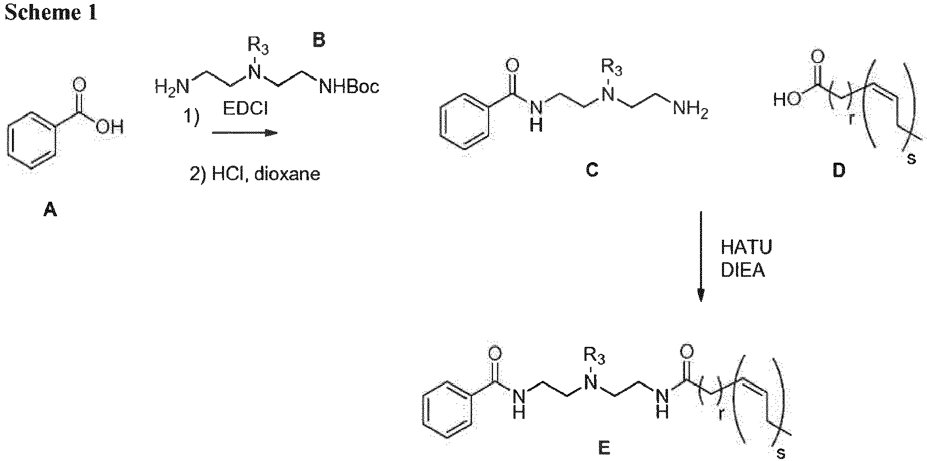 wherein R₃, r, and s are as defined above.

The mono-BOC protected amine of the formula **B** can be obtained from commercial sources or prepared according to the procedures outlined in Krapcho et al. Synthetic Communications 1990, 20, 2559-2564. Compound **A** can be amidated with the amine **B** using a coupling reagent such as DCC, CDI, EDC, or optionally with a tertiary amine base and/or catalyst, e.g., DMAP, followed by deprotection of the BOC group with acids such as TFA or HCl in a solvent such as CH₂Cl₂ or dioxane to produce the coupled compound **C.** Activation of compound **C** with a coupling agent such as HATU in the presence of an amine such as DIEA followed by addition of a fatty acid of formula **D** affords compounds of the formula **E.** To those familiar in the art, compound **A** can be substituted with any other aryl, heteroaryl or heterocyclic carboxylic acid. 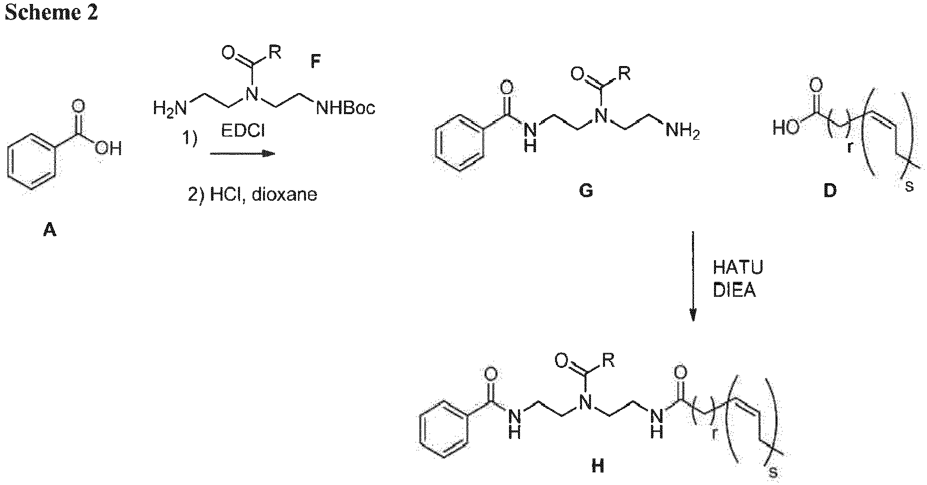 wherein R, r, and s are as defined above.

The acylated amine of the formula **F** can be prepared using the procedures outlined in Andruszkiewicz et al. Synthetic Communications 2008, 38, 905-913. Compound **A** can be amidated with the amine **F** using a coupling reagent such as DCC, CDI, EDC, or optionally with a tertiary amine base and/or catalyst, e.g., DMAP, followed by deprotection of the BOC group with acids such as TFA or HCl in a solvent such as CH₂Cl₂ or dioxane to produce the coupled compound **G.** Activation of compound **G** with a coupling agent such as HATU in the presence of an amine such as DIEA followed by addition of a fatty acid of formula **D** affords compounds of the formula **H.** 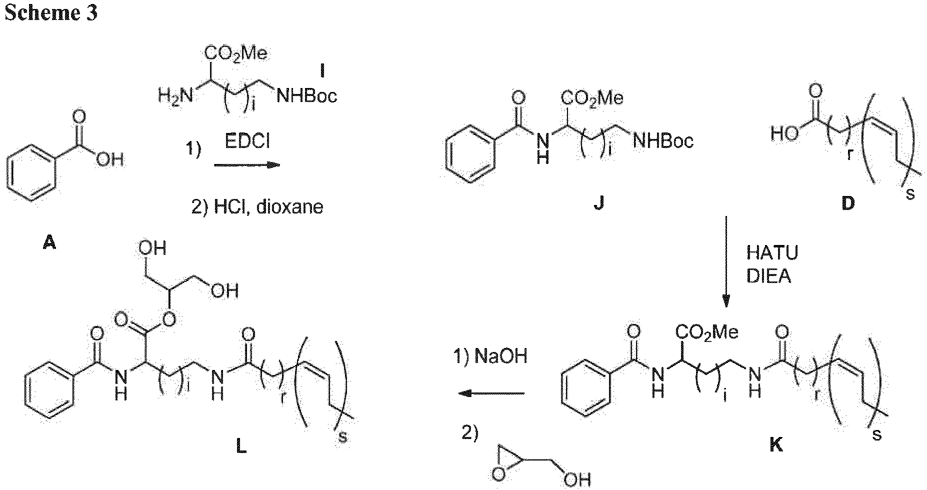 wherein r and s are as defined above.

Compound **A** can be amidated with the corresponding amine **I** (where i = 0, 1, 2 or 3) using a coupling reagent such as DCC, CDI, EDC, or optionally with a tertiary amine base and/or catalyst, e.g., DMAP, followed by deprotection of the BOC group with acids such as TFA or HCl in a solvent such as CH₂Cl₂ or dioxane to produce the coupled compound **J**. Activation of compound **J** with a coupling agent such as HATU in the presence of an amine such as DIEA followed by addition of a fatty acid of formula **D** affords compounds of the formula **K.** Hydrolysis of the ester under basic conditions such as NaOH or LiOH produces the corresponding acid, which can be coupled with glycidol to afford compounds of the formula **L.** 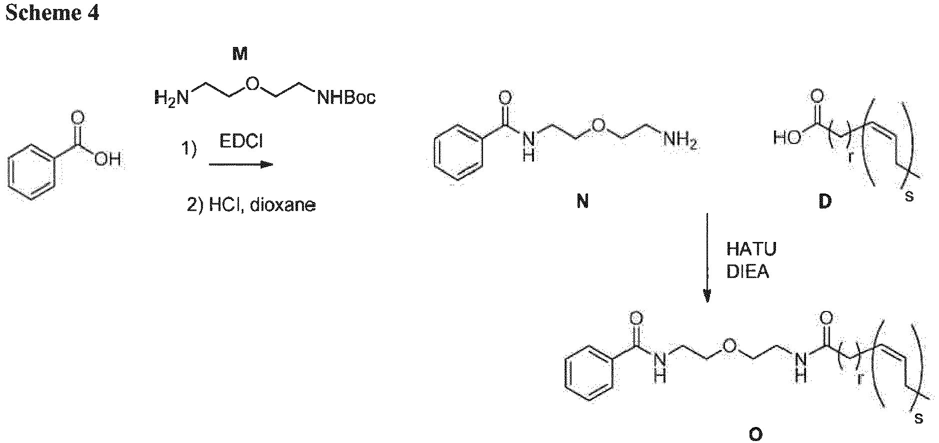 wherein r and s are as defined above.

The amine **M** can be prepared according to the procedures outlined in Dahan et al. J. Org. Chem. 2007, 72, 2289-2296. Compound **A** can be coupled with the amine **M** using a coupling reagent such as DCC, CDI, EDC, or optionally with a tertiary amine base and/or catalyst, e.g., DMAP, followed by deprotection of the BOC group with acids such as TFA or HCl in a solvent such as CH₂Cl₂ or dioxane to produce the coupled compound **N.** Activation of compound **N** with a coupling agent such as HATU in the presence of an amine such as DIEA followed by addition of a fatty acid of formula **D** affords compounds of the formula **O.** 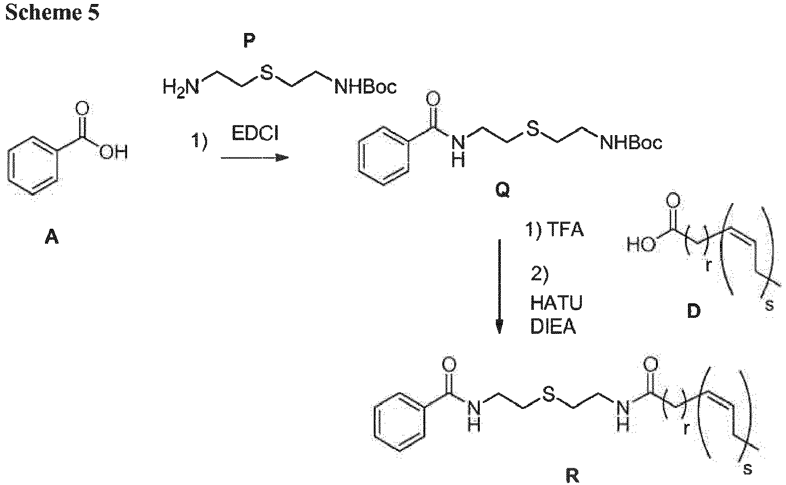 wherein r and s are as defined above.

Compound **A** can be amidated with the commercially available amine **P** using a coupling reagent such as DCC, CDT, EDC, or optionally with a tertiary amine base and/or catalyst, e.g., DMAP, to afford compound **Q.** The BOC group in compound **Q** can be removed with acids such as TFA or HCl in a solvent such as CH₂Cl₂ or dioxane and the resulting amine can be coupled with a fatty acid of formula **D** using a coupling agent such as HATU in the presence of an amine such as DIEA to afford compounds of the formula **R.** To those skilled in the art, the sulfur group in formula **Q** can be oxidized to the corresponding sulfoxide or sulfone using an oxidizing agent such as H₂O₂ or oxone. 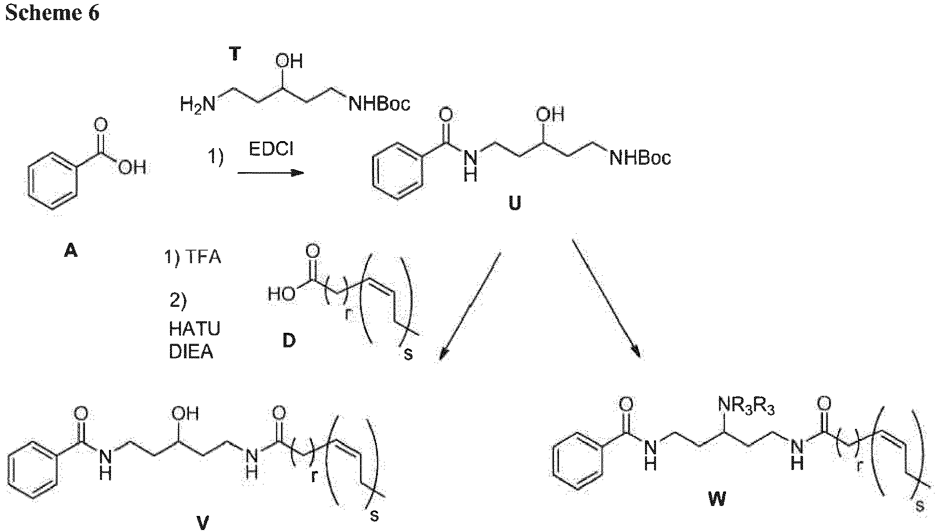 wherein R₃, r, and s are as defined above.

The amine **T** can be prepared from the commercially available diamine according to the procedures outlined in Dahan et al. J. Org. Chem. 2007, 72, 2289-2296. Compound **A** can be amidated with the amine **T** using a coupling reagent such as DCC, CDI, EDC, or optionally with a tertiary amine base and/or catalyst, e.g., DMAP, to afford compound **U.** The BOC group of compound **U** can be removed with acids such as TFA or HCl in a solvent such as CH₂Cl₂ or dioxane and the resulting amine can be coupled with a fatty acid of formula **D** using HATU in the presence of an amine such as DIEA to afford compounds of the formula **V.** To those skilled in the art, the hydroxyl group in compound **U** can be further acylated or converted to an amino group by standard mesylation chemistry followed by displacement with sodium azide and hydrogenation over a catalyst such as Pd/C. The amine can be further acylated or alkylated, followed by the removal of the BOC group. The resulting amine can be coupled with a fatty acid of the formula **D** to afford compounds of the formula **W.** 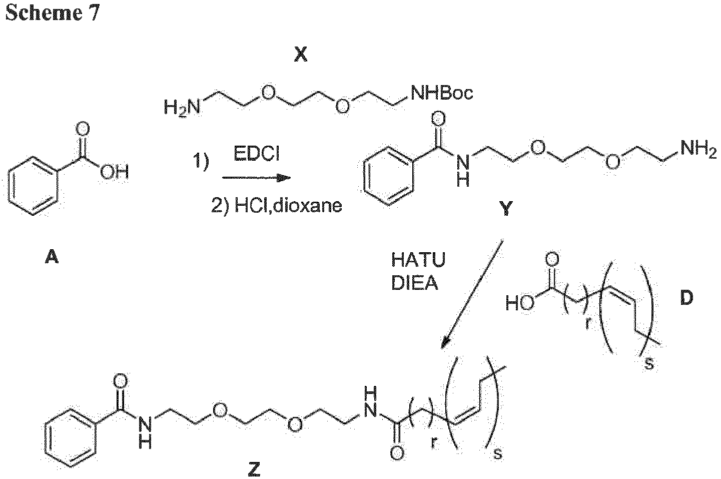 wherein r and s are as defined above.

Compound **A** can be amidated with the commercially available amine **X** using a coupling reagent such as DCC, CDI, EDC, optionally with a tertiary amine base and/or catalyst, e.g., DMAP to afford compound **Y.** The BOC group in compound **Y** can be removed with acids such as TFA or HCl in a solvent such as CH₂Cl₂ or dioxane. The resulting amine can be coupled with a fatty acid of the formula **D** using a coupling agent such as HATU in the presence of an amine such as DIEA to afford compounds of the formula **Z.** 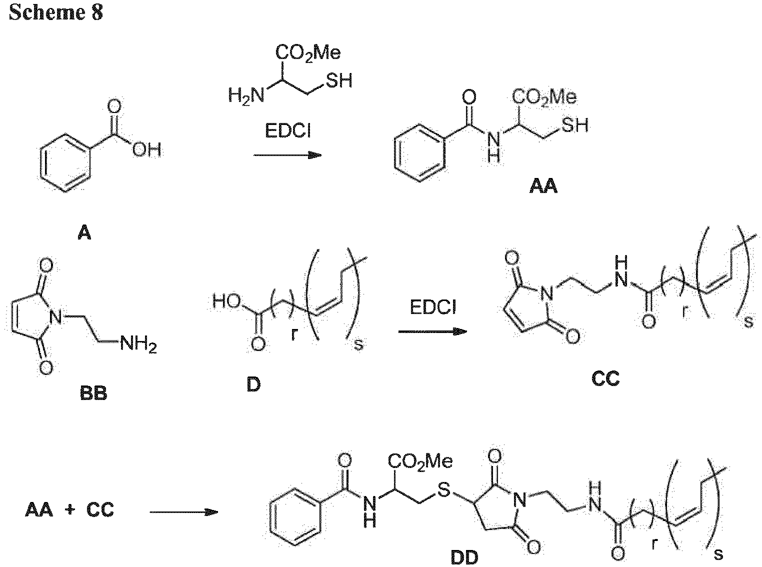 wherein r and s are as defined above.

Compound **A** can be amidated with the commercially available cysteine methyl ester using a coupling reagent such as DCC, CDI, EDC, or optionally with a tertiary amine base and/or catalyst, e.g., DMAP, to afford compound **AA.** The commercially available maleimide derivative **BB** can be coupled with a fatty acid of the formula **D** using a coupling agent such as HATU or EDCI to afford compounds of the formula **CC.** Compound **AA** can be coupled to compounds of the formula **CC** in a solvent such as acetonitrile to afford compounds of the formula **DD.** 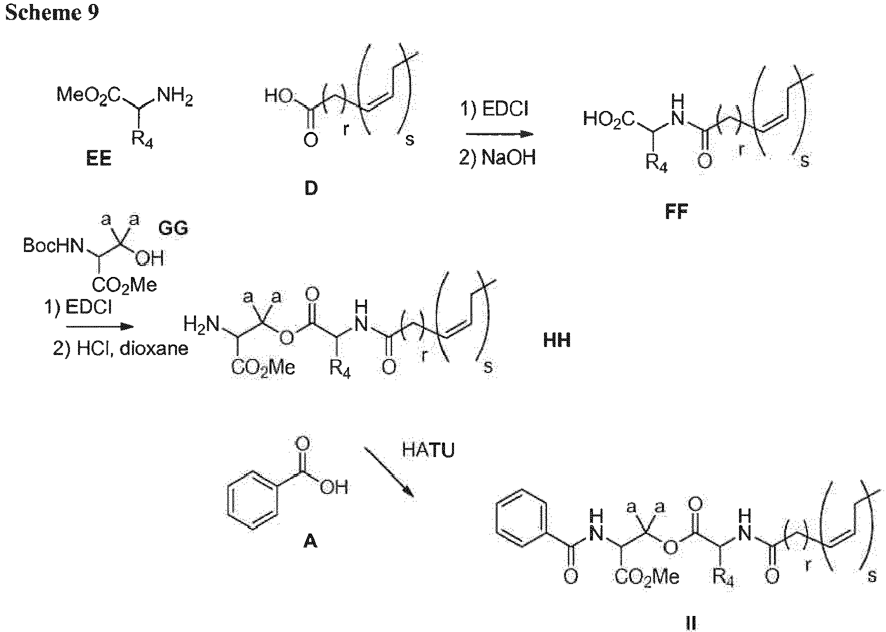 wherein R₄, a, r, and s are as defined above.

The commercially available amino acid esters **EE** can be coupled with a fatty acid of the formula **D** using a coupling agent such as EDCI or HATU, followed by alkaline hydrolysis of the methyl ester to afford compounds of the formula **FF.** Compounds of the formula **FF** can be coupled with the commercially available BOC-amino acid derivatives **GG** using a coupling agent such as EDCI or HATU. The BOC group can be removed by treatment with acids such as TFA or HCl to afford compounds of the formula **HH** which can then be coupled with compound **A** to afford compounds of the formula **II.**

### EXAMPLES

The disclosure is further illustrated by the following examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

### Example 1

### Effect of fatty acid derivatives on ApoA1 and ApoB secretion in HepG2 cells

Niacin has been reported to increase serum levels of HDL to LDL cholesterol *in vivo.* Similarly, niacin has been reported to increase the secretion of **ApoA1** (Jin, F- Y. et al. Arterioscler. Thromb. Vasc. Biol. 1997, 17(10), 2020-2028) while inhibiting the secretion of ApoB (Jin, F- Y. et al. Arterioscler. Thromb. Vasc. Biol. 1999, 19, 1051-1059) in the media supernatants of HcpG2 cultures. Independently, DHA has been demonstrated to lower ApoB as well (Pan, M. et al. J. Clin. Invest. 2004, 113, 1277-1287) by a very different mechanism. Thus, the secretion of ApoA1 and ApoB from HepG2 cells possesses utility as a cell based read-out for niacin-DHA derivative small molecules.

HepG2 cells (ATCC) are seeded at 10,000 cells per well in 96 well plates. After adhering overnight, growth media (10% FBS in DMEM) is removed and cells are serum starved for 24 hours in DMEM containing 0.1% fatty acid free bovine serum albumin (Sigma). Cells are then treated with the compounds at 6 concentrations (2 fold dilutions starting at 100 µM). Niacin at 1.5 mM is used as a positive control. All treatments are performed in triplicate. Simultaneous with compound treatment, ApoB secretion is stimulated with addition of 0.1 oleate complexed to fatty acid free BSA in a 5:1 molar ratio. Incubation with compounds and oleate is conducted for 24 hours. Media supernatants are removed and ApoA1 and ApoB concentrations arc measured using ELISA kits (Mabtcch AB). ApoA1 is expressed as a percent increase over vehicle (0.1% ethanol) treated wells. Percent inhibition of ApoB secretion is determined by normalizing data to vehicle treated wells. For a given compound, an IC₅₀ (concentration at which 50% of ApoB secretion is inhibited) is determined by using a 4 parameter-fit inhibition curve model (Graph Pad Prism®). In each experiment, cell viability is determined using the ATPlite 1-Step kit (Perkin Elmer), such that compound effects due to cytotoxicity can be monitored.

### Example 2

### Effect of the compounds of the invention in the PCSK9 assay

### Cell Culture

HepG2 cells (from ATCC, Catalog no. HB-8065) were maintained in DMEM (Invitrogen) supplemented with 10% fetal bovine serum (Invitrogen). The day prior to the PCSK9 assay, cells are seeded in 96-well collagen coated plates at 25,000 cells/well.

### Compound Preparation

The compounds of the invention were stored at -20°C until used. The test article compound was dissolved in 100% ethanol to a 50mM stock solution. This was then diluted in FBS to a final concentration of 1mM. This solution was placed in a sonicating water bath for 30 minutes. Subsequent dilutions were then made in FBS supplemented with an equivalent volume of ethanol and mixed by vortexing.

### PCSK9 Secretion Assay

HepG2 cells were seeded onto a collagen coated 96-well plate (Becton Dickinson, Catalog no. 35-4407) the day prior to the assay, as described above. The next day, the cell medium was removed, washed once with 100µL serum free DMEM to remove any residual PCSK9, and replaced with 90µL of serum free DMEM. Ten microliters of each compound concentration prepared in FBS was then added. Each concentration of compound was tested in triplicate. The compound was incubated with the cells overnight for 16 hours. Following this incubation, 10µL of AlamarBlue was added to each well and cells incubated for another 2 hours. The plates were then removed and AlamarBlue fluorescence was measured (excitation, 550nm and excitation, 590nm) to assess cell viability. Cell culture supernatant was then diluted 1:5 in 1:5 in 1x RD5P Calibrator Diluent and PCSK9 ELISA was then performed with 50µL of this diluted sample, as per the manufacturer's instructions. The ELISA was measured on a Victor X5 multilabel plate reader (PerkinElmer) at an absorbance of 450nm with background correction measured at 550nm (The PCSK9 Elisa kits can be purchased from R&D System, Catalog no. DPC900).

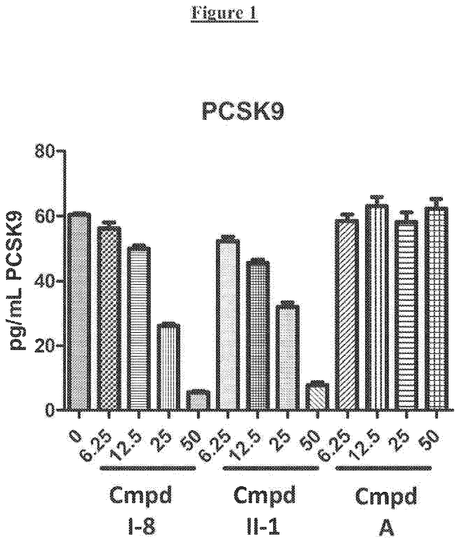

The following 3 compounds were evaluated in this PCSK9 assay: N-(2-((5Z,8Z,11Z,14Z,17Z)-icosa-5,8,11,14,17-pentaenamido)ethyl)nicotinamide **(I-8),** N-(2-((5Z.8Z.11Z,14Z,17Z)-icosa-5,8,11,14,17-pentaenamido)ethyl)benzamide **(II-1)** and (5Z,8Z,11Z,14Z,17Z)-N-(2-acetamidoethyl)icosa-5,8,11,14,17-pentaenamide (Compound A). As summarized in Figure 1, compounds **I-8** and **II-1** were active in this assay and both compounds showed significant lowering of PCSK9 at 25 and 50 µM. Compound A, with a simple acetate group instead of the aryl or heteroaryl group niacin, showed essentially no activity toward PCSK9 at the highest tested concentration of 50 µM.

Alternatively, an IC₅₀ could also be obtained when this type of assay was carried out using at least 6 different concentrations of the test compounds. Table 1 lists the IC₅₀ values for the compounds tested in this assay. In table 1, a ++ value denotes IC₅₀ of < 25 µM; a + value denotes IC₅₀ that is >25 µM but < 50 µM; a - value denotes IC₅₀ that is > 50µM.

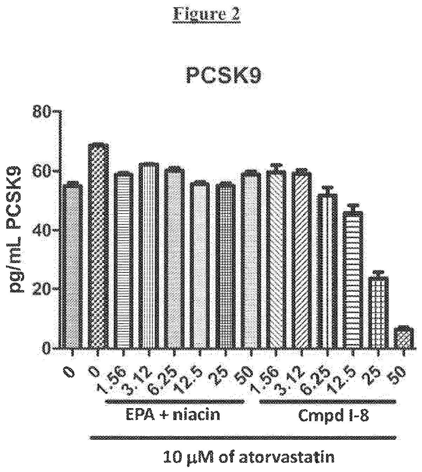

Figure 2 summarizes an experiment that demonstrates the synergy of the fatty acid niacin derivative in the same HepG2 assay. HepG2 cells were incubated with 10 µM of atorvastatin along with either compound **I-8** or a combination of EPA and niacin. As shown in Figure 2, atorvastatin increases PCSK9 secretion. Compound **I-8** decrease PCSK9 levels in a dose response manner to well below the atorvastatin level induced levels. The combination of EPA and niacin did not have a similar effect.

**Table 1. IC₅₀ in the PCSK9 assay**

| **Compound** | **IC₅₀** | **Compound** | **IC₅₀** | **Compound** | **IC₅₀** |
|---|---|---|---|---|---|
| **I-2** | ++ | **I-34** | ++ | **II-39** | ++ |
| **I-7** | ++ | **I-41** | ++ | **V-2** | + |
| **I-8** | ++ | **I-42** | - | **V-8** | ++ |
| **I-13** | + | **I-48** | ++ | **VI-1** | - |
| **I-14** | ++ | **I-49** | + | **VI-2** | + |
| **I-19** | ++ | **I-59** | - | **VI-3** | |
| **I-21** | ++ | **I-60** | | **VI-4** | |
| **I-22** | ++ | **I-61** | - | **VI-5** | |
| **I-23** | ++ | **I-64** | + | **VI-6** | |
| **I-25** | + | **II-1** | + | **VI-7** | ++ |
| **I-26** | + | **II-7** | - | **VII-1** | ++ |
| **I-27** | ++ | **II-8** | - | **VII-2** | ++ |
| **I-28** | ++ | **II**-**9** | + | **VII**-**3** | ++ |
| **I-29** | ++ | **II-10** | - | **VII-4** | ++ |
| **I-30** | + | **II-11** | - | **VII-5** | ++ |
| **I-31** | ++ | **II-33** | ++ | **VII-6** | ++ |
| **I-32** | ++ | **II-34** | ++ | **VII-7** | ++ |
| **I-33** | ++ | **II-36** | ++ | **VII-8** | ++ |

| **Compound** | **IC₅₀** | **Compound** | **IC₅₀** | **Compound** | **IC₅₀** |
|---|---|---|---|---|---|
| **VII-9** | + | **VII-16** | + | **VII-24** | - |
| **VII-10** | ++ | **VII-17** | ++ | **VII-25** | ++ |
| **VII-11** | - | **VII-19** | ++ | **VII-41** | + |
| **VII-12** | - | **VII-20** | ++ | **VII-42** | ++ |
| **VII-13** | ++ | **VII-21** | ++ | **III-1** | - |
| **VII-14** | + | **VII-22** | ++ | **III-2** | - |
| **VII-15** | ++ | **VII-23** | ++ | **III-3** | ++ |

### Example 3

### The effect of lowering plasma triglycerides after a high fat meal

In this experiment, healthy human volunteers are divided into 4 treatment groups. The first treatment group is a placebo group (n = 6). The other three groups consists of the test compound, a fatty acid niacin conjugate, administered as a single oral dose at either 300 mg (n = 6), 1000 mg (n = 7) or 2000 mg (n = 4). All subjects are given an NIH high fat breakfast in order to induce an elevated level of triglycerides (In a typical NIH high fat breakfast, 450 calories are derived from fat). The test compound is then administered as a single oral dose at the three indicated doses at three different time points: immediately following the high fat meal, 2 hours following the high fat meal and 4 hours following the high fat meal. At each of the time points, plasma triglyceride levels can be determined according to standard protocols. Test compound that lowers the plasma triglyceride level at these various time points is useful for the treatment of type I hyperlipoproteinemia and type 5 hyperlipoproteinemia.

### Examples 4

### The effect of the compounds of the invention on the plasma triglyceride level of the Zucker fa/fa Rat Model of Dyslipidemia

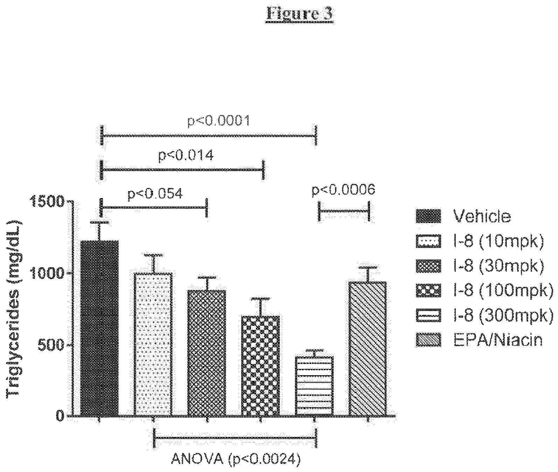

Male Zucker rats (*HsdHlr: Zucker- Lepr*^*fa*) between 8-10 weeks of age were purchased from Harlan and maintained on Purina Rodent Diet (5001) for the duration of the study. Animals were randomized and assigned to treatment arms based on body weight and plasma triglyceride (TG) levels (n = 8). Inclusion criteria for the study include body weight > 300 grams and fed TG levels in plasma > 800 mg/dL. Dosing will be initiated on day 1 and continue through day 5. Dosing will be daily (qd) by oral gavage (po) for all treatment arms (Compound **I-8** was administered orally at 4 different doses, 10, 30, 100 and 300 mg/kg; in addition, a combination of niacin/EPA in a ratio of 100/200 mg/kg was also employed). Body weights will be measured for all rats on days 1 through 5. On day 4, a blood sample (fed) will be collected from each rat, processed for plasma and stored at -80°C. Plasma triglyceride level was determined from commercial kits using standard protocols. Figure 3 showed the dose dependent decrease of fed plasma triglyceride level upon oral administration of compound **I-8.** As shown in figure 3, this effect could not be replicated by a simple combination of niacin and EPA. Because compound **I-8** was able to lower fed plasma triglyceride, it is useful to treat dyslipidemia as well as other diseases such as type I hyperlipoproteinemia and type 5 hyperlipoproteinemia.

### Example 5

### The effect of a combination of compound I-8 and atorvastatin on plasma cholesterol and other lipids in ApoE3Leiden mice.

The study was conducted using female APOE*3Leiden mice (groups of each n=10) and one untreated reference control group on chow (n=5). To induce dyslipidemia, a high cholesterol Western type diet containing 1% cholesterol, 15% cacao butter, 40.5% sucrose and 1% corn oil (WTD) was fed to the mice for a total experimental period of 20 weeks (of which 4 weeks are a run-in period). To prevent oxidation of the test compound (**I-8**), 30 mg/kg alpha-tocopherol was added to the high cholesterol diets, i.e. also in the high cholesterol diet control.

In the first 4 weeks (run-in period), a pro-atherogenic state of dyslipidemia characterized by elevated plasma cholesterol levels (about 15-20 mM) was induced in all mice by feeding them an atherogenic diet containing 1% cholesterol. The mice were then separated into a control group (no treatment) and three treatment groups: i) compound **I-8**, ii) atorvastatin and iii) compound **I-8**+atorvastatin as as described below. The dyslipidemic mice were grouped on the basis of plasma cholesterol at t=0 assayed in 4h fasting blood. Mice with low cholesterol after the run-in period were excluded so that homogenous experimental groups were obtained. A group of reference mice (n=5) remained on a chow diet during the complete study period (normolipidemic reference mice).

The doses of the test compounds were as follows:
- Compound **I-8:** 0.75% w/w in diet.
- Atorvastatin: 0.0015% w/w in diet (to achieve about 20% reduction in plasma cholesterol).
- Alpha-tocopherol: 0.0030% w/w in diet

The test compounds, sufficient for approx. 3 kg of diet (i.e. 25 g of compound **I-8**), and alpha-tocopherol (>200 mg) were formulated before the start of the treatment period (t=0), by adding the test compounds to melted, hand warm cocoa butter and mixed for 5 min. This mix was then added to the master mix (containing the rest of the ingredients) and mixed thoroughly. The diet was frozen to -20°C. On the subsequent day, the diet was broken into small pellets (approx 5 g per piece) and freeze dried, and stored in vacuum scaled bags (approx 500g) at -20°C until use. The diets were refreshed daily and unused diet was discarded.

The following parameters were taken at the indicated timepoints (individually unless mentioned otherwise):
1) Body weight at -4, 0, 2, 4 weeks
2) Food intake (g/day/mouse) at 0, 2, 4 weeks (per cage)
3) Plasma total cholesterol at -4, 0, 2, 4 weeks (individually)
4) Plasma triglycerides at -4, 0, 2, 4 weeks (individually)
5) Lipoprotein profiles at 0 (pool of all animals) and 4 weeks (cholesterol distribution over VLDL, LDL and HDL-sized particles, analysis on group level).

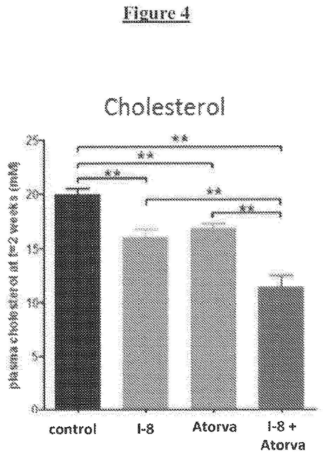

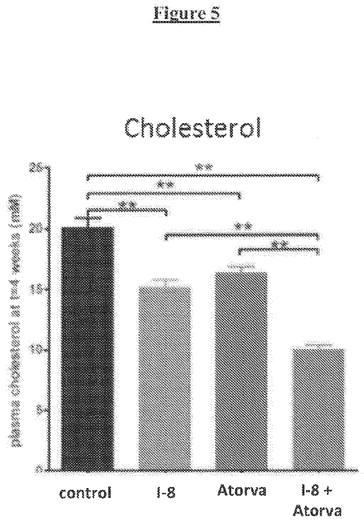

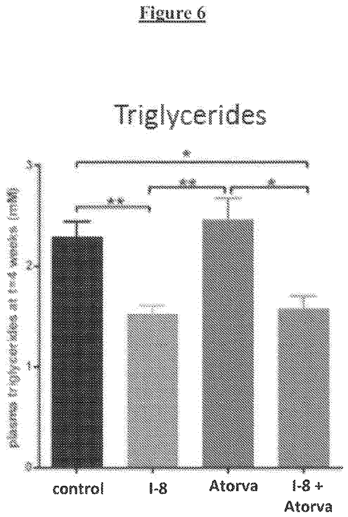

EDTA plasma was collected in weeks -4, 0, 2 and 4 weeks. Plasma cholesterol, plasma triglyceride levels and lipoprotein profileswere assayed immediately in fresh plasma. Figure 4 shows the cholesterol level at t = 2 weeks of treatment between the control group, the group treated with compound **I-8,** the group treated with atorvastatin, and the group treated with a combination of compound **I-8** and atorvastatin. There was a statistically significant reduction of plasma cholesterol at t = 2 weeks for groups treated with either compound **I-8** and atorvastatin. The group treated with the combination of compound **I-8** and atorvastatin showed a more substantial decrease in plasma cholesterol. Figures 5 and 6 show the plasma cholesterol and triglyceride levels respectively after 4 weeks of treatment. As shown in Figure 5, the reduction in plasma cholesterol level was maintained after 4 weeks of treatment across all treatment groups. Comparable level of cholesterol reduction was observed in groups treated with either compound **1-8** or atorvastatin. A significant reduction in plasma cholesterol was observed in the groups treated with a combination of compound **I-8** and atorvastatin.

Figure 6 shows the corresponding plasma triglyceride levels in the same treatment groups after 4 weeks of treatment. ApoE*3 Leiden mice treated with compound **I-8** showed a significant reduction in triglycerides after 4 weeks of treatment. In sharp contrast, ApoE*3 Leiden mice treated with atorvastatin failed to show a statistically significant change triglyceride level after 4 weeks of treatment. ApoE*3 Leiden mice treated with a combination of compound **I-8** and atorvastatin showed a significant reduction in plasma triglycerides after 4 weeks of treatment.

### Examples 7

### The effect administering compound I-8 on liver weight of ApoE*3 Leiden mice

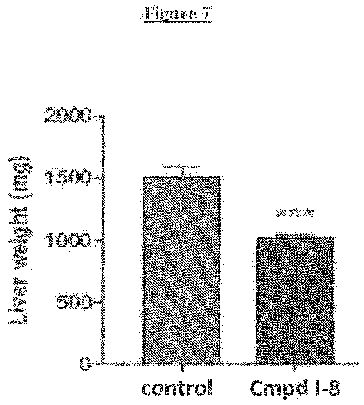

The same experimental design used in example 6 was used. Two treatment groups were used (n = 15). The control animals were kept on a Western type diet (WTD) consisting of 1% cholesterol, 15% cacao butter, 40.5% sucrose, and 1% corn oil. For the treatment group, compound **I-8** was administered in the above WTD at a ratio of 7.5 g/kg. Animals were maintained on the WTD or treatment group for 16 weeks. At the conclusion of the study, plasma cholesterol and triglyceride levels were recorded, as well as the weight of the liver. There was a significant decrease in plasma cholesterol, triglyceride levels as well as liver weight in the treatment group. After 16 weeks of treatment, the cholesterol level of the treatment group was 420 mg/dL, compared with a level of 750 mg/dL for the control group; the triglyceride level was 110 mg/dL, compared with a level of 160 mg/dL for the control group. The statistically significant drop in liver weight is shown in Figure 7.

### Compounds

The following non-limiting compound examples serve to illustrate further embodiments of the fatty acid niacin derivatives. It is to be understood that any embodiments listed in the Examples section are embodiments of the fatty acid niacin derivatives and, as such, are suitable for use in the methods and compositions described above.

### Example 8

### Preparation of N-(2-(4Z,7Z,10Z,13Z,16Z,19Z)-docosa-4,7,10,13,16,19-hexaenamidoethyl)nicotinamide (I-7)

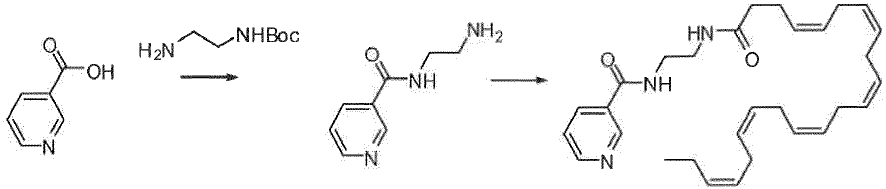

In a typical run, nicotinic acid (2.0 g, 16.2 mmol) was taken up in CH₂Cl₂ (20 mL) along with oxalyl chloride (1.4 mL, 16.2 mmol). After a few drops of DMF were added, the reaction mixture was stirred at room temperature until all the solids had dissolved and all gas evolution had ceased (1 h). This freshly prepared solution of the acid chloride was added dropwise at 0 °C to a solution containing *tert*-butyl 2-aminoethylcarbamate (2.6 g, 16.2 mmol) and Et₃N (3.4 mL, 24.2 mmol) in CH₂Cl₂ (200 mL). The resulting reaction mixture was warmed to room temperature and stirred for 2 h. It was then washed with brine, dried over Na₂SO₄, filtered and concentrated under reduced pressure. Purification by silica gel chromatography (CH₂Cl₂) afforded *tert*-butyl 2-(nicotinamido)ethylcarbamate (3.1 g, 74%).

*tert*-Butyl 2-(nicotinamido)ethylcarbamate (3.1 g, 11.7 mmol) was taken up in 25% TFA in CH₂Cl₂ (10 mL). The resulting reaction mixture was allowed to stand at room temperature for 1 h. At this point, a considerable amount of precipitate formed and the clear filtrate was removed. The remaining solids were dried to afford of the TFA salt of *N*-(2-aminoethyl)nicotinamide (1.6 g).

The TFA salt of *N*-(2-aminoethyl)nicotinamide (5.0 mmol) was taken up in CH₃CN (20 mL) along with (4Z,7Z,10Z,13Z,16Z,19Z)-docosa-4,7,10,13,16,19-hexaenoic acid (5.0 mmol), HATU (5.5 mmol) and DIEA (15 mmol). The resulting reaction mixture was stirred at room temperature for 2 h and diluted with EtOAc. The organic layer was washed with saturated aqueous NaHCO₃, brine, dried over Na₂SO₄, filtered and concentrated under reduced pressure. Purification by silica gel chromatography (5% MeOH-CH₂Cl₂) afforded *N*-(2-(4Z,7Z,10Z,13Z,16Z,19Z)-docosa-4,7,10,13,16,19-hexaenamidoethyl)nicotinamide. MS calculated for C₃₀H₄₁N_{A}O₂: 475.32; found: [M+H]⁺ 476.

### Example 9

### Preparation of N-(2-(5Z,8Z,11Z,14Z,17Z)-eicosa-5,8,11,14,17-pentaenamidoethyl)nicotinamide (I-8)

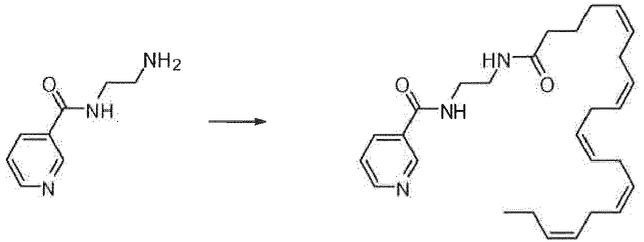

The TFA salt of *N*-(2-aminoethyl)nicotinamide (1.6 g, 5.7 mmol) was taken up in CH₃CN (15 mL) along with (5Z,8Z,11Z,14Z,17Z)-elcosa-5,8,11,14,17-pentaenoic acid (1.7 g, 5.7 mmol), HATU (2.4 g, 6.3 mmol) and DIEA (3 mL, 17 mmol). The resulting reaction mixture was stirred at room temperature for 2 h and diluted with EtOAc. The organic layer was washed with saturated aqueous NaHCO₃, brine, dried over Na₂SO₄, filtered and concentrated under reduced pressure. Purification by silica gel chromatography (5% MeOH-CH₂Cl₂) afforded *N*-(2-(5Z,8Z,11Z,14Z,17Z)-eicosa-5,8,11,14,17-pentaenamidoethyl)nicotinamide (1.6 g, 62%). MS calculated for C₂₈H₃₉N₃O₂: 449.3; found: [M+H]⁺ 450.

### Example 10

### Preparation of N-(2-(2-(2-(4Z,7Z,10Z,13Z,16Z,19Z)-docosa-4,7,10,13,16,19-hexaenamidoethyl)disulfanyl)ethyl)nicotinamide (I-3)

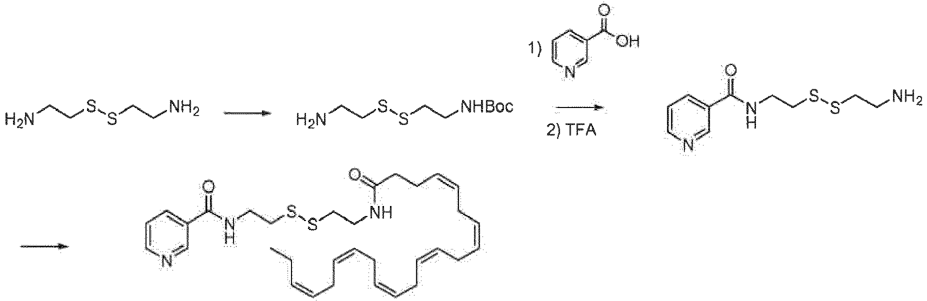

Cystamine dihydrochloride (1.0 g, 4.44 mmol) was dissolved in McOH (50 mL). Triethylamine (1.85 mL, 3 eq) was added at room temperature, followed by dropwise addition of Boc₂O (0.97 g, 4.44 mmol) as a solution in MeOH (5 mL). The resulting reaction mixture was stirred at room temperature for 3 h. It was then concentrated under reduced pressure and the resulting residue was taken up in 1M aqueous NaH₂PO₄ (20 mL). The aqueous layer was washed with a 1:1 solution of pentane/EtOAc (10 mL), basified to pH 9 with 1M aqueous NaOH, and extracted with EtOAc. The combined organic layers were washed with brine, dried over Na₂SO₄, filtered and concentrated under reduced pressure to afford *tert*-butyl 2-(2-(2-aminoethyl)disulfanyl)ethylcarbamate (500 mg, 44 %).

Separately, nicotinic acid (246 mg, 2.0 mmol) was taken up in CH₃CN (10 mL) along with *tert*-butyl 2-(2-(2-aminoethyl)disulfanyl)ethylcarbamate (503 mg, 2.0 mmol), EDCI (422 mg, 2.2 mmol). The resulting reaction mixture was stirred at room temperature for 4 h and then diluted with EtOAc. The organic layer was washed with dilute aqueous NaHCO₃, brine, dried over Na₂SO₄, filtered and concentrated under reduced pressure. Purification by silica gel chromatography (CH₂Cl₂) afforded *tert*-butyl 2-(2-(2-(nicotinamido)ethyl)disulfanyl)ethylcarbamate (400 mg, 56%).

*tert*-Butyl 2-(2-(2-(nicotinamido)ethyl)disulfanyl)ethylcarbamate (200 mg, 0.56 mmol) was taken up in 25% TFA in CH₂Cl₂ solution (5 mL) and allowed to stand at room temperature for 4 h. The reaction mixture was then concentrated under reduced pressure to afford the TFA salt of *N*-(2-(2-(2-aminoethyl)disulfanyl)ethyl)nicotinamide, This material was taken up in CH₃CN (10 mL) along with (4Z,7Z,10Z,13Z,16Z,19Z)-docosa-4,7,10,13,16,19-hexaenoic acid (184 mg, 0.56 mmol), HATU (234 mg, 0.62 mmol) and DIEA (0.30 mL). The resulting reaction mixture was stirred at room temperature for 2 h. It was then diluted with EtOAc and washed successively with saturated aqueous NaHCO₃ and brine. The organic layer was dried over Na₂SO₄, filtered and concentrated under reduced pressure. Purification by silica gel chromatography (5% MeOH-CH₂Cl₂) afforded (*N*-(2-(2-(2-(4Z,7Z,10Z,13Z,16Z,19Z)-docosa-4,7,10,13,16,19-hexacnamidoethyl)disulfanyl)ethyl)nicotinamide (300 mg, 86%). MS calculated for C₃₂H₄₅N₃O₂S₂: 567.3; found: [M+H]⁺ 568.

### Example 11

### Preparation of N-(2-(2-(4Z,7Z,10Z,13Z,16Z,19Z)-docosa-4,7,10,13,16,19-hexaenamidoethoxy)ethyl)nicotinamide (I-1)

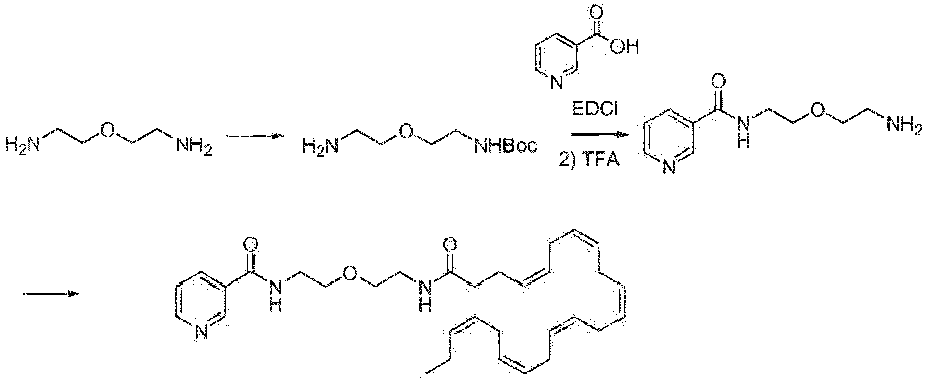

In a typical run, sodium hydroxide (400 mg, 10 mmol) was dissolved in McOH (70 mL) and 2-(2-aminoethoxy)ethanamine dihydrochloride (1.0 g, 5.65 mmol) was added. The resulting reaction mixture was stirred at room temperature for 30 min. A solution containing BoC₂O (740 mg, 3.40 mmol) in THF (15 mL) was then added dropwise, at room temperature, over a period of 15 min. The resulting reaction mixture was stirred at room temperature for 18 h. It was then concentrated under reduced pressure. The resulting residue was taken up in CH₂Cl₂ (200 mL) and stirred vigorously at room temperature for 4 h. The mixture was filtered and the filtrate was concentrated under reduced pressure to afford *tert-*butyl 2-(2-aminoethoxy)ethylcarbamate (850 mg, 74%).

*tert*-Butyl 2-(2-aminoethoxy)ethylcarbamate (420, 2.06 mmol) was then taken up in CH₃CN (20 mL) along with nicotinic acid (253 mg, 2.06 mmol) and EDCI (434 mg, 2.3 mmol). The resulting reaction mixture was stirred at room temperature for 18 h. It was then diluted with EtOAc (20 mL), washed with saturated aqueous NaHCO₃, brine, dried over Na₂SO₄, filtered and concentrated under reduced pressure. The resulting residue was purified by silica gel chromatography (9:1 CH₂Cl₂/MeOH) to afford *tert*-butyl 2-(2-(nicotinamido)ethoxy)ethylcarbamate (280 mg, 44%). MS calculated for C₁₅H₂₃N₃O₄: 309.17; found: [M+H]⁺ 310.

*tert*-Butyl 2-(2-(nicotinamido)ethoxy)ethylcarbamate (140 mg, 0.453 mmol) was taken up in 25% TFA in CH₂Cl₂ (10 mL). The reaction mixture was allowed to stand at room temperature for 2 h and then concentrated under reduced pressure to afford the TFA salt of *N-*(2-(2-aminoethoxy)ethyl)nicotinamide. This material was then taken up in CH₃CN (10 mL) along with (4Z,7Z,10Z,13Z,16Z,19Z)-docosa-4,7,10,13,16,19-hexaenoic acid (148 mg, 0.453 mmol), HATU (190 mg, 0.498 mmol) and DIEA (0.24 mL). The resulting reaction mixture was stirred at room temperature for 2 h. It was then diluted with EtOAc and washed successively with saturated aqueous NaHCO₃ and brine. The organic layer was dried over Na₂SO₄, filtered and concentrated under reduced pressure. Purification by silica gel chromatography (9:1 CH₂Cl₂/MeOH) afforded *N*-(2-(2-(4Z,7Z,10Z,13Z,16Z,19Z)-docosa-4,7,10,13,16,19-hexaenamidoethoxy)ethyl)nicotinamide (75 mg, 31%). MS calculated for C₃₁H₄₆N₂O₅: 526.34; found: [M+H]⁺ 527.

### Example 12

### Preparation of N-(2-((2-(4Z,7Z,10Z,13Z,16Z,19Z)-docosa-4,7,10,13,16,19-hexaenamidoethyl)(methyl)amino)ethyl)nicotinamide (I-2)

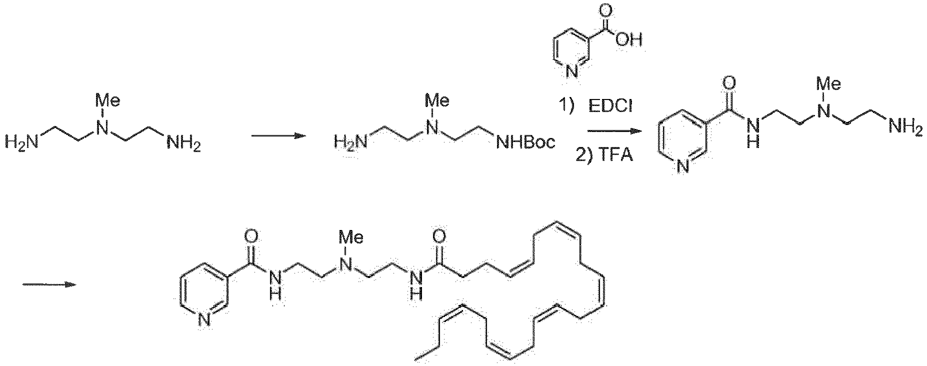

*N*1-(2-Aminoethyl)-*N*1-methylethane-1,2-diamine (5.0 g, 42.7 mmol) was dissolved in CH₂Cl₂ (100 mL) and cooled to 0 °C. A solution of Boc₂O (0.93 g, 4.27 mmol) in CH₂Cl₂ (10 mL) was then added dropwise at 0 °C over a period of 15 min. The resulting reaction mixture was stirred at 0 °C for 30 min and then warmed to room temperature. After stirring at room temperature for 2 h, the reaction mixture was diluted with CH₂Cl₂ (100 mL). The organic layer was washed with brine (3 x 25 mL), dried over Na₂SO₄, filtered and concentrated under reduced pressure to afford *tert*-butyl 2-((2-aminoethyl)(methyl)amino)ethylcarbamate (1.1 g).

*tert*-Butyl 2-((2-aminoethyl)(methyl)amino)ethylcarbamate (400 mg, 1.84 mmol) was taken up in CH₃CN (10 mL) along with nicotinic acid (227 mg, 1.84 mmol) and EDCI (353 mg, 2.02 mmol). The resulting reaction mixture was stirred at room temperature for 18 h and then diluted with EtOAc. The organic layer was washed with saturated aqueous NaHCO₃, brine, dried over Na₂SO₄, filtered and concentrated under reduced pressure. The resulting residue was purified by silica gel chromatography (5% MeOH-CH₂Cl₂) to afford *tert*-butyl 2-(methyl(2-(nicotinamido)ethyl)amino)ethylcarbamate (180 mg, 30%). MS calculated for C₁₆H₂₆N₄O₃: 322.2; found: [M+H]⁺ 323.

*tert*-Butyl 2-(methyl(2-(nicotinamido)ethyl)amino)ethylcarbamate (90 mg, 0.279 mmol) was taken up in a 25% TFA in CH₂Cl₂ solution (5 mL) and allowed to stand at room temperature for 3 h. The reaction mixture was concentrated under reduced pressure to afford the TFA salt of *N*-(2-((2-aminoethyl)(methyl)amino)ethyl)nicotinamide. This material was taken up in CH₃CN (10 mL) along with (4Z,7Z,10Z,13Z,16Z,19Z)-docosa-4,7,10,13,16,19-hexaenoic acid (90 mg, 0.279 mmol), HATU (117 mg, 0.31 mmol) and DIEA (0.15 mL). The resulting reaction mixture was stirred at room temperature for 2 h. It was then diluted with EtOAc and washed successively with saturated aqueous NaHCO₃ and brine. The organic layer was dried over Na₂SO₄, filtered and concentrated under reduced pressure. Purification by silica gel chromatography (5% MeOH-CH₂Cl₂) afforded *N*-(2-((2-(4Z,7Z,10Z,13Z,16Z,19Z)-docosa-4,7,10,13,16,19-hexaenamidoethyl)(methyl)amino)ethyl)nicotinamide (30 mg, 20%). MS calculated for C₃₃H₄₈N₄O₂: 532.38; found: [M+H]⁺ 533.

### Example 13

### Preparation of (2S,3R)-methyl 3-((S)-2-((4Z,7Z,10Z,13Z,16Z,19Z)-docosa-4,7,10,13,16,19-hexaenamido)propanoyloxy)-2-(nicotinamido)butanoate (I-9)

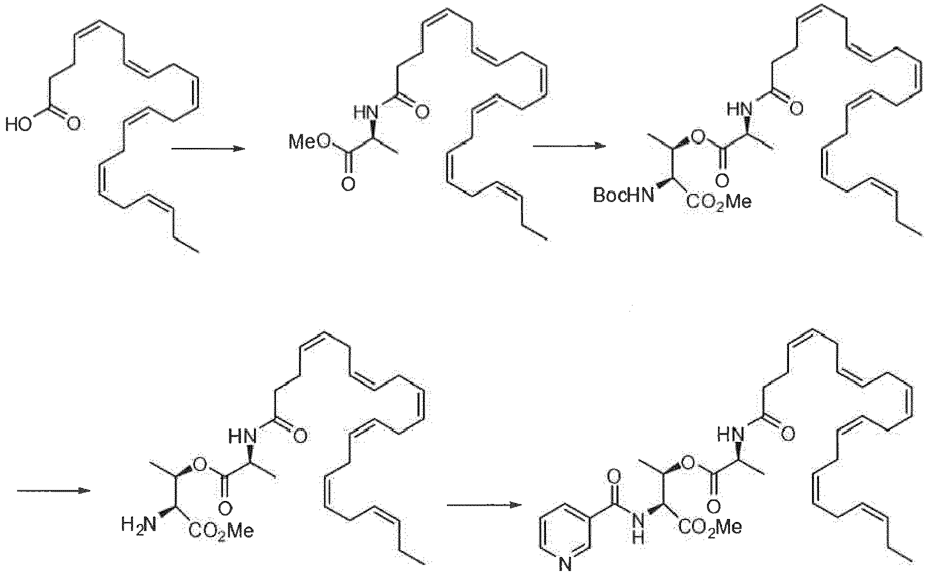

L-Alanine methyl ester hydrochloride (0.85 g, 6.1 mmol) was taken up in CH₃CN (20 mL) along with (4Z,7Z,10Z,13Z,16Z,19Z)-docosa-4,7,10,13,16,19-hexaenoic acid (2.0 g, 6.1 mmol), EDCI (1.3 g, 6.72 mmol) and DIEA (1.3 mL). The resulting reaction mixture was stirred at room temperature for 2 h. It was then diluted with EtOAc and washed with dilute aqueous NaHCO₃ and brine. The organic layer was dried over Na₂SO₄, filtered and concentrated under reduced pressure to afford (*S*)-methyl 2-((4Z,7Z,10Z,13Z,16Z,19Z)-docosa-4,7,10,13,16,19-hexaenamido)propanoate (2.0 g, 79%).

(*S*)-methyl 2-((4Z,7Z,10Z,13Z,16Z,19Z)-docosa-4,7,10,13,16,19-hexaenamido)propanoate (2.0 g, 4.8 mmol) was taken up in THF (8 mL) along with 5M aqueous NaOH (5 mL) and stirred vigorously at room temperature for 3 h. The reaction mixture was diluted with water and concentrated under reduced pressure. Enough 6N HCl was then added to adjust the pH to 2. The resulting mixture was extracted with EtOAc. The combined organic layers were dried over Na₂SO₄, filtered and concentrated under reduced pressure to afford (*S*)-2-((4Z,7Z,10Z,13Z,16Z,19Z)-docosa-4,7,10,13,16,19-hexaenamido)propanoic acid. This was taken up in CH₃CN (15 mL) along with *N*-Boc-L-threonine methyl ester (1.11 g, 4.78 mmol), HATU (2.0 g, 5.3 mmol) and DIEA (1.2 mL). The resulting reaction mixture was stirred at room temperature for 6 h and diluted with EtOAc. The organic layer was washed with NaHCO₃, brine, dried over Na₂SO₄, filtered and concentrated under reduced pressure. Purification by silica gel chromatography (CH₂Cl₂) afforded (2*S*,3*R*)-methyl 2-(*tert*-butoxycarbonyl)-3-((*S*)-2-((4Z,7Z,10Z,13Z,16Z,19Z)-docosa-4,7,10,13,16,19-hexaenamido)propanoyloxy)butanoate (1.0 g).

(2*S*,3*R*)-methyl 2-(*tert*-butoxycarbonyl)-3-((*S*)-2-((4Z,7Z, 10Z, 13Z, 16Z, 19Z)-docosa-4,7,10,13,16,19-hexaenamido)propanoyloxy)butanoate (300 mg, 0.488 mmol) was taken up in 4M HCl in dioxane (2 mL) and allowed to stand at room temperature for 10 min. The reaction mixture was then diluted with EtOAc and concentrated under reduced pressure to afford the HCl salt of (2*S*,3*R*)-methyl 2-amino-3-((*S*)-2-((4Z,7Z,10Z,13Z,16Z,19Z)-docosa-4,7,10,13,16,19-hexaenamido)propanoyloxy)butanoate. This material was taken up in CH₃CN (5 mL) along with nicotinic acid (60 mg, 0.488 mmol), HATU (204 mg, 0.54 mmol) and DIEA (0.25 mL, 1.46 mmol). The resulting reaction mixture was stirred at room temperature for 1 h and concentrated under reduced pressure. The resulting oily residue was purified by silica gel chromatography (9:1 CH₂Cl₂/MeOH) to afford (2*S*,3*R*)-methyl 3-((*S*)-2-((4Z,7Z,10Z,13Z,16Z,19Z)-docosa-4,7,10,13,16,19-hexaenamido)propanoyloxy)-2-(nicotinamido)butanoate (120 mg, 40%). MS calculated for C₃₆H₄₉N₃O₆: 619.36; found: [M+H]¹ 620.

### Example 14

### Preparation of (2S,3R)-methyl 3-((S)-2-((5Z,8Z,11Z,14Z,17Z)-icosa-5,8,11,14,17-pentaenamido)propanoyloxy)-2-(nicotinamido)butanoate (I-10)

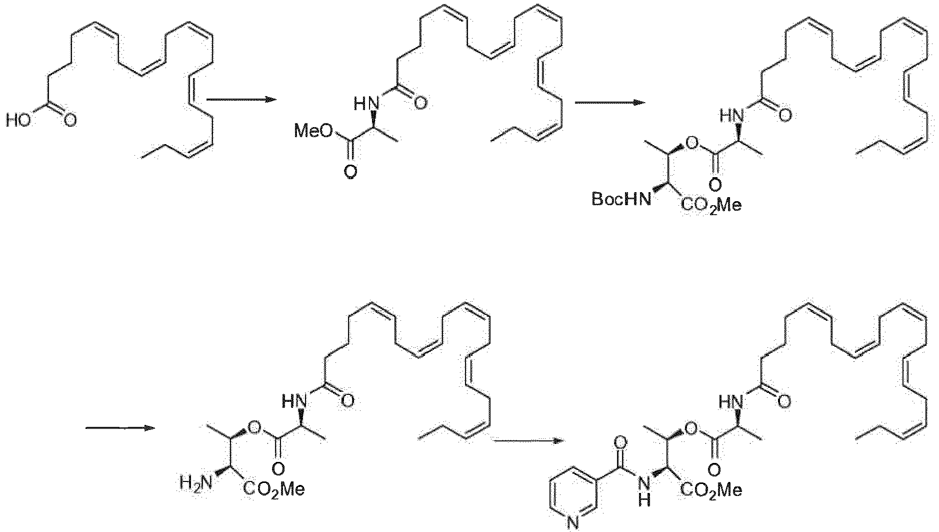

The same synthetic sequence outlined above for the preparation of (2*S*,3*R*)-methyl 3-((*S*)-2-((4Z,7Z,10Z,13Z,16Z,19Z)-docosa-4,7,10,13,16,19-hexaenamido)propanoyloxy)-2-(nicotinamido)butanoate was used, except that (5Z,8Z,11Z,14Z,17Z)-eicosa-5,8,11,14,17-pentaenoic acid (EPA) was used instead of DHA. MS calculated for C₃₄H₄₇N₃O₆: 593.35; found: [M+H]⁺ 594.

### Example 15

### Preparation of (S)-methyl 6-((4Z,7Z,10Z,13Z,16Z,19Z)-docosa-4,7,10,13,16,19-hexaenamido)-2-(nicotinamido)hexanoate (I-11)

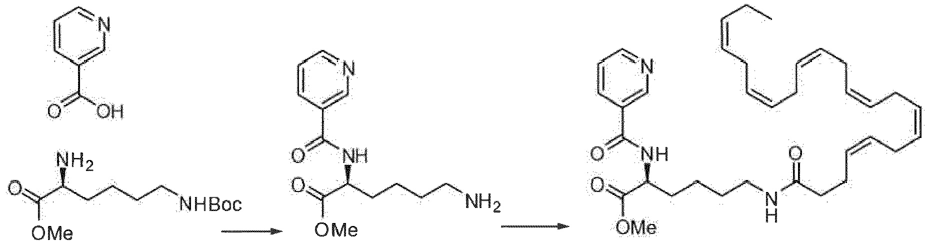

H-Lysine-(BOC)-OMe hydrochloride (500 mg, 1.68 mmol) was taken up in CH₃CN (10 mL) along with nicotinic acid (207 mg, 1.68 mmol), EDCI (354 mg, 1.85 mmol) and DIEA (0.90 mL). The resulting reaction mixture was stirred at room temperature for 18 h and diluted with EtOAc. The organic layer was washed with dilute aqueous NaHCO_{3,} brine, dried over Na₂SO₄, filtered and concentrated under reduced pressure. Purification by silica gel chromatography (CH₂Cl₂) afforded (*S*)-methyl, 6-(*tert*-butoxycarbonyl)-2-(nicotinamido)hexanoate (520 mg, 85%).

(*S*)-Methyl 6-(*tert*-butoxycarbonyl)-2-(nicotinamido)hexanoate (260 mg, 0.71 mmol) was taken up in 4M HCl in dioxane (2 mL) and allowed to stand at room temperature for 1 h. The reaction mixture was diluted with EtOAc and concentrated under reduced pressure to afford the HCl salt of (*S*)-methyl 6-amino-2-(nicotinamido)hexanoate. This material was taken up in CH₃CN (5 mL) along with (4Z,7Z,10Z,13Z,16Z,19Z)-docosa4,7,10,13,16,19-hexaenoic acid (233 mg, 0.71 mmol), HATU (297 mg, 0.78 mmol) and DIEA (0.4 mL). The resulting reaction mixture was stirred at room temperature for 2 h and diluted with EtOAc. The organic layer was washed with dilute aqueous NaHCO₃, brine, dried over Na₂SO₄, filtered and concentrated under reduced pressure. Purification by silica gel chromatography (9:1 CH₂Cl₂/McOH) afforded (*S*)-methyl 6-((4Z,7Z,10Z,13Z,16Z,19Z)-docosa-4,7,10,13,16,19-hexaenamido)-2-(nicotinamido)hexanoate (280 mg, 72%). MS calculated for C₁₅H₄₉N₃O₄: 575.37; found: [M+H]⁺ 576.

### Example 16

### Preparation of N-(2-((5Z,8Z,11Z,14Z,17Z)-N-methylicosa-5,8,11,14,17-pentaenamido)ethyl)nicotinamide (1-15)

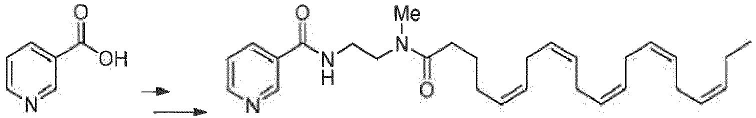

N-(2-(5Z,8Z,11Z,14Z,17Z)-N-Methylicosa-5,8,11,14,17-pentaenamido)ethyl)nicotinamide was prepared according to the procedures outlined in example 8, substituting the commercially available tert-butyl (2-aminoethyl)(methyl)carbamate for the diamine and EPA for the fatty acid component. MS calculated for C₂₉H₄₁N₃O₂: 463.32; found: [M+H] 464.

### Example 17

### Preparation of N-((1-((5Z,8Z,11Z,14Z,17Z)-icosa-5,8,11,14,17-pentaenoyl)piperidin-4-yl)methyl)nicotinamide (1-31):

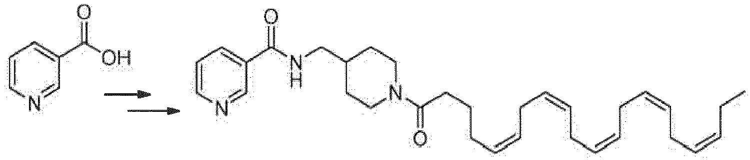

N-((1-((5Z,8Z,11Z,14Z,17Z)-Icosa-5,8,11,14,17-pentaenoyl)piperidin-4-yl)methyl)nicotinamide was prepared according to the procedures outlined in example 8, substituting the commercially available tert-butyl 4-(aminomethyl)piperidine-1-carboxylate for the diamine and EPA for the fatty acid component. MS calculated for C₃₂H₄₅N₃O₂: 503.35; found: [M+H]⁺ 504.

### Example 18

### Preparation of (5Z,8Z,11Z,14Z,17Z)-N-((1-nicotinoylpiperidin-4-yl)methyl)icosa-5,8,11,14,17-pentaenamide (I-29):

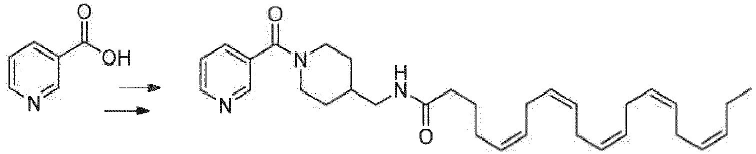

The commercially available tert-butyl 4-(aminomethyl)piperidine-1-carboxylate (1 mmol) was taken up in 25 mL of CH₂Cl₂ along with EPA (1 mmol) and EDC (1.1 mmol). The resulting reaction mixture was stirred at room temperature for 4 h and then washed with saturated NH₄Cl, brine, dried (Na₂SO₄) and concentrated under reduced pressure. The resulting residue was purified by silica gel chromatography (95% CH₂Cl₂, 5% MeOH) to afford tert-butyl 4-((5Z,8Z,11Z,14Z,17Z)-icosa-5,8,11,14,17-pentaenamidomethyl)piperidine-1-carboxylate. tert-Butyl 4-((5Z,8Z,11Z,14Z,17Z)-icosa-5,8,11,14,17-pentaenamidomethyl)piperidine-1-carboxylate (0.5 mmol) was taken up in 3 mL of 4 N HCl in dioxane and allowed to stir at room temperature for 15 min. The resulting reaction mixture was diluted with EtOAc and concentrated under reduced pressure to afford the HCl salt of (5Z,8Z,11Z,14Z,17Z)-N-(piperidin-4-ylmethyl)icosa-5,8,11,14,17-pcntacnamidc. This material was taken up in 20 mL of CH₂Cl₂ along with nicotinic acid (0.5 mmol), HATU (1.1 mmol) and DIEA (0.75 mmol). The resulting reaction mixture was stirred at room temperature for 6 h. It was then washed with saturated NH₄Cl, brine, dried (Na₂SO₄) and concentrated under reduced pressure. The resulting residue was purified by silica gel chromatography (95% CH₂Cl₂, 5% MeOH) to afford (5Z,8Z,11Z,14Z,17Z)-N-((1-nicotinoylpiperidin-4-yl)methyl)icosa-5,8,11,14,17-pentaenamide. MS calculated for C₃₂H₄₅N₃O₂: 503.35; found: [M+H]⁺ 504.

### Example 19

### Preparation of N-(((1R,4R)-4-((5Z,8Z,11Z,14Z,17Z)-icosa-5,8,11,14,17-pentaenamido)cyclohexyl)methyl)nicotinamide (I-41):

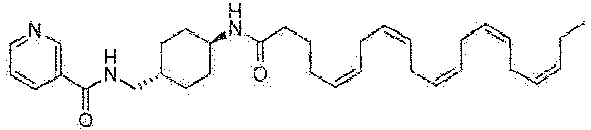

N-(((1R,4R)-4-((5Z,8Z,11Z,14Z,17Z)-Icosa-5,8,11,14,17-pentaenamido)cyclohexyl)methyl)nicotinamide was prepared according to the procedures outlined in example 8, using the commercially available tert-butyl ((1r,4r)-4-(aminomcthyl)cyclohexyl)carbamate as the diamine. MS calculated for C₃₃H₄₇N₃O₂: 517.37; found: [M+H]⁺ 518.

### Example 20

### Preparation of N-((S)-1-(4-((5Z,8Z,11Z,14Z,17Z)-icosa-5,8,11,14,17-pentaenoyl)piperazin-1-yl)-3-methyl-1-oxobutan-2-yl)nicotinamide (I-51):

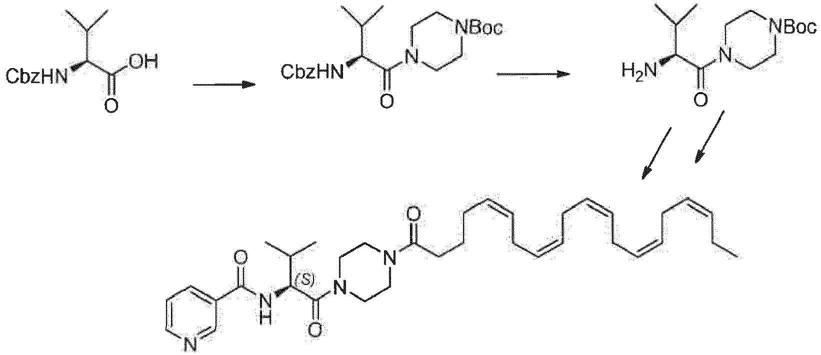

To a suspension of (S)-2-(((benzyloxy)carbonyl)amino)-3-methylbutanoic acid (25.1 g, 100 mmol), EDC.HCl (23 g, 120 mmol), HOBt (16.2 g, 120 mmol) and Boc-piperazine (18.6 g, 100 mmol) in 250 mL of CH₂Cl₂ was added Et₃N (20.2 g, 200 mmol) at 0 °C. The resulting reaction mixture was stirred at room temperature for 18 h and then diluted with CH₂Cl₂ (250 mL). The organic layer was washed with saturated aq. NH₄Cl (3 x 200 mL) and brine (3 x 200 mL). The organic layer was dried over anhydrous Na₂SO₄ and concentrated under reduced pressure. The resulting residue was purified by silica gel chromatography (EtOAc/pentanes) to afford 20.0 g of (S)-tert-butyl 4-(2-(((benzyloxy)carbonyl)amino)-3-methylbutanoyl)piperazine-1-carboxylate (48%).

A mixture of (S)-tert-butyl 4-(2-(((benzyloxy)carbonyl)amino)-3-methylbutanoyl)piperazine-1-carboxylate (20.0 g, 47.7 mmol) and 10% Pd/C (2 g) in McOH (150 mL) was stirred under1 atmosphere of H₂ at room temperature for 18 h. The solution was filtered through Celite, and the filtrate was concentrated under reduced pressure to afford (S)-tert-butyl 4-(2-amino-3-methylbutanoyl)piperazine-1-carboxylate (11.4 g, 40mmol) as a white solid (84%). N-((S)-1-(4-((5Z,8Z,11Z,14Z,17Z)-icosa-5,8,11,14,17-pentaenoyl)piperazin-1-yl)-3-methyl-1-oxobutan-2-yl)nicotinamide was then prepared using the procedures outlined in example 8, substituting (S)-tcrt-butyl 4-(2-(((benzyloxy)carbonyl)amino)-3-methylbutanoyl)piperazine-1-carboxylate for the diamine component. MS calculated for C₃₅H₄₆N₄O₃: 574.39; found: [M+H]⁺ 575.

### Example 21

### Preparation of N-(4-(4-((5Z,8Z,11Z,14Z,17Z)-icosa-5,8,11,14,17-pentaenoyl)piperazine-1-carbonyl)phenyl)nicotinamide (I-56):

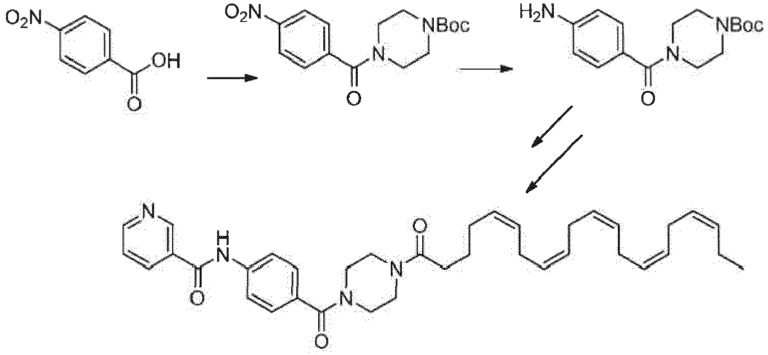

To a suspension of 4-nitrobenzoic acid (16.7 g, 100 mmol), EDC.HCl (22.92 g, 120 mmol), HOBt (16.2 g, 120 mmol) and Boc-piperazine (18.6 g, 100 mmol) in 400 mL of CH₂Cl₂ was added Et₃N (20.2 g, 200 mmol) at 0°C. The resulting reaction mixture was stirred at room temperature for 18 h and then diluted with CH₂Cl₂ (200 mL). The organic layer was washed with saturated aq.NH₄Cl (3 x 200 mL) and brine (3 x 200 mL). The organic layer was dried over anhydrous Na₂SO₄ and concentrated under reduced pressure. The resulting residue was purified by silica gel chromatography (EtOAc/pentancs) to afford 20 g of tert-butyl 4-(4-nitrobenzoyl)piperazine-1-carboxylate (60%).

A mixture of tert-butyl 4-(4-nitrobenzoyl)piperazine-1-carboxylate (20 g, 60 mmol) and 10% Pd/C (4 g) in MeOH (600 mL) was stirred under 1 atmosphere of H₂ at room temperature for for 18 h. The solution was filtered through Celite and the filtrate was concentrated under reduced pressure to afford 18 g of tert-butyl 4-(4-aminobenzoyl)piperazine-1-carboxylate as a white solid (100%).

N-(4-(4-((5Z,8Z,11Z,14Z,17Z)-icosa-5,8,11,14,17-pentaenoyl)piperazine-1-carbonyl)phenyl)nicotinamide was then prepared according to the procedures outlined in example 8, substituting tert-butyl 4-(4-aminobenzoyl)piperazine-1-carboxylate for the diamine component. MS calculated for C₃₇H₄₆N₄O₃: 594.36; found: [M+H]⁺ 595.

### Example 22

### Preparation of N-(2-(2-((5Z,8Z,11Z,14Z,17Z)-N-Methylicosa-5,8,11,14,17-pentaenamido)acetamido)ethyl)nicotinamide (I-57):

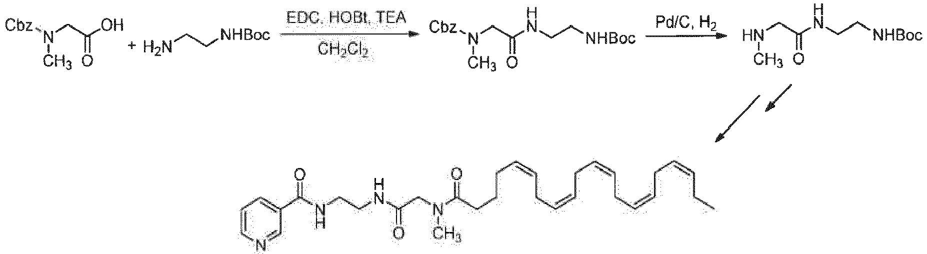

The same procedures outlined in example 21 were used to prepare tert-butyl (2-(2-(methylamino)acetamido)ethyl)carbamate, substituting 2-(((benzyloxy)carbonyl)(methyl)amino)acetic acid and tert-butyl (2-aminoethyl)carbamate as the appropriate starting materials. N-(2-(2-((5Z,8Z,11Z,14Z,17Z)-N-Methylicosa-5,8,11,14,17-pentaenamido)acetamido)ethyl)nicotinamide was then prepared using tert-butyl (2-(2-(methylamino)acetamido)ethyl)carbamate according to the procedures outlined in example 18. MS calculated for C₃₁H₄₄N₄O₃: 520.34; found: [M+H]⁺ 521.

### Example 23

### Preparation of N-((1-((5Z,8Z,11Z,14Z,17Z)-icosa-5,8,11,14,17-pentaenoyl)piperidin-4-yl)methyl)-N-methylnicotinamide (1-62):

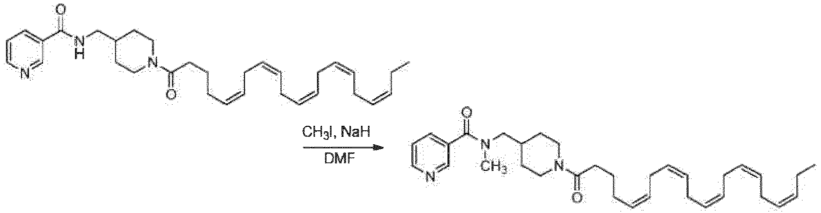

N-((1-((5Z,8Z,11Z,14Z,17Z)-icosa-5,8,11,14,17-pentaenoyl)piperidin-4-yl)methyl)nicotinamide (example 17) was used as the starting material.

To 0.4 mmol of N-((1-((5Z,8Z,11Z,14Z,17Z)-icosa-5,8,11,14,17-pentaenoyl)piperidin-4-yl)methyl)nicotinamide, was added 1 mL DMF, followed by 3.0 equivalents of 60% NaH and 1.3 equivalents of methyl iodide under Argon. The resulting reaction mixture was stirred at room temperature for 1 hour and then quenched with half-saturated NH₄Cl. The mixture was diluted with ethyl acetate (100 mL). The organic layer was separated and washed with brine (3 x 10 mL), dried over Na₂SO₄ and concentrated under reduced pressure. The resulting residue was purificed by silica gel chromatography (gradient elution from 0-10% methanol in dichloromethane) to afford N-((1-((5Z,8Z,11Z,14Z,17Z)-icosa-5,8,11,14,17-pentaenoyl)piperidin-4-yl)methyl)-N-methylnicotinamide (95%). MS calculated for C₃₃H₄₇N₃O₂: 517.37; found: [M+H]⁺ 518.

### Example 24

### Preparation of N-((4-((5Z,8Z,11Z,14Z,17Z)-icosa-5,8,11,14,17-pentaenamido)-2-methylpyrimidin-5-yl)methyl)nicotinamide (I-64):

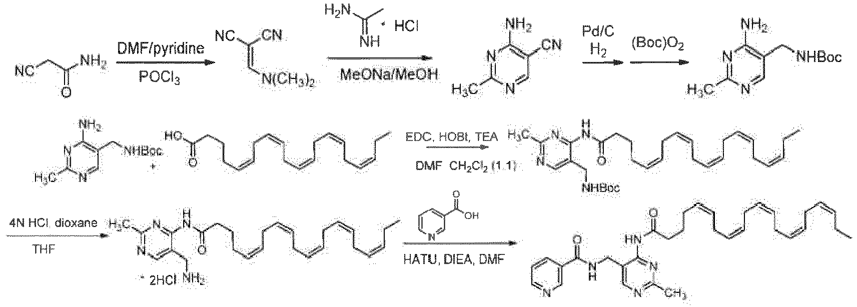

A mixture of 2-cyanoacetamide (50 g, 595 mmol), pyridine (4.7 g, 60 mmol), and DMF (91 g, 1.26 mol) was cooled to -10 °C. Then POCl₃ was to the cooled mixture dropwise over a period of 2 hours. After the addition was completed, the reaction was poured into ice-water (2 L) and then enough 30% aqueous NaOH solution was added to adjust the pH = 3. The resulting mixture was extracted with ethyl acetate (3 x 1 L). The combined organic layers were dried (Na₂SO₄) and concentrated under reduced pressure to afford 45 g of 2-((dimethylamino)methylene)malononitrile (62 %).

To an ice-cooled solution of sodium methoxide (15.9 g, 294 mmol) in methanol (150 mL) was added acetamidine hydrochloride (27.9 g, 292 mmol). The reaction mixture was stirred for 10 min and quickly filtered from precipitated sodium chloride. To the cooled filtrate was added a solution of 2-((dimethylamino)methylene)malononitrile (32.4 g, 223 mmol) in methanol (100 mL) over a period of 30 min. After stirring for 12 hrs at room temperature, the mixture was cooled to 0 °C, and the precipitate was collected by filtration and dried to afford 29 g of 4-amino-2-methylpyrimidine-5-carbonitrile (97 %).

In an autoclave the mixture of 4-amino-2-methylpyrimidine-5-carbonitrile (16 g, 119 mmol), modified Raney nickel (wet weight 15 g), and saturated methanol solution of ammonia (200 mL) was heated to 60 °C and stirred for 24 hrs at this temperature under 4 MPa of hydrogen pressure. The resulting reaction mixture was cooled to room temperature and filtered. The filtrate was concentrated under reduced pressure and the resulting residue was purified by silica gel chromatography (CH₂Cl₂/MeOH = 30/1∼10/1) to afford 14.8 g of 5-(aminomethyl)-2-methylpyrimidin-4-amine (90 %).

5-(Aminomethyl)-2-methylpyrimidin-4-amine (10 g, 72.5 mmol) was dissolved in 100 mL of CH₂Cl₂ and 50 mL of methanol, and triethylamine (8 mL, 109 mmol) was added, followed by (Boc)₂O. The resulting reaction mixture was stirred at room temperature for 12 hrs and then concentrated under reduced pressure. The resulting residue was purified by silica gel chromatography (CH₂Cl₂/MeOH = 50/1) to afford 14.63 g of tert-butyl ((4-amino-2-methylpyrimidin-5-yl)methyl)carbamate (85 %).

MS calculated for C₁₁H₁₈N₄O₂: 238.2; found: 239.1 [M+H]¹.

¹H NMR (300MHz, DMSO_d6): δ 7.80 (s, 1H), 7.24 - 7.28 (t, *J* = 11.1 Hz, 1H), 6.64 (s, 2H), 3.86 - 3.88 (d, *J* = 6 Hz, 2H), 2.28 (s, 3H), 1.37 (s, 9H).

To 1 g (4.2 mmol) of *tert*-butyl ((4-amino-2-mcthylpyrimidin-5-yl)methyl)carbamate in 20 mL dichloromethane and 20 mL dimethylformamide, was added 0.9 equivalents (5Z,8Z,11Z,14Z,17Z)-icosa-5,8,11,14,17-pentaenoic acid, 1.2 equivalents EDC, 1.2 equivalents HOBt and 6 equivalents triethylamine. The reaction was purged with nitrogen and run at room temperature. Upon completion, the crude reaction was washed with half-saturated NH₄Cl, brine, dried over Na₂SO₄, concentrated under reduced pressure. The resulting residue was purified by silica gel chromatography using a mixture of CH₂Cl₂/MeOH (gradient elution, from 0-10% methanol in dichloromethane) to give N-((4-((5Z,8Z, 11Z,14Z,17Z)-icosa-5,8,11,14,17-pentaenamido)-2-methylpyrimidin-5-yl)methyl)nicotinamide in 40% isolated yield. This intermediate was dissolved in THF, to which 4 equivalents of 4N HCl in dioxane was added and reaction was stirred for 45 minutes. The reaction mixture was diluted with ethyl acetate and concentrated under reduced pressure to give (5Z,8Z,11Z,14Z,17Z)-N-(5-(aminomethyl)-2-methylpyrimidin-4-yl)icosa-5,8,11,14,17-pentaenamide hydrochloride.

To 1.15 mmol of (5Z,8Z,11Z,14Z,17Z)-N-(5-(aminomethyl)-2-methylpyrimidin-4-yl)icosa-5,8,11,14,17-pentaenamide hydrochloride in 2 mL dimethylformamide was added 1.1 equivalents niacin, followed by 1.2 equivalents HATU, and 6.0 equivalents diisopropylethylamine. The resulting reaction mixture was stirred at room temperature for 16 hours. The crude reaction mixture was washed with half-saturated NH₄Cl, brine, dried over Na₂SO₄, and concentrated under reduced pressure. The resulting residue was purified by silica gel chromatography using a mixture of CH₂Cl₂/MeOH (gradient elution from 0-10% methanol in dichloromethane) to afford N-((4-((5Z,8Z,11Z,14Z,17Z)-icosa-5,8,11,14,17-pentaenamido)-2-methylpyrimidin-5-yl)methyl)nicotinamide (30%). MS calculated for C₃₂H₄₁N₅O₂: 527.33; found: [M+H]⁺ 528.

### Example 25

### Preparation of 2-fluoro-N-(2-((5Z,8Z,11Z,14Z,17Z)-icosa-5,8,11,14,17-pentaenamido)ethyl)nicotinamide (VI-7):

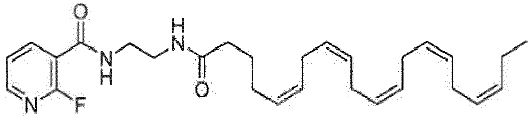

2-Fluoro-N-(2-((5Z,8Z,11Z,14Z,17Z)-icosa-5,8,11,14,17-pentaenamido)ethyl)nicotinamide was prepared according to the procedures outlined in example 8, substituting 2-fluoronicotinic acid as the appropriate starting material. MS calculated for C₂₈H₃₈FN₃O₂: 467.29; found: [M+H]⁺ 468.

### Example 28

### Preparation of N-((S)-1-((5Z,8Z,11Z,14Z,17Z)-icosa-5,8,11,14,17-pentaenoyl)pyrrolidin-3-yl)-5-methyl-4-oxo-5-phenyl-4,5-dihydrofuran-2-carboxamide (VII-4):

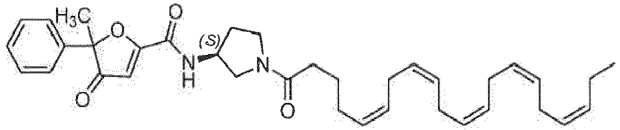

N-((S)-1-((5Z,8Z,11Z,14Z,17Z)-icosa-5,8,11,14,17-pentaenoyl)pyrrolidin-3-yl)-5-methyl-4-oxo-5-phenyl-4,5-dihydrofuran-2-carboxamide was prepared according to the procedures outlined in example 8, substituting acifran as the appropriate starting material. MS calculated for C₃₆H₄₆N₂O₄: 570.35; found: [M+H]⁺ 571.

### Example 29:

### Preparation of 2-(((S)-1-((5Z,8Z,11Z,14Z,17Z)-icosa-5,8,11,14,17-pentaenoyl)pyrrolidin-3-yl)carbamoyl)-5-methylpyrazine 1-oxide (VII-12):

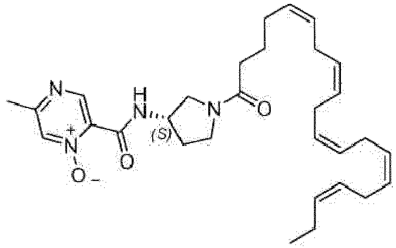

2-(((S)-1-((5Z,8Z,11Z,14Z,17Z)-icosa-5,8,11,14,17-pentaenoyl)pyrrolidin-3-yl)carbamoyl)-5-methylpyrazine 1-oxide was prepared according to the procedures outlined in example 8, substituting acipimox as the appropriate starting material. MS calculated for C₃₀H₄₂N₄O₄: 506.33; found: [M+H]⁺ 507.

### Example 30

### Preparation of (5Z,8Z,11Z,14Z,17Z)-N-((S)-1-(2-(4-(4-chlorobenzoyl)phenoxy)-2-methylpropanoyl)pyrrolidin-3-yl)icosa-5,8,11,14,17-pentaenamide (VII-28):

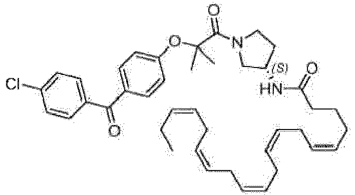

(5Z,8Z,11Z,14Z,17Z)-N-((S)-1-(2-(4-(4-chlorobenzoyl)phenoxy)-2-methylpropanoyl)pyrrolidin-3-yl)icosa-5,8,11,14,17-pentaenamide was prepared according to the procedures outlined in example 8, substituting 2-(4-(4-chlorobenzoyl)phenoxy)-2-mcthylpropanoic acid as the appropriate starting material. MS calculated for C₄₁H₅₁ClN₂O₄: 670.35; found: [M+H]⁺ 671.

The present invention is not to be limited in scope by the specific embodiments disclosed in the examples which are intended as illustrations of a few aspects of the invention and any embodiments that are functionally equivalent are within the scope of this invention. Indeed, various modifications of the invention in addition to those shown and described herein will become apparent to those skilled in the art and are intended to fall within the scope of the appended claims. All patents, patent applications, and publications cited herein are hereby incorporated by reference in their entireties.

### EQUIVALENTS

Those skilled in the art will recognize, or be able to ascertain, using no more than routine experimentation, numerous equivalents to the specific embodiments described specifically herein. Such equivalents are intended to be encompassed in the scope of the following claims.

### Disclosed Items

1. A method for treating a metabolic disease comprising inhibiting the production of or lowering serum levels of the proprotein convertase subtilisin/kexin type 9 (PCSK9) by administering to a patient in need thereof an effective amount of a fatty acid bioactive derivative.
2. The method of item 1, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
3. The method of item 2, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
4. The method of item 2, wherein the method further comprises administering another ACE inhibitor selected from the group consisting of enalapril, ramipril, quinapril, perindopril, lisinopril, imidapril, zofenopril, trandolapril, fosinopril, and captopril.
5. The method of item 2, wherein the method further comprises administering another fibrate selected from the group consisting of bezafibrate, citprofibrate, clofibrate, gemfibrozil, and fenofibrate.
6. The method of item 2, wherein the method further comprises administering another angiotensin II receptor blocker (ARB) selected from the group consisting of, but not limited to, termisartan, losartan, irbesartan, azilsartan, and olmesartan.
7. The method of item 2, wherein the method further comprises administering another therapeutic agent selected from the group consisting of, but not limited to, a PCSK9 monoclonal antibody, a biologic agent, a small interfering RNA (siRNA) and a gene silencing oligonucleotide.
8. The method of item 2, wherein the method further comprises administering another omega-3 fatty acid selected from the group consisting of, but not limited to, *all-cis-*7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis*-11,14,17-eicosatrienoic acid), eicosatetraenoic acid (ETA or *all-cis-*8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis-*9,12,15,18,21-docosahexaenoic acid), or tetracosahexaenoic acid (nisinic acid or *all-cis-*6,9,12,15,18,21-tetracosenoic acid).
9. The method of item 2, wherein the method further comprises administering another therapeutic agent selected from the group consisting of, but not limited to, niacin, acifran and acipimox.
10. A compound of the **Formula II'**: 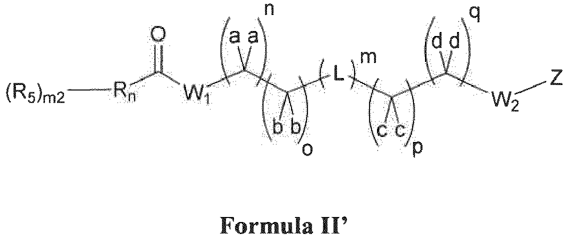 or a pharmaceutically acceptable salt, hydrate, solvate, prodrug, enantiomer or stereoisomer thereof
   wherein
   Rₙ is phenyl, naphthyl, heteroaryl, or a heterocycle ;
   W₁ and W₂ are each independently null, O, S, NH, NR, or W₁ and W₂ can be taken together can form an imidazolidine or piperazine group, with the proviso that W₁ and W₂ can not be O simultaneously;
   W₃ is independently O or null;
   R₁₂ is independently H, OH, OR", R", or OC(O)R" where R" is independently C₁-C₆ alkyl;
   each m1 is independently 0, 1, 2 or 3;
   each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently null, -O-, -S-, -S(O)-, -S(O)₂-, -S-S-, -(C₁-C₆alkyl)-, -(C₃-C₆cycloalkyl)-, a heterocycle, a heteroaryl, 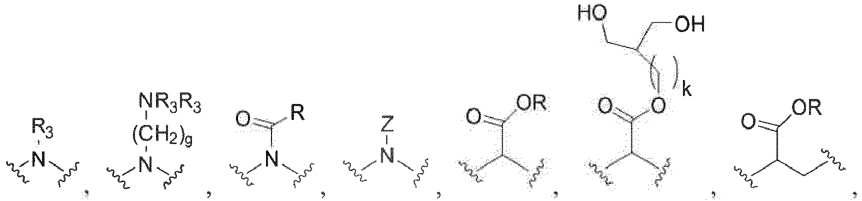 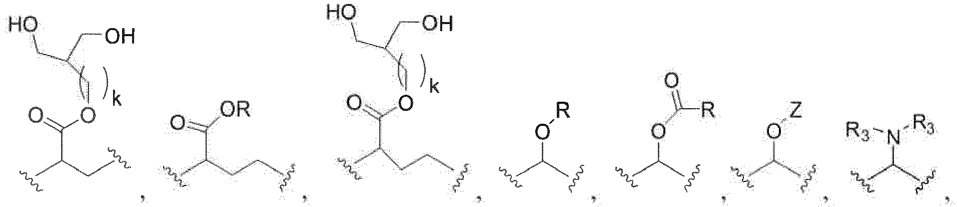 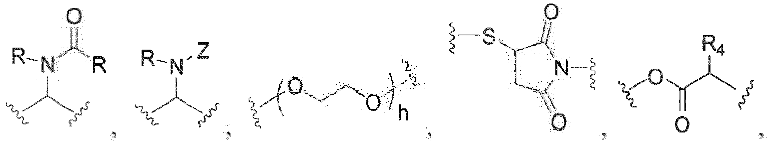 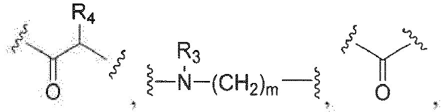 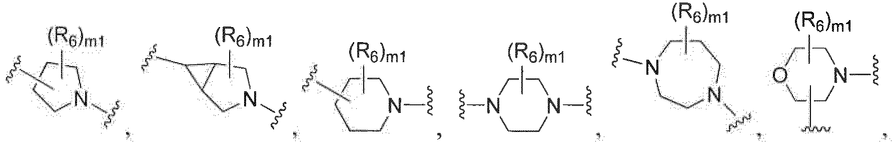 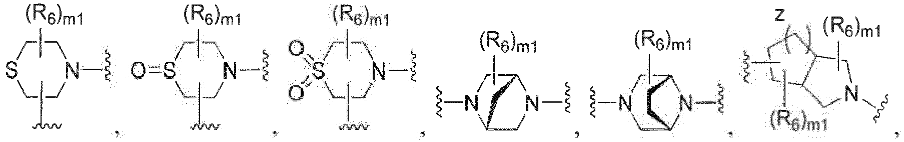 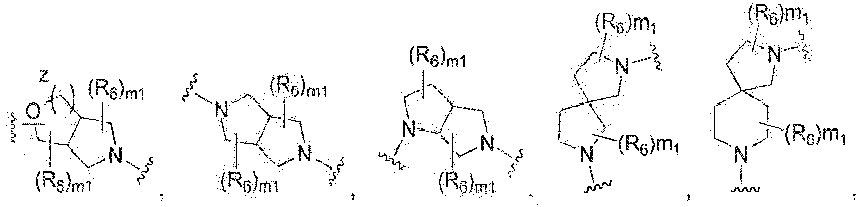 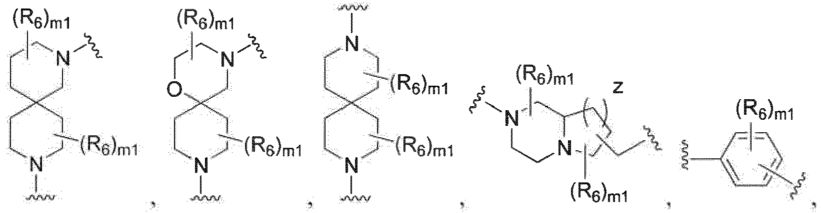 wherein the representation of L is not limited directionally left to right as is depicted, rather either the left side or the right side of L can be bound to the W₁ side of the compound of Formula II';
   with the proviso that when L is independently -O-, -S-, -S(O)-, -S(O)₂-, -S-S-, 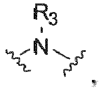 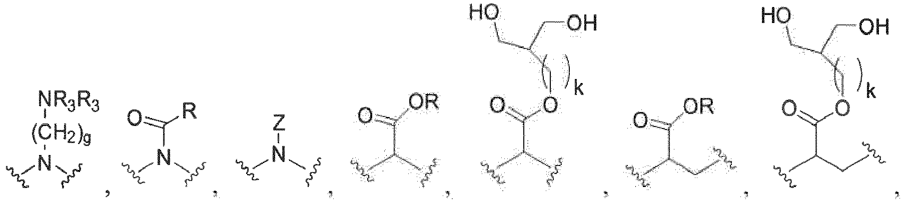 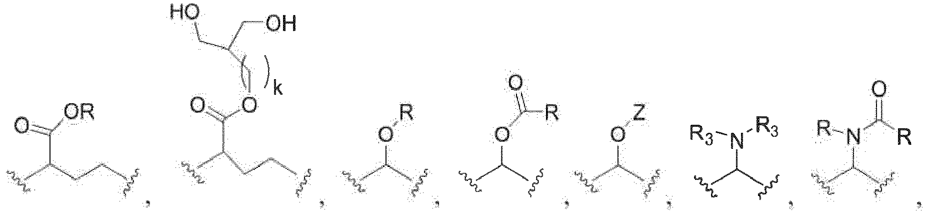 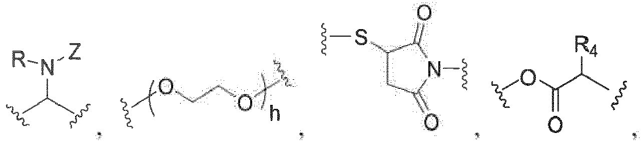 then Rn is not 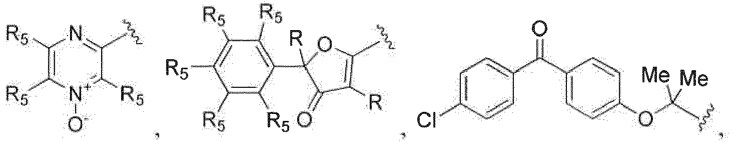 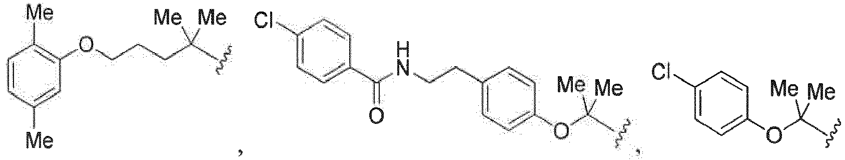 and in which g, h, k, R, R₃, R₅ and Z are as defined below;
   and with the further proviso that Rn is not: 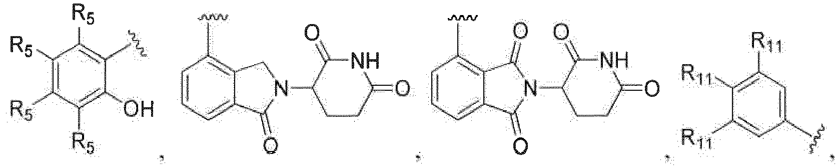 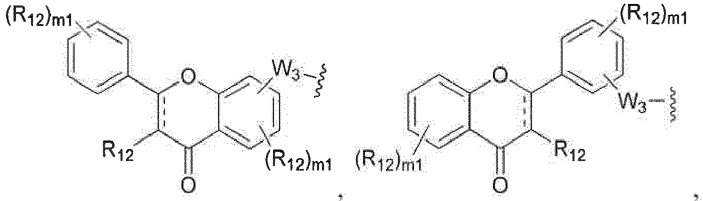 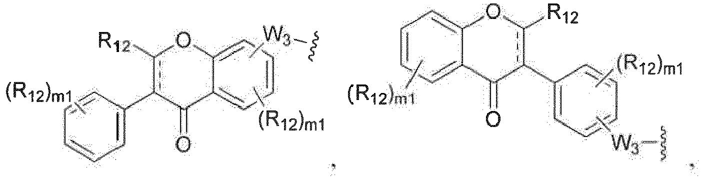 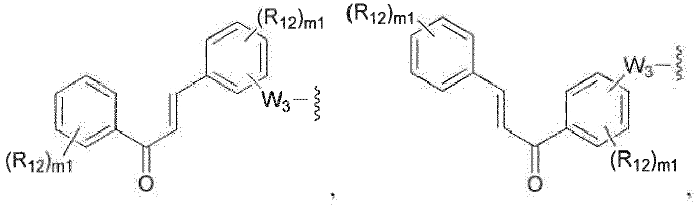 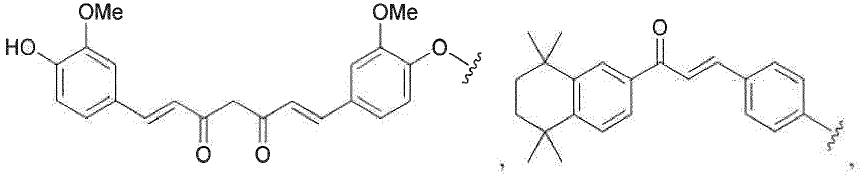 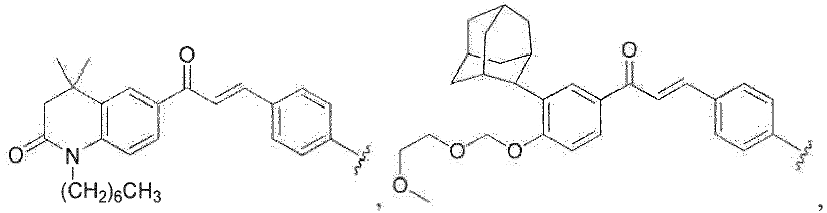 and 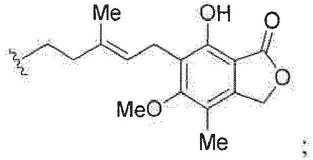 R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₅ is each independently selected from the group consisting of -H, -D, -Cl, -F, -CN, OH, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
   each g is independently 2, 3 or 4;
   each h is independently 1, 2, 3 or 4;
   m is 0, 1, 2, or 3; if m is more than 1, then L can be the same or different;
   m1 is 0, 1, 2 or 3;
   m2 is 0, 1, 2, 3, 4 or 5;
   k is 0, 1, 2, or 3;
   z is 1, 2, or 3;
   each R₃ is independently H or C₁-C₆ alkyl, or both R₃ groups, when taken together with the nitrogen to which they are attached, can form a heterocycle;
   each R₄ is independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, 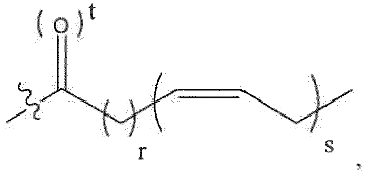 or 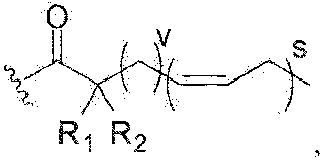 with the proviso that there is at least one 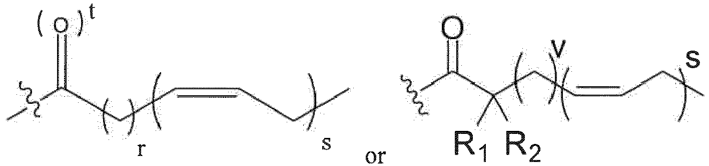 in the compound;
   each r is independently 2, 3, or 7;
   each s is independently 3, 5, or 6;
   each t is independently 0 or 1;
   each v is independently 1, 2, or 6;
   R₁ and R₂ are each independently hydrogen, deuterium, -C₁-C₄ alkyl, -halogen, -OH, -C(O)C,-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
   each R is independently -H, -C₁-C₃ alkyl, phenyl or straight or branched C₁-C₄ alkyl optionally substituted with OH, or halogen.
11. A pharmaceutical composition comprising a compound of item 10 and a pharmaceutically acceptable carrier.
12. A method for treating a metabolic disease by inhibiting proprotein convertase subtilisin/kexin type 9 (PCSK9) by administering to a patient in need thereof an effective amount of a compound of item 11.
13. The method of item 12, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
14. The method of item 12, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
15. The method of item 12, wherein the method further comprises administering another ACE inhibitor selected from the group consisting of enalapril, ramipril, quinapril, perindopril, lisinopril, imidapril, zofenopril, trandolapril, fosinopril, and captopril.
16. The method of item 12, wherein the method further comprises administering another fibrate selected from the group consisting of, bezafibrate, citprofibrate, clofibrate, gemfibrozil, and fenofibrate.
17. The method of item 12, wherein the method further comprises administering another angiotensin II receptor blocker (ARB) selected from the group consisting of termisartan, losartan, irbesartan, azilsartan, and olmesartan.
18. The method of item 12, wherein the method further comprises administering another therapeutic agent selected from the group consisting of a PCSK9 monoclonal antibody, a biologic agent, a small interfering RNA (siRNA) and a gene silencing oligonucleotide.
19. The method of item 12, wherein the method further comprises administering another omega-3 fatty acid selected from the group consisting of *all-cis-*7,10,13-hexadecatrienoic acid, α-linolenic acid (ALA or *all-cis-*9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
20. The method of item 12, wherein the method further comprises administering another therapeutic agent selected from the group consisting of niacin, acifran and acipimox.
21. A compound of the **Formula VI**: 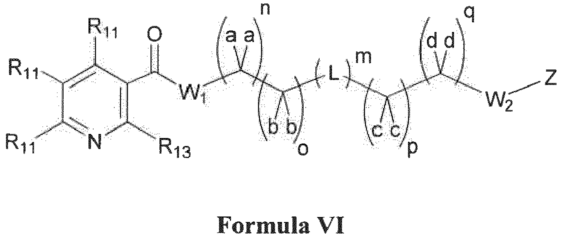 or a pharmaceutically acceptable salt, hydrate, solvate, prodrug, enantiomer or a stereoisomer thereof;
   wherein
   W₁ and W₂ are each independently null, O, S, NH, NR, or W₁ and W₂ can be taken together can form an imidazolidine or piperazine group, with the proviso that W₁ and W₂ can not be O simultaneously;
   R₁₁ is independently H, -OH, -OC(O)-R, -O-aryl, -aryl, -heteroaryl, or -heterocyclic;
   R₁₃ is independently H, C₁-C₃alkyl, -OH, -OC(O)-R, or halogen;
   each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently null, -O-, -S-, -S(O)-, -S(O)₂-, -S-S-, -(C₁-C₆alkyl)-, -(C₃-C₆cycloalkyl)-, a heterocycle, a heteroaryl, 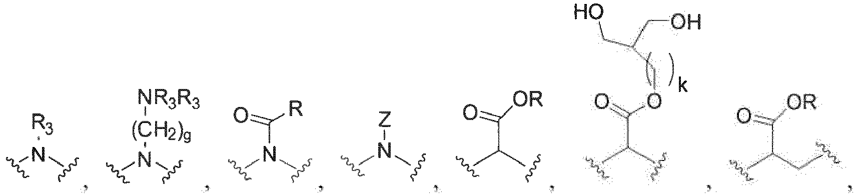 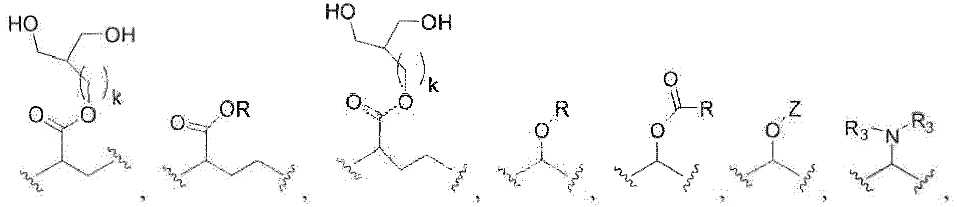 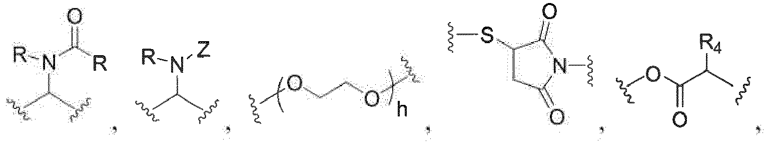 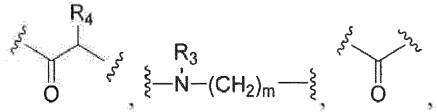 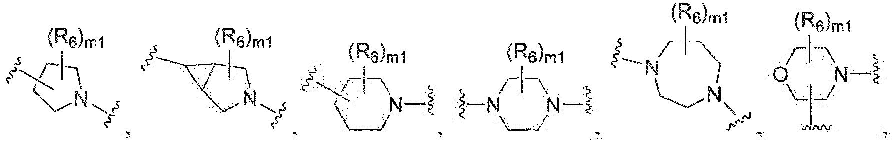 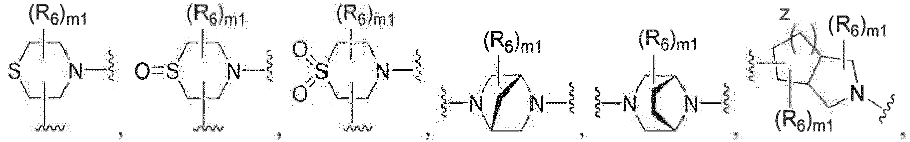 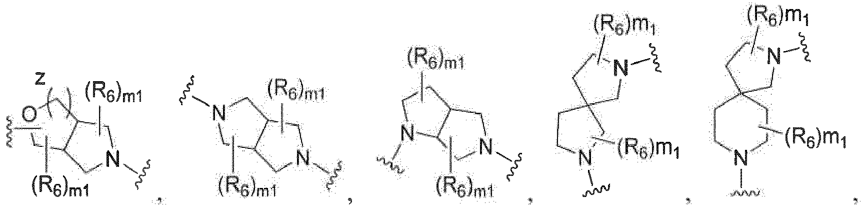 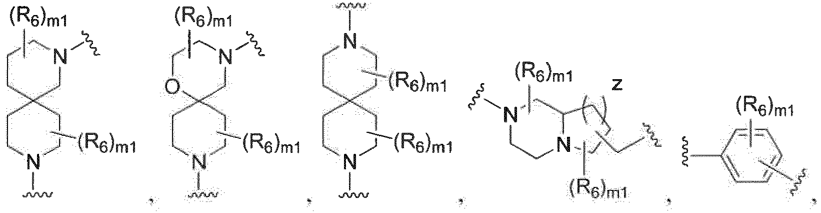 wherein the representation of L is not limited directionally left to right as is depicted, rather either the left side or the right side of L can be bound to the W₁ side of the compound of Formula VI;
   R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₅ is each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
   each g is independently 2, 3 or 4;
   each h is independently 1, 2, 3 or 4;
   m is 0, 1, 2, or 3; if m is more than 1, then L can be the same or different;
   m1 is 0, 1,2 or 3;
   k is 0, 1, 2, or 3;
   z is 1, 2, or 3;
   each R₃ is independently H or C₁-C₆ alkyl, or both R₃ groups, when taken together with the nitrogen to which they are attached, can form a heterocycle;
   each R₄ is independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, 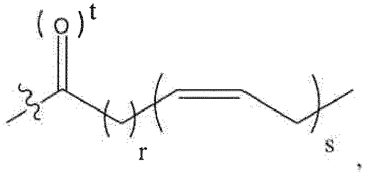 or 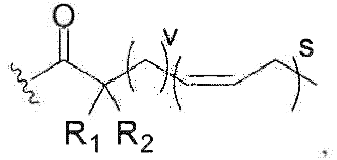 with the proviso that there is at least one 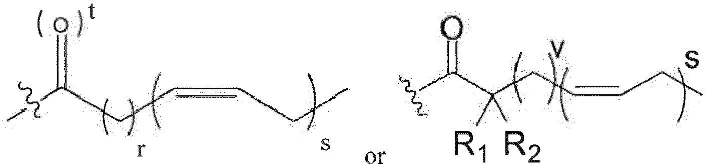 in the compound;
   each r is independently 2, 3, or 7;
   each s is independently 3, 5, or 6;
   each t is independently 0 or 1;
   each v is independently 1, 2, or 6; R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
   each R is independently -H, -C₁-C₃ alkyl, phenyl or straight or branched C₁-C₄ alkyl optionally substituted with OH, or halogen.
22. A method for treating a metabolic disease by inhibiting proprotein convertase subtilisin/kexin type 9 (PCSK9) by administering to a patient in need thereof an effective amount of a compound of item 21.
23. The method of item 22, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
24. The method of item 22, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
25. The method of item 22, wherein the method further comprises administering another ACE inhibitor selected from the group consisting of enalapril, ramipril, quinapril, perindopril, lisinopril, imidapril, zofenopril, trandolapril, fosinopril, and captopril.
26. The method of item 22, wherein the method further comprises administering another fibrate selected from the group consisting of bezafibrate, citprofibrate, clofibrate, gemfibrozil, and fenofibrate.
27. The method of item 22, wherein the method further comprises administering another angiotensin II receptor blocker (ARB) selected from the group consisting of termisartan, losartan, irbesartan, azilsartan, and olmesartan.
28. The method of item 22, wherein the method further comprises administering another therapeutic agent selected from the group consisting of a PCSK9 monoclonal antibody, a biologic agent, a small interfering RNA (siRNA) and a gene silencing oligonucleotide.
29. The method of item 22, wherein the method further comprises administering another omega-3 fatty acid selected from the group consisting of *all-cis-*7,10,13-hexadecatrienoic acid, α-linolenic acid (ALA or *all-cis-*9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis-*8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis-*9,12,15,18,21-docosahexaenoic acid), or tetracosahexaenoic acid (nisinic acid or *all-cis-*6,9,12,15,18,21-tetracosenoic acid).
30. The method of item 22, wherein the method further comprises administering another therapeutic agent selected from the group consisting of niacin, acifran and acipimox.
31. A compound of the **Formula VII**: 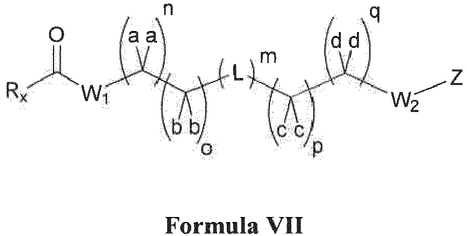 or a pharmaceutically acceptable salt, hydrate, solvate, prodrug, enantiomer or a stereoisomer thereof;
   wherein Rₓ is independently 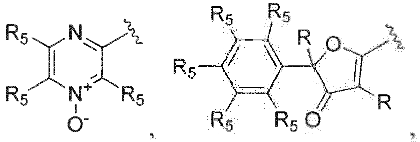 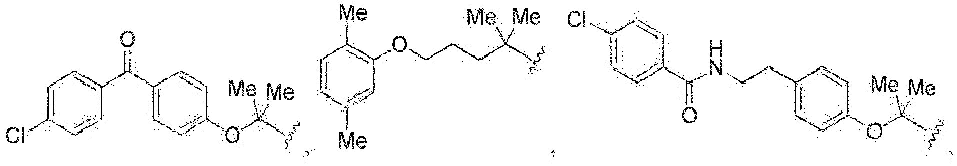 or 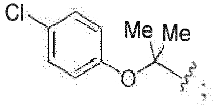 W₁ and W₂ are each independently null, O, S, NH, NR, or W₁ and W₂ can be taken together can form an imidazolidine or piperazine group, with the proviso that W₁ and W₂ can not be O simultaneously;
   each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently null, -(C₁-C₆alkyl)-, -(C₃-C₆cycloalkyl)-, a heterocycle, a heteroaryl, 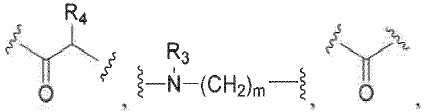 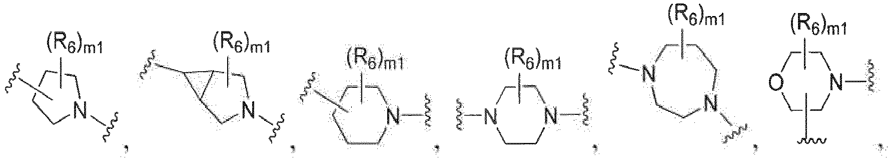 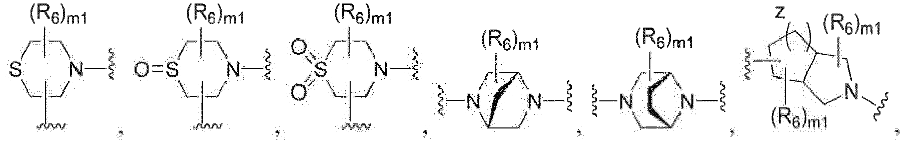 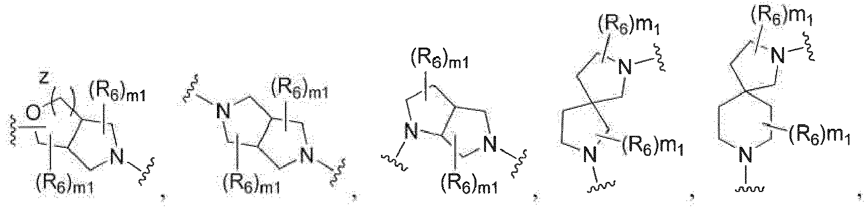 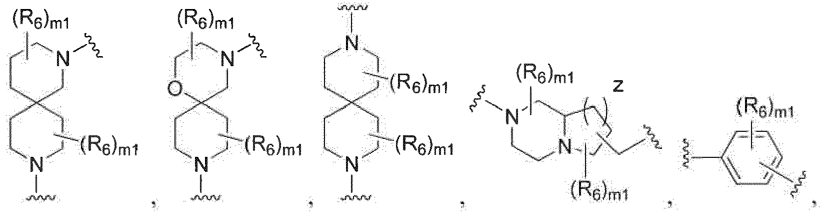 wherein the representation of L is not limited directionally left to right as is depicted, rather either the left side or the right side of L can be bound to the W₁ side of the compound of Formula VII;
   R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₅ is each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
   each g is independently 2, 3 or 4;
   each h is independently 1, 2, 3 or 4;
   m is 0, 1, 2, or 3; if m is more than 1, then L can be the same or different;
   m1 is 0, 1, 2 or 3;
   k is 0, 1, 2, or 3;
   z is 1, 2, or 3;
   each R₃ is independently H or C₁-C₆ alkyl, or both R₃ groups, when taken together with the nitrogen to which they are attached, can form a heterocycle;
   each R₄ is independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, 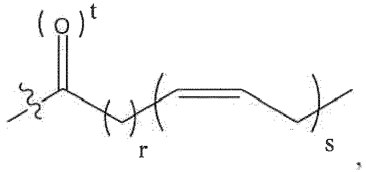 or 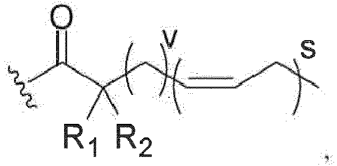 with the proviso that there is at least one 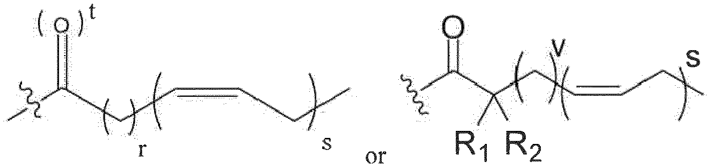 in the compound;
   each r is independently 2, 3, or 7;
   each s is independently 3, 5, or 6;
   each t is independently 0 or 1;
   each v is independently 1, 2, or 6;
   R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
   each R is independently -H, -C₁-C₃ alkyl, phenyl or straight or branched C₁-C₄ alkyl optionally substituted with OH, or halogen.
32. A method for treating a metabolic disease by inhibiting proprotein convertase subtilisin/kexin type 9 (PCSK9) by administering to a patient in need thereof an effective amount of a compound of item 31.
33. The method of item 32, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
34. The method of item 32, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
35. The method of item 32, wherein the method further comprises administering another ACE inhibitor selected from the group consisting of enalapril, ramipril, quinapril, perindopril, lisinopril, imidapril, zofenopril, trandolapril, fosinopril, and captopril.
36. The method of item 32, wherein the method further comprises administering another fibrate selected from the group consisting of bezafibrate, citprofibrate, clofibrate, gemfibrozil, and fenofibrate.
37. The method of item 32, wherein the method further comprises administering another angiotensin II receptor blocker (ARB) selected from the group consisting of termisartan, losartan, irbesartan, azilsartan, and olmesartan.
38. The method of item 32, wherein the method further comprises administering another therapeutic agent selected from the group consisting of a PCSK9 monoclonal antibody, a biologic agent, a small interfering RNA (siRNA) and a gene silencing oligonucleotide.
39. The method of item 32, wherein the method further comprises administering another omega-3 fatty acid selected from the group consisting of *all-cis-*7,10,13-hexadecatrienoic acid, α-linolenic acid (ALA or *all-cis-*9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis-*8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis-*9,12,15,18,21-docosahexaenoic acid), or tetracosahexaenoic acid (nisinic acid or *all-cis-*6,9,12,15,18,21-tetracosenoic acid).
40. The method of item 32, wherein the method further comprises administering another therapeutic agent selected from the group consisting of niacin, acifran and acipimox.
41. A method of inhibiting the production of PCSK9 or lowering serum levels of PCSK9, comprising administering to a patient in need thereof a compound of the **Formula I:** 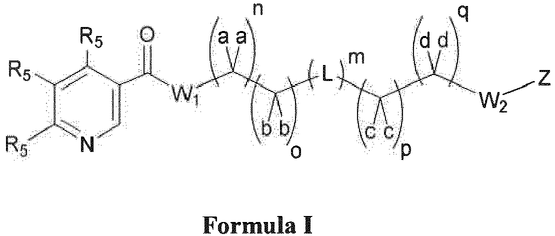 or a pharmaceutically acceptable salt, hydrate, solvate, prodrug, enantiomer or a stereoisomer thereof;
   wherein
   W₁ and W₂ are each independently null, O, S, NH, NR, or W₁ and W₂ can be taken together can form an imidazolidine or piperazine group, with the proviso that W₁ and W₂ can not be O simultaneously;
   each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently null, -O-, -S-, -S(O)-, -S(O)₂-, -S-S-, -(C₁-C₆alkyl)-, -(C₃-C₆cycloalkyl)-, a heterocycle, a heteroaryl, 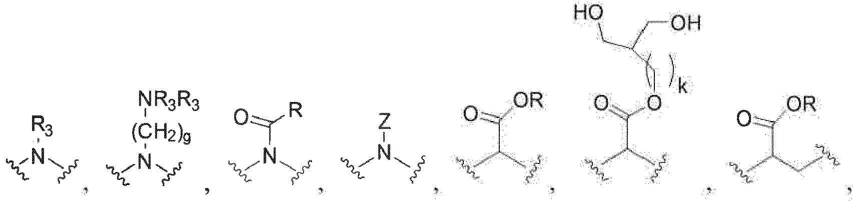 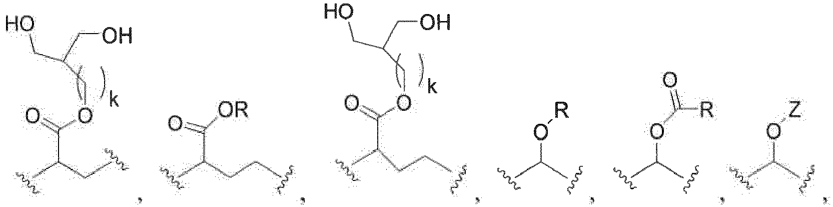 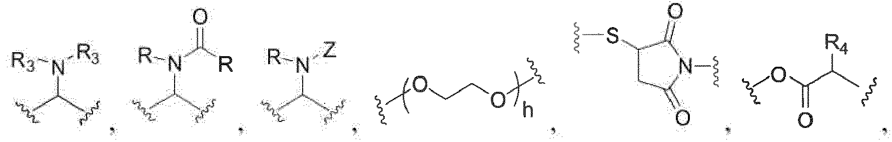 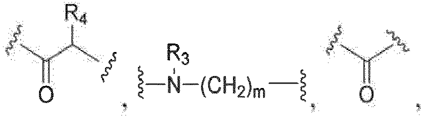 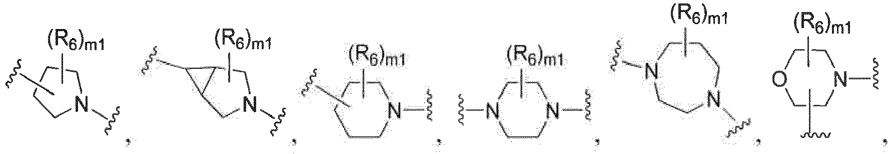 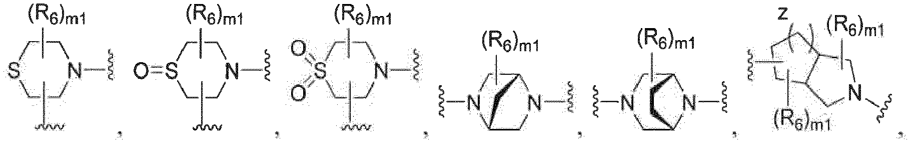 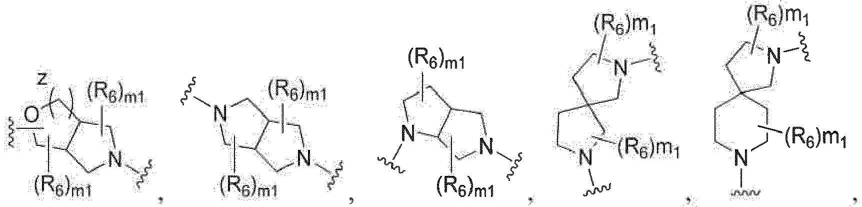 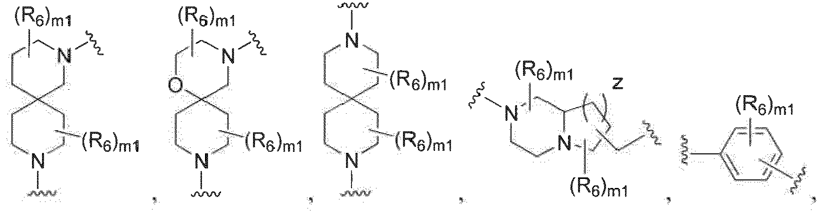 wherein the representation of L is not limited directionally left to right as is depicted, rather either the left side or the right side of L can be bound to the W₁ side of the compound of Formula I;
   R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₅ is each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
   each g is independently 2, 3 or 4;
   each h is independently 1, 2, 3 or 4;
   m is 0, 1, 2, or 3; if m is more than 1, then L can be the same or different;
   m1 is 0, 1, 2 or 3;
   k is 0, 1,2, or 3;
   z is 1, 2, or 3;
   each R₃ is independently H or C₁-C₆ alkyl, or both R₃ groups, when taken together with the nitrogen to which they are attached, can form a heterocycle;
   each R₄ is independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, 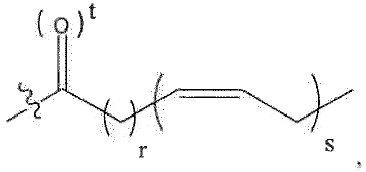 or 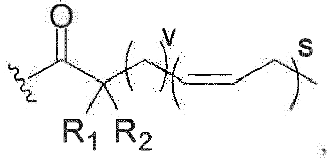 with the proviso that there is at least one 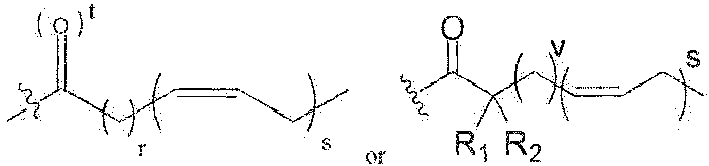 in the compound;
   each r is independently 2, 3, or 7;
   each s is independently 3, 5, or 6;
   each t is independently 0 or 1;
   each v is independently 1, 2, or 6;
   R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
   each R is independently -H, -C₁-C₃ alkyl, phenyl or straight or branched C₁-C₄ alkyl optionally substituted with OH, or halogen.
42. A method for treating a metabolic disease by inhibiting proprotein convertase subtilisin/kexin type 9 (PCSK9) by administering to a patient in need thereof an effective amount of a compound of item 41.
43. The method of item 42, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
44. The method of item 42, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
45. The method of item 42, wherein the method further comprises administering another ACE inhibitor selected from the group consisting of enalapril, ramipril, quinapril, perindopril, lisinopril, imidapril, zofenopril, trandolapril, fosinopril, and captopril.
46. The method of item 42, wherein the method further comprises administering another fibrate selected from the group consisting of bezafibrate, citprofibrate, clofibrate, gemfibrozil, and fenofibrate.
47. The method of item 42, wherein the method further comprises administering another angiotensin II receptor blocker (ARB) selected from the group consisting of termisartan, losartan, irbesartan, azilsartan, and olmesartan.
48. The method of item 42, wherein the method further comprises administering another therapeutic agent selected from the group consisting of a PCSK9 monoclonal antibody, a biologic agent, a small interfering RNA (siRNA) and a gene silencing oligonucleotide.
49. The method of item 42, wherein the method further comprises administering another omega-3 fatty acid selected from the group consisting of *all-cis-*7,10,13-hexadecatrienoic acid, α-linolenic acid (ALA or *all-cis-*9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
50. The method of item 42, wherein the method further comprises administering another therapeutic agent selected from the group consisting of niacin, acifran and acipimox.
51. A method of inhibiting the production of PCSK9 or lowering serum levels of PCSK9, comprising administering to a patient in need thereof a compound of formula **III**: 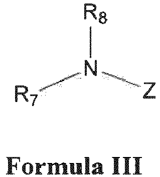 or a pharmaceutically acceptable salt, hydrate, solvate, enantiomer and stereoisomer thereof;
   wherein 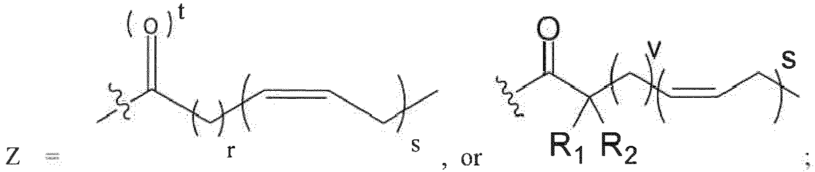 each r is independently 2, 3, or 7;
   each s is independently 3, 5, or 6;
   each t is independently 0 or 1;
   each v is independently 1, 2, or 6;
   R₁ and R₂ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₇ and R₈ are independently 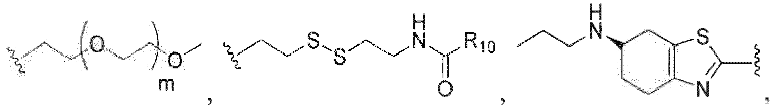 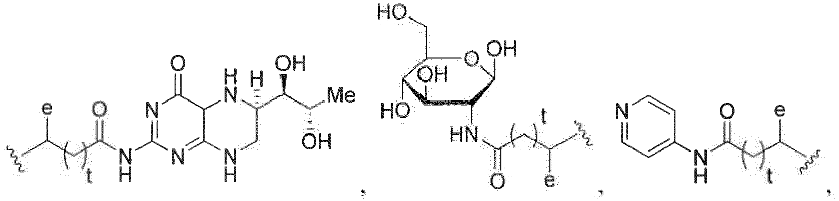 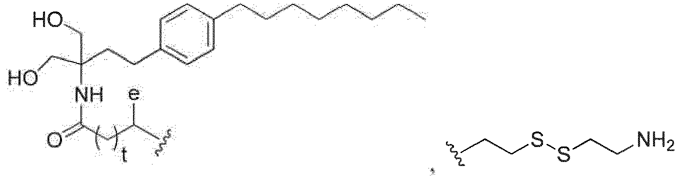 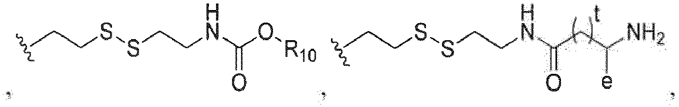 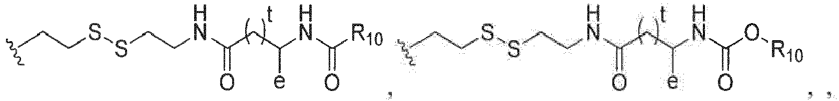 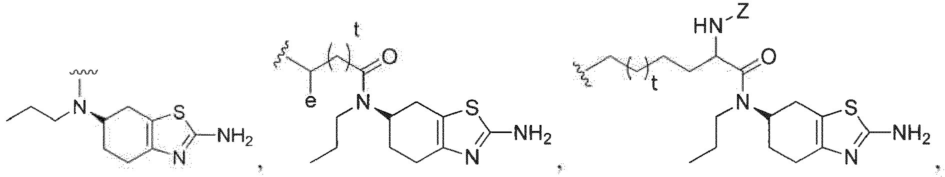 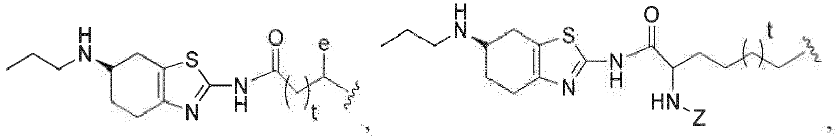 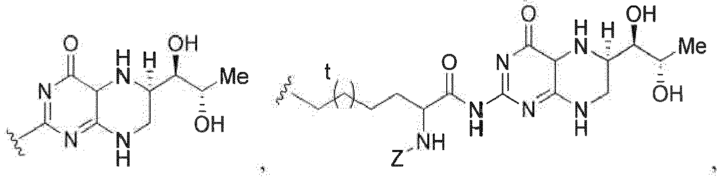 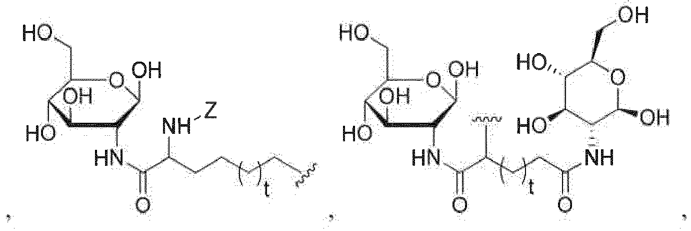 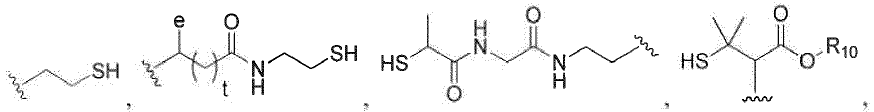 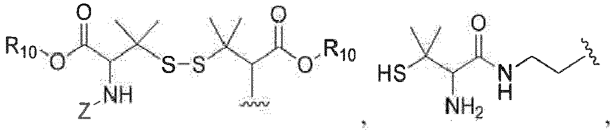 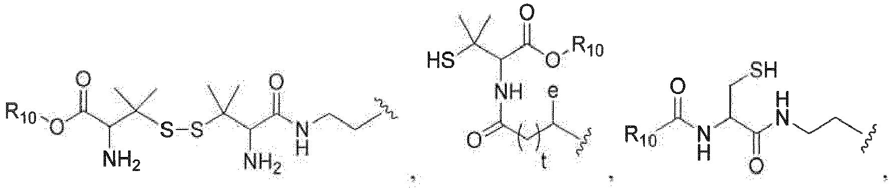 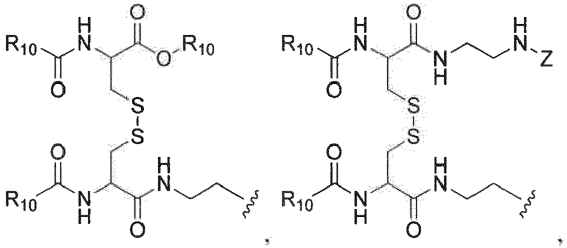 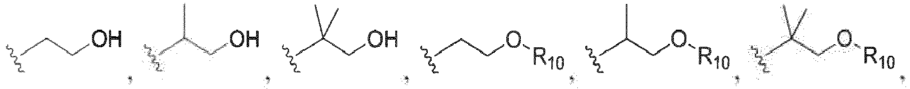 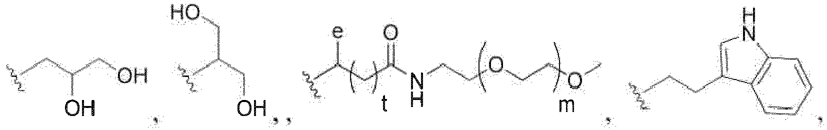 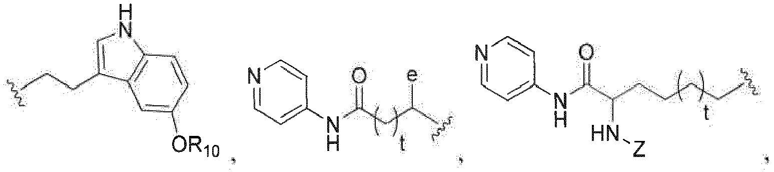 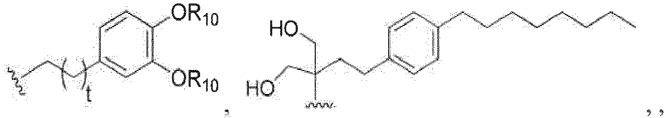 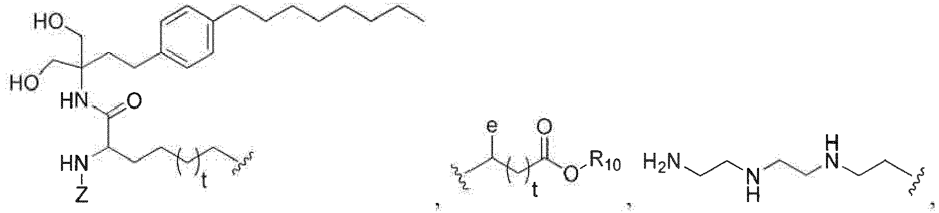 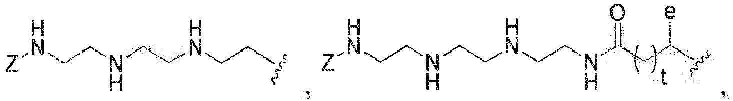 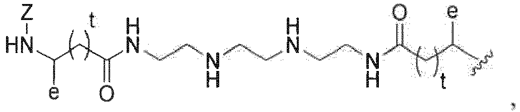 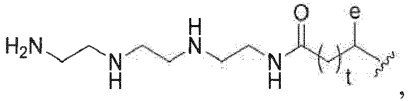 H, D, C₁-C₆ alkyl, C₃-C₆ cycloalkyl, aryl, heteroaryl, and heterocycle.
   each e is independently H or any one of the side chains of the naturally occurring amino acids;
   each m is independently 1, 2, 3, 4, 5, 6, 7, 8, 9, 10, 11, or 12;
   each R₁₀ is independently -H, straight or branched -C₁-C₆ alkyl, -C₁-C₆ cycloalkyl, aryl, heteroaryl or heterocyclic that is optionally substituted with one, two, three, four or five groups selected from OH, CN, halogen, CO₂R₉, CONHR₉, CONR₉R₉, S(O)₂NR₉R₉, NR₉R₉, NR₉COR₉,-(OCH₂CH₂)ₘ-OCH₃; and
   each R₉ is independently -H, -C₁-C₃ alkyl, or straight or branched C₁-C₄ alkyl optionally
   substituted with OH, or halogen.
52. A method for treating a metabolic disease by inhibiting proprotein convertase subtilisin/kexin type 9 (PCSK9) by administering to a patient in need thereof an effective amount of a compound of item 51.
53. The method of item 52, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
54. The method of item 52, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
55. The method of item 52, wherein the method further comprises administering another ACE inhibitor selected from the group consisting of enalapril, ramipril, quinapril, perindopril, lisinopril, imidapril, zofenopril, trandolapril, fosinopril, and captopril.
56. The method of item 52, wherein the method further comprises administering another fibrate selected from the group consisting of bezafibrate, citprofibrate, clofibrate, gemfibrozil, and fenofibrate.
57. The method of item 52, wherein the method further comprises administering another angiotensin II receptor blocker (ARB) selected from the group consisting of termisartan, losartan, irbesartan, azilsartan, and olmesartan.
58. The method of item 52, wherein the method further comprises administering another therapeutic agent selected from the group consisting of a PCSK9 monoclonal antibody, a biologic agent, a small interfering RNA (siRNA) and a gene silencing oligonucleotide.
59. The method of item 52, wherein the method further comprises administering another omega-3 fatty acid selected from the group consisting of *all-cis-*7,10,13-hexadecatrienoic acid, α-linolenic acid (ALA or *all-cis-*9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis-*9,12,15,18,21-docosahexaenoic acid), or tetracosahexaenoic acid (nisinic acid or *all-cis-*6,9,12,15,18,21-tetracosenoic acid).
60. The method of item 52, wherein the method further comprises administering another therapeutic agent selected from the group consisting of niacin, acifran and acipimox.
61. A method of inhibiting the production of PCSK9 or lowering serum levels of PCSK9 is provided, the method comprising administering to a patient in need thereof a compound of the Formula IV: 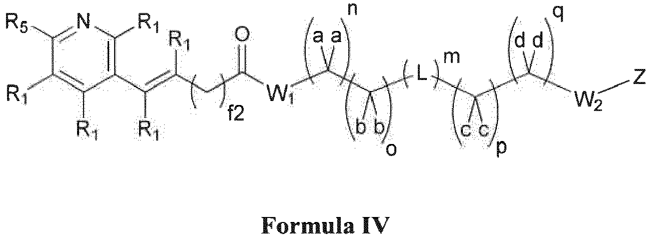 or a pharmaceutically acceptable salt, hydrate, solvate, prodrug, enantiomer or a stereoisomer thereof
   R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₅ is independently selected from the group consisting of H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₆ alkyl, -O-C₁-C₃ alkyl, -S(O)C₁-C₃ alkyl, -S(O)₂C₁-C₃ alkyl, an aryl, a cycloalkyl, a heterocycle and 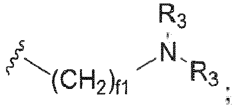 R₃ is independently H or C₁-C₆ alkyl, or both R₃ groups, when taken together with the nitrogen to which they are attached, can form 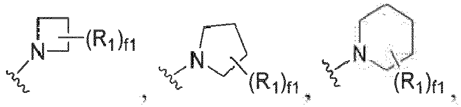 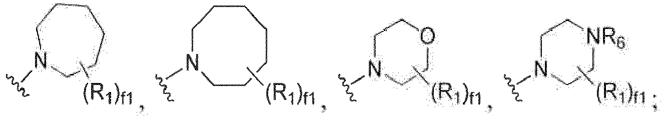 f1 = 1, 2, 3 or 4;
   f2 = 1, 2 or 3;
   W₁ and W₂ are each independently null, O, S, NH, NR, or W₁ and W₂ can be taken together can form an imidazolidine or piperazine group, with the proviso that W₁ and W₂ can not be O simultaneously;
   each a, b, c, and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently-O-, -S-, -S(O)-, -S(O)₂-, -S-S-, -(C₁-C₆alkyl)-, -(C₃-C₆cycloalkyl)-, a heterocycle, a heteroaryl, 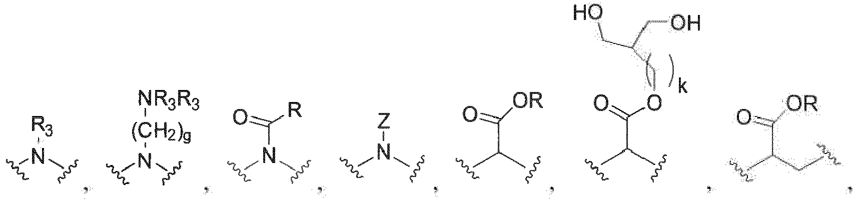 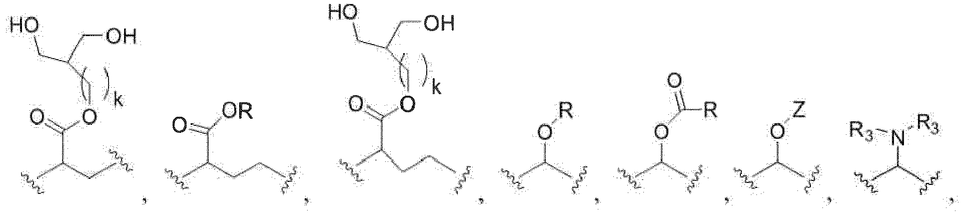 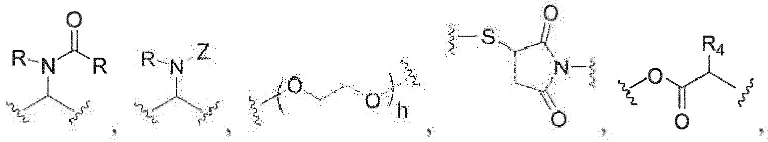 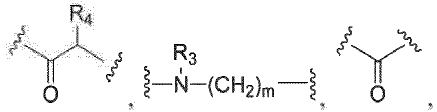 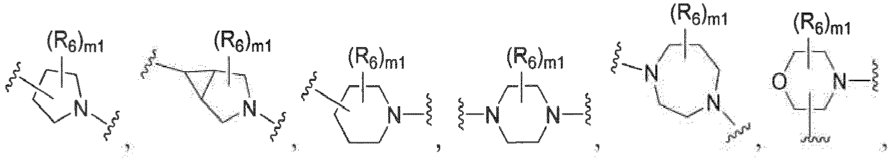 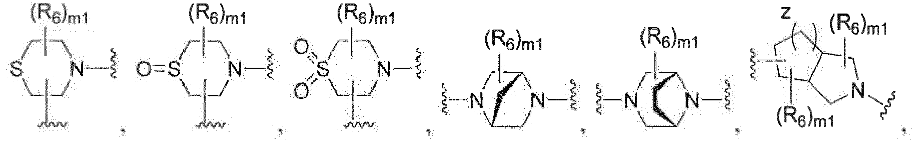 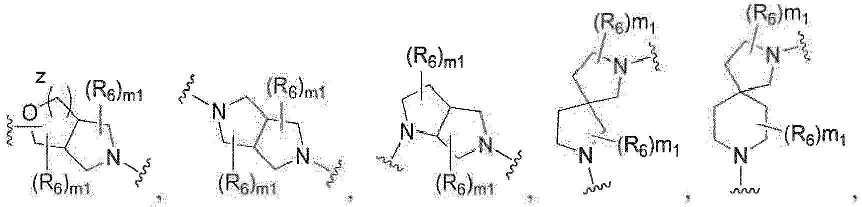 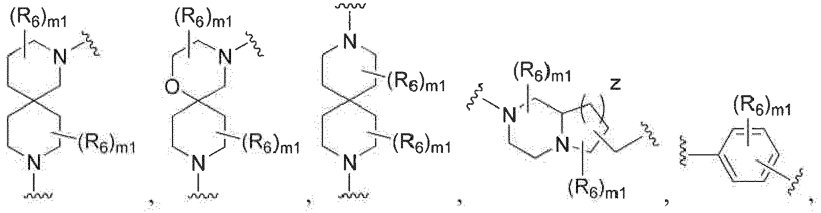 wherein the representation of L is not limited directionally left to right as is depicted, rather either the left side or the right side of L can be bound to the W₁ side of the compound of Formula IV;
   R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   each g is independently 2, 3 or 4;
   each h is independently 1, 2, 3 or 4;
   m is 0, 1, 2, 3, 4 or 5; if m is more than 1, then L can be the same or different;
   m1 is 0, 1, 2 or 3;
   k is 0, 1, 2, or 3;
   z is 1, 2, or 3;
   each R₄ independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, or 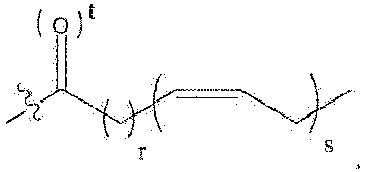 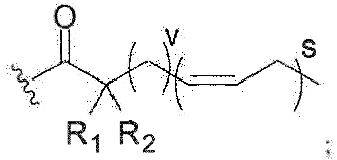 or 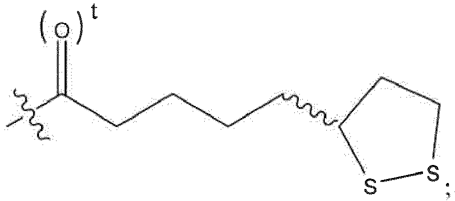 with the proviso that there is at least one 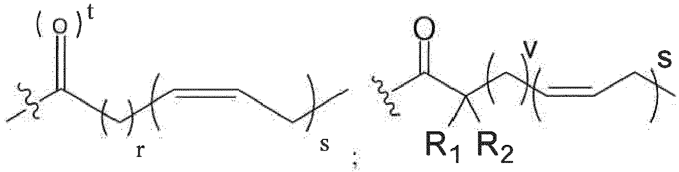 or 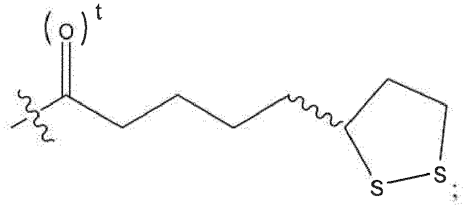 in the compound;
   each r is independently 2, 3, or 7;
   each s is independently 3, 5, or 6;
   each t is independently 0 or 1;
   each v is independently 1, 2, or 6;
   each R is independently -H, -C₁-C₃ alkyl, or straight or branched C₁-C₄ alkyl optionally substituted with OH, or halogen;
   provided that
   when m, n, o, p, and q are each 0, W₁ and W₂ are each null, and Z is 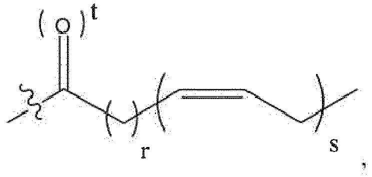 then t must be 0; and
   when m, n, o, p, and q are each 0, and W₁ and W₂ are each null, then Z must not be 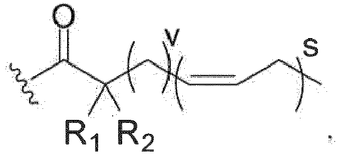
62. A method for treating a metabolic disease by inhibiting proprotein convertase subtilisin/kexin type 9 (PCSK9) by administering to a patient in need thereof an effective amount of a compound of item 61.
63. The method of item 62, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
64. The method of item 62, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
65. The method of item 62, wherein the method further comprises administering another ACE inhibitor selected from the group consisting of enalapril, ramipril, quinapril, perindopril, lisinopril, imidapril, zofenopril, trandolapril, fosinopril, and captopril.
66. The method of item 62, wherein the method further comprises administering another fibrate selected from the group consisting of, bezafibrate, citprofibrate, clofibrate, gemfibrozil, and fenofibrate.
67. The method of item 62, wherein the method further comprises administering another angiotensin II receptor blocker (ARB) selected from the group consisting of termisartan, losartan, irbesartan, azilsartan, and olmesartan.
68. The method of item 62, wherein the method further comprises administering another therapeutic agent selected from the group consisting of a PCSK9 monoclonal antibody, a biologic agent, a small interfering RNA (siRNA) and a gene silencing oligonucleotide.
69. The method of item 62, wherein the method further comprises administering another omega-3 fatty acid selected from the group consisting of *all-cis*-7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
70. The method of item 62, wherein the method further comprises administering another therapeutic agent selected from the group consisting of niacin, acifran and acipimox.
71. A method of inhibiting the production of PCSK9 or lowering serum levels of PCSK9 is provided, the method comprising administering to a patient in need thereof a compound of the **Formula V:** 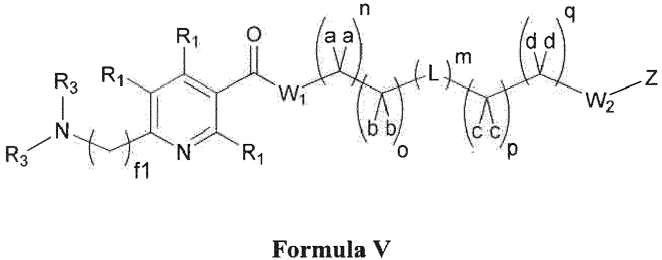 or a pharmaceutically acceptable salt, hydrate, solvate, prodrug, enantiomer or a stereoisomer thereof;
   wherein
   R₁ and R₂ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   R₃ is independently H or C₁-C₆ alkyl, or both R₃ groups, when taken together with the nitrogen to which they are attached, can form 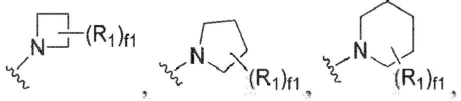 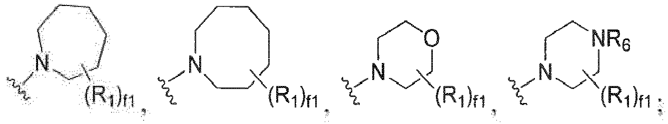 f1 = 1, 2, 3 or 4;
   W₁ and W₂ are each independently null, O, S, NH, NR, or W₁ and W₂ can be taken together can form an imidazolidine or piperazine group, with the proviso that W₁ and W₂ can not be O simultaneously;
   each a, b, c, and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
   each n, o, p, and q is independently 0, 1 or 2;
   each L is independently-O-, -S-, -S(O)-, -S(O)₂-, -S-S-, -(C₁-C₆alkyl)-, -(C₃-C₆cycloalkyl)-, a heterocycle, a heteroaryl, 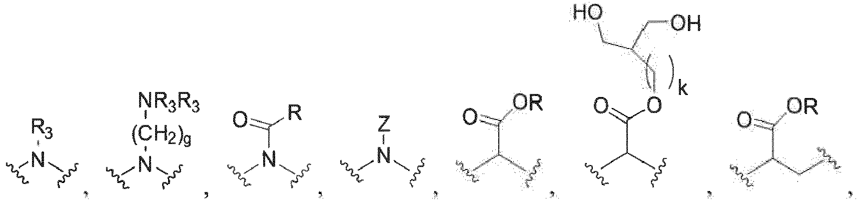 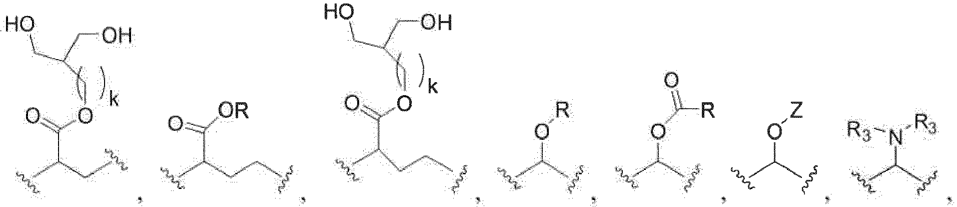 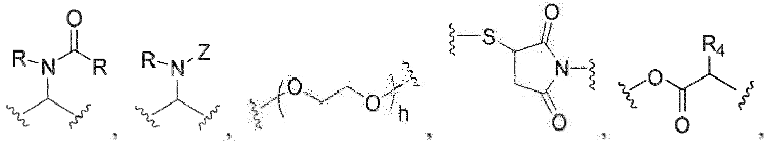 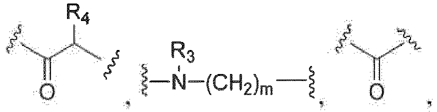 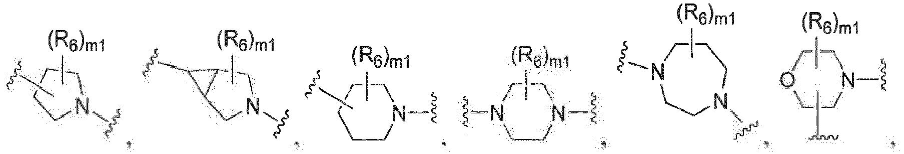 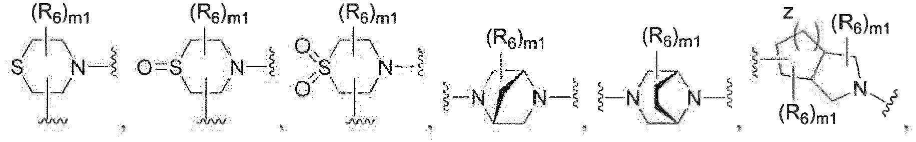 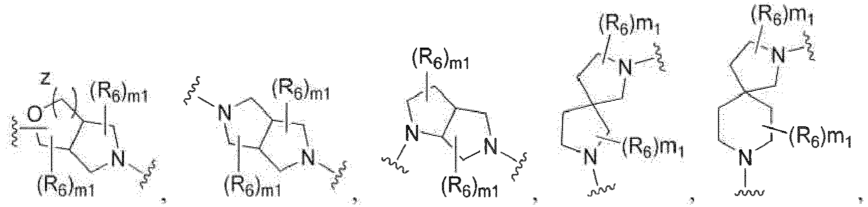 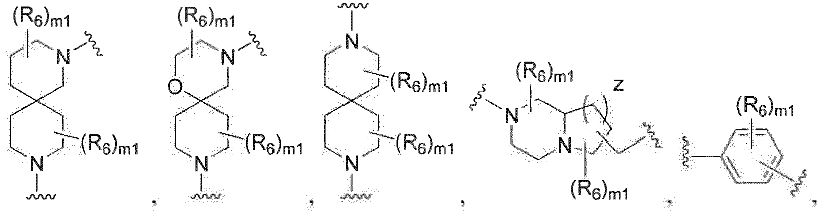 wherein the representation of L is not limited directionally left to right as is depicted, rather either the left side or the right side of L can be bound to the W₁ side of the compound of Formula V;
   R₆ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
   each g is independently 2, 3 or 4;
   each h is independently 1, 2, 3 or 4;
   m is 0, 1, 2, 3, 4 or 5; if m is more than 1, then L can be the same or different;
   ml is 0, 1, 2 or 3;
   k is 0, 1, 2, or 3;
   z is 1, 2, or 3;
   each R₄ independently e, H or straight or branched C₁-C₁₀ alkyl which can be optionally substituted with OH, NH₂, CO₂R, CONH₂, phenyl, C₆H₄OH, imidazole or arginine;
   each e is independently H or any one of the side chains of the naturally occurring amino acids;
   each Z is independently -H, or 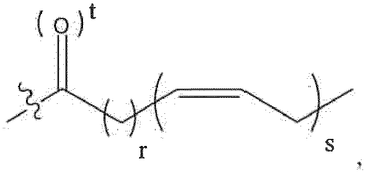 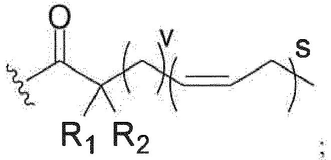 or 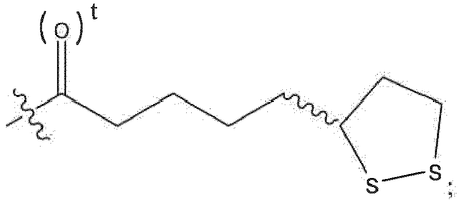 with the proviso that there is at least one 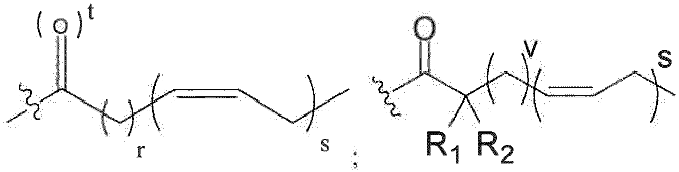 or 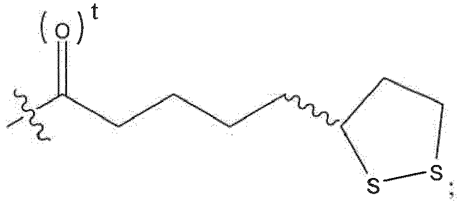 in the compound;
   each r is independently 2, 3, or 7;
   each s is independently 3, 5, or 6;
   each t is independently 0 or 1;
   each v is independently 1, 2, or 6;
   each R is independently -H, -C₁-C₃ alkyl, or straight or branched C₁-C₄ alkyl optionally substituted with OH, or halogen;
   provided that
   when m, n, o, p, and q are each 0, W₁ and W₂ are each null, and Z is 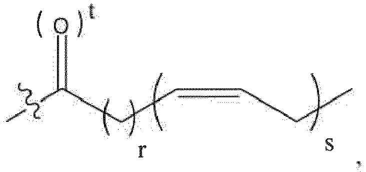 then t must be 0; and
   when m, n, o, p, and q are each 0, and W₁ and W₂ are each null, then Z must not be 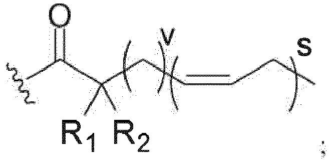
72. A method for treating a metabolic disease by inhibiting proprotein convertase subtilisin/kexin type 9 (PCSK9) by administering to a patient in need thereof an effective amount of a compound of item 71.
73. The method of item 72, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
74. The method of item 72, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
75. The method of item 72, wherein the method further comprises administering another ACE inhibitor selected from the group consisting of enalapril, ramipril, quinapril, perindopril, lisinopril, imidapril, zofenopril, trandolapril, fosinopril, and captopril.
76. The method of item 72, wherein the method further comprises administering another fibrate selected from the group consisting of bezafibrate, citprofibrate, clofibrate, gemfibrozil, and fenofibrate.
77. The method of item 72, wherein the method further comprises administering another angiotensin II receptor blocker (ARB) selected from the group consisting of termisartan, losartan, irbesartan, azilsartan, and olmesartan.
78. The method of item 72, wherein the method further comprises administering another therapeutic agent selected from the group consisting of a PCSK9 monoclonal antibody, a biologic agent, a small interfering RNA (siRNA) and a gene silencing oligonucleotide.
79. The method of item 72, wherein the method further comprises administering another omega-3 fatty acid selected from the group consisting of *all-cis*-7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
80. The method of item 72, wherein the method further comprises administering another therapeutic agent selected from the group consisting of niacin, acifran and acipimox.
81. A method for treating a metabolic disease by inhibiting proprotein convertase subtilisin/kexin type 9 (PCSK9) by administering to a patient in need thereof an effective amount of a fatty acid niacin conjugate selected from a group consisting of: 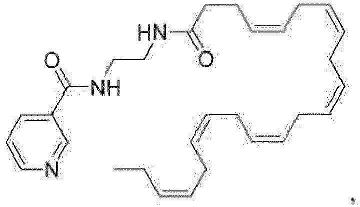
   N-(2-((4Z,7Z,10Z,13Z,16Z,19Z)-docosa-4,7,10,13,16,19-hexaenamido)ethyl)nicotinamide **(I-7)** 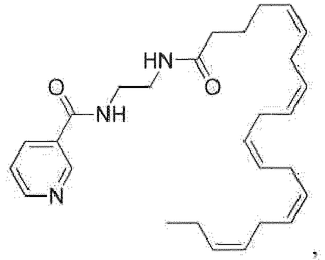
      N-(2-((5Z,8Z,11Z,14Z,17Z)-icosa-5,8,11,14,17-pentaenamido)ethyl)nicotinamide **(I-8)** 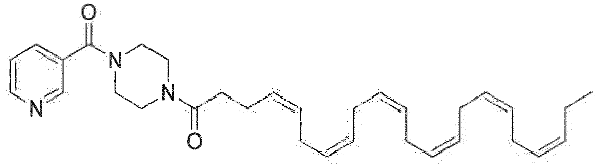
   (4Z,7Z,10Z,13Z,16Z,19Z)-1-(4-nicotinoylpiperazin-1-yl)docosa-4,7,10,13,16,19-hexaen-1-one **(I-12)** 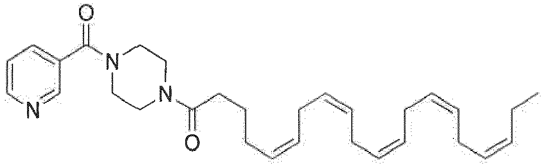
   (5Z,8Z,11Z,14Z,17Z)-1-(4-nicotinoylpiperazin-1-yl)icosa-5,8,11,14,17-pentaen-1-one **(I-13)** 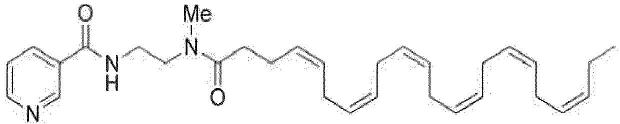
   N-(2-((4Z,7Z,10Z,13Z, 16Z,19Z)-N-methyldocosa-4,7,10,13,16,19-hexaenamido)ethyl)nicotinamide **(I-14)** 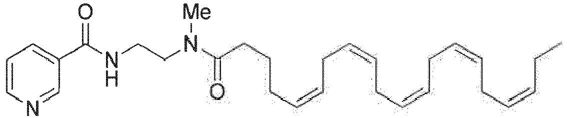
   N-(2-((5Z,8Z,11Z,14Z,17Z)-N-methylicosa-5,8,11,14,17-pentaenamido)ethyl)nicotinamide **(I-15)** 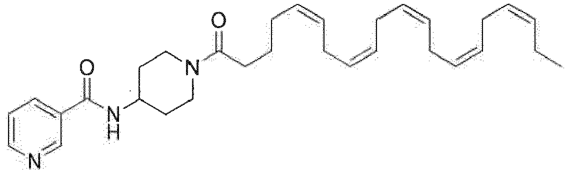
   N-(1-((5Z,8Z,11Z,14Z,17Z)-icosa-5,8,11,14,17-pentaenoyl)piperidin-4-yl)nicotinamide **(I-22)** 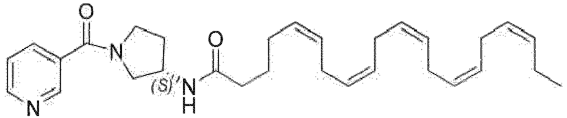
   (5Z,8Z,11Z,14Z,17Z)-N-((S)-1-nicotinoylpyrrolidin-3-yl)icosa-5,8,11,14,17-pentaenamide (I-23) 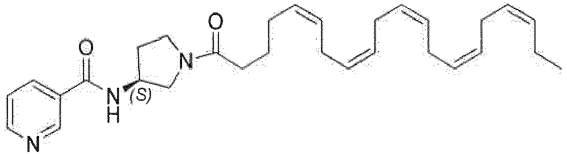
   N-((S)-1-((5Z,8Z,11Z,14Z,17Z)-icosa-5,8,11,14,17-pentaenoyl)pyrrolidin-3-yl)nicotinamide **(I-24)** 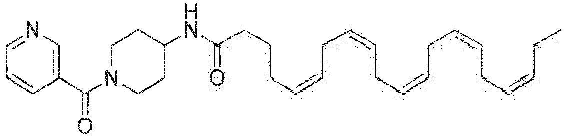
   (5Z,8Z,11Z,14Z,17Z)-N-(1-nicotinoylpiperidin-4-yl)icosa-5,8,11,14,17-pentaenamide (**I-28**) 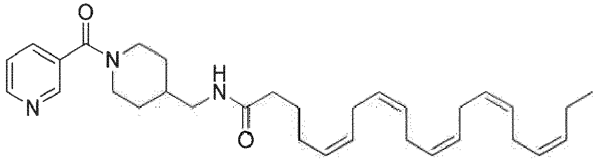
   (5Z,8Z,11Z,14Z,17Z)-N-((1-nicotinoylpiperidin-4-yl)methyl)icosa-5,8,11,14,17-pentaenamide **(I-29)** 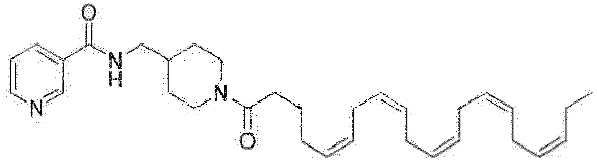
   N-((1-((5Z,8Z,11Z,14Z,17Z)-icosa-5,8,11,14,17-pentaenoyl)piperidin-4-yl)methyl)nicotinamide **(I-31)** 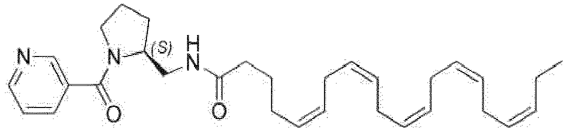
   (5Z,8Z,11Z,14Z,17Z)-N-((S)-1-nicotinoylpyrrolidin-2-yl)methyl)icosa-5,8,11,14,17-pentaenamide **(I-32)** 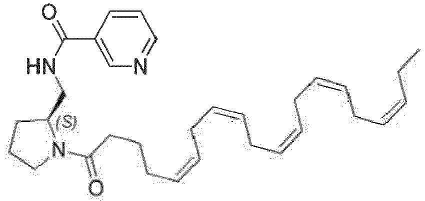
   N-(((S)-1-((5Z,8Z,11Z,14Z,17Z)-icosa-5,8,11,14,17-pentaenoyl)pyrrolidin-2-yl)methyl)nicotinamide **(I-34)** 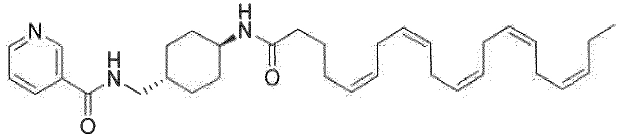
   N-(((1R,4R)-4-((5Z,8Z,11Z,14Z,17Z)-icosa-5,8,11,14,17-pentaenamido)cyclohexyl)methyl)nicotinamide **(I-41)** 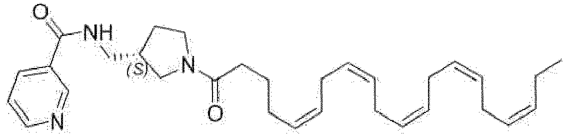
   N-(((S)-1-((5Z,8Z,11Z,14Z,17Z)-icosa-5,8,11,14,17-pentaenoyl)pyrrolidin-3-yl)methyl)nicotinamide **(I-43)** 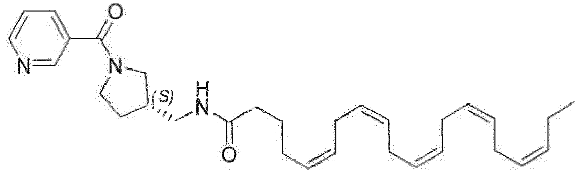
   (5Z,8Z,11Z,14Z,17Z)-N-(((S)-1-nicotinoylpyrrolidin-3-yl)methyl)icosa-5,8,11,14,17-pentaenamide **(I-46)** 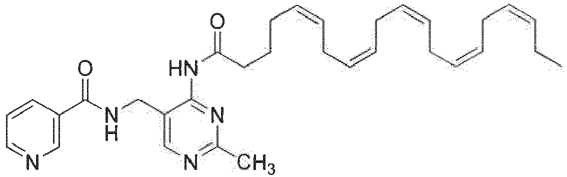
   N-((4-((5Z,8Z,11Z,14Z,17Z)-icosa-5,8,11,14,17-pentaenamido)-2-methylpyrimidin-5-yl)methyl)nicotinamide **(I-64)**
82. The method of item 81, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
83. The method of item 2, wherein the method further comprises administering another therapeutic agent selected from the group consisting ofatorvastatin, cerivastatin, fluvastatin, lovastatin, pitavastatin, pravastatin, rosuvastatin, simvastatin, ezetimibe, and the combination of ezetimibe/simvastatin (Vytorin®).
84. The method of item 2, wherein the method further comprises administering another ACE inhibitor selected from the group consisting of enalapril, ramipril, quinapril, perindopril, lisinopril, imidapril, zofenopril, trandolapril, fosinopril, and captopril.
85. The method of item 2, wherein the method further comprises administering another fibrate selected from the group consisting of bezafibrate, citprofibrate, clofibrate, gemfibrozil, and fenofibrate.
86. The method of item 2, wherein the method further comprises administering another angiotensin II receptor blocker (ARB) selected from the group consisting of termisartan, losartan, irbesartan, azilsartan, and olmesartan.
87. The method of item 2, wherein the method further comprises administering another therapeutic agent selected from the group consisting of a PCSK9 monoclonal antibody, a biologic agent, a small interfering RNA (siRNA) and a gene silencing oligonucleotide.
88. The method of item 2, wherein the method further comprises administering another omega-3 fatty acid selected from the group consisting of *all-cis*-7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
89. The method of item 2, wherein the method further comprises administering another therapeutic agent selected from the group consisting ofniacin, acifran and acipimox.
90. A method for treating a metabolic disease by inhibiting proprotein convertase subtilisin/kexin type 9 (PCSK9) by administering to a patient in need thereof an effective amount of a compound of claim. 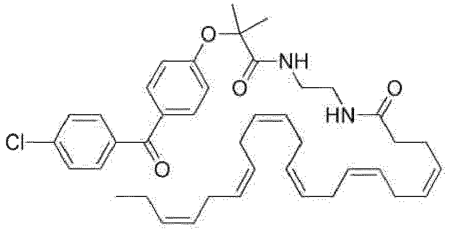
   (4Z,7Z,10Z,13Z, 16Z,19Z)-N-(2-(2-(4-(4-chlorobenzoyl)phenoxy)-2-methylpropanamido)ethyl)docosa-4,7,10,13,16,19-hexaenamide **(II-36)** 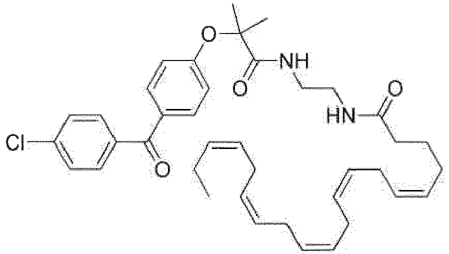
   (5Z,8Z,11Z,14Z,17Z)-N-(2-(2-(4-(4-chlorobenzoyl)phenoxy)-2-methylpropanamido)ethyl)icosa-5,8,11,14,17-pentaenamide **(II-37)** 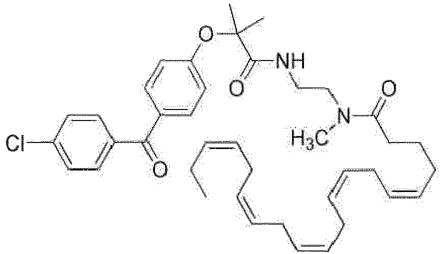
   (5Z,8Z,11Z,14Z,17Z)-N-(2-(2-(4-(4-chlorobenzoyl)phenoxy)-2-methylpropanamido)ethyl)-N-methylicosa-5,8,11,14,17-pentaenamide **(II-40)** 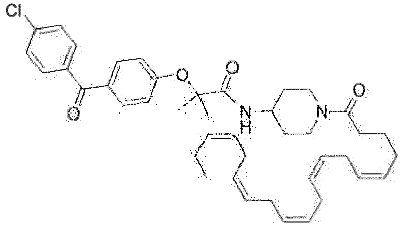
   2-(4-(4-chlorobenzoyl)phenoxy)-N-(1-((5Z,8Z,11Z,14Z,17Z)-icosa-5,8,11,14,17-pentaenoyl)piperidin-4-yl)-2-methylpropanamide (VII-19) 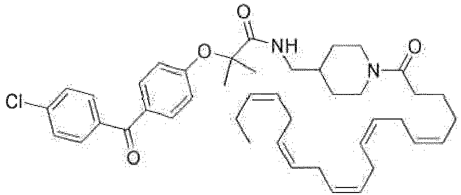
   2-(4-(4-chlorobenzoyl)phenoxy)-N-((1-((5Z,8Z,11Z,14Z,17Z)-icosa-5,8,11,14,17-pentaenoyl)piperidin-4-yl)methyl)-2-methylpropanamide **(VII-20)** 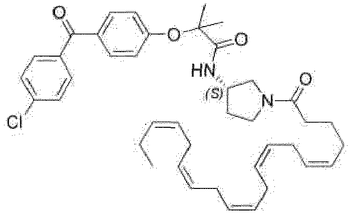
   2-(4-(4-chlorobenzoyl)phenoxy)-N-((S)-1-((5Z,8Z,11Z,14Z,17Z)-icosa-5,8,11,14,17-pentaenoyl)pyrrolidin-3-yl)-2-methylpropanamide **(VII-22)** 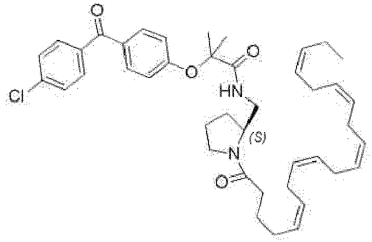
   2-(4-(4-chlorobenzoyl)phenoxy)-N-(((S)-1-((5Z,8Z,11Z,14Z,17Z)-icosa-5,8,11,14,17-pentaenoyl)pyrrolidin-2-yl)methyl)-2-methylpropanamide **(VII-24)** 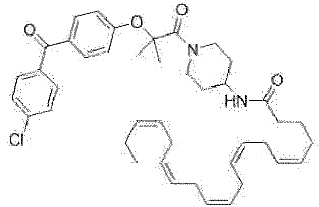
   (5Z,8Z,11Z,14Z,17Z)-N-(1-(2-(4-(4-chlorobenzoyl)phenoxy)-2-methylpropanoyl)piperidin-4-yl)icosa-5,8,11,14,17-pentaenamide **(VII-25)** 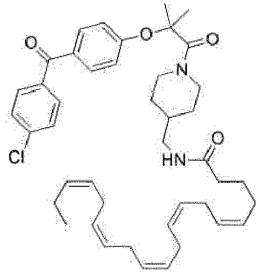
   (5Z,8Z,11Z,14Z,17Z)-N-((1-(2-(4-(4-chlorobenzoyl)phenoxy)-2-methylpropanoyl)piperidin-4-yl)methyl)icosa-5,8,11,14,17-pentaenamide **(VII-26)** 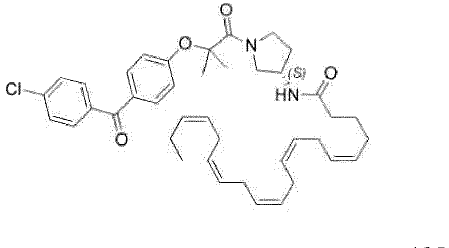
   (5Z,8Z,11Z,14Z,17Z)-N-((S)-1-(2-(4-(4-chlorobenzoyl)phenoxy)-2-methylpropanoyl)pyrrolidin-3-yl)icosa-5,8,11,14,17-pentaenamide **(VII-28)** 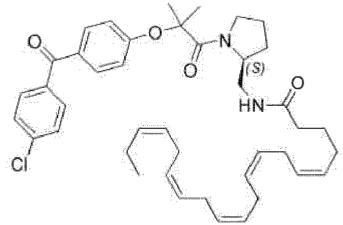
   (5Z,8Z,11Z,14Z,17Z)-N-(((S)-1-(2-(4-(4-chlorobenzoyl)phenoxy)-2-methylpropanoyl)pyrrolidin-2-yl)methyl)icosa-5,8,11,14,17-pentaenamide **(VII-30)**
91. The method of item 90, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia (which can include 3 subtypes: Type Ia, also called Buerger-Gruetz syndrome or familial hyperchylomicronemia; Type Ib, also called familial apoprotein CII deficiency, and Type Ic), Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.
92. The method of item 91, wherein the method further comprises administering another therapeutic agent selected from the group consisting of atorvastatin, cerivastatin, fluvastatin, lovastatin, pitavastatin, pravastatin, rosuvastatin, simvastatin, ezetimibe, and the combination of ezetimibe/simvastatin (Vytorin®).
93. The method of item 91, wherein the method further comprises administering another ACE inhibitor selected from the group consisting of enalapril, ramipril, quinapril, perindopril, lisinopril, imidapril, zofenopril, trandolapril, fosinopril, and captopril.
94. The method of item 91, wherein the method further comprises administering another fibrate selected from the group consisting of bezafibrate, citprofibrate, clofibrate, gemfibrozil, and fenofibrate.
95. The method of item 91, wherein the method further comprises administering another angiotensin II receptor blocker (ARB) selected from the group consisting of termisartan, losartan, irbesartan, azilsartan, and olmesartan.
96. The method of item 91, wherein the method further comprises administering another therapeutic agent selected from the group consisting of a PCSK9 monoclonal antibody, a biologic agent, a small interfering RNA (siRNA) and a gene silencing oligonucleotide.
97. The method of item 91, wherein the method further comprises administering another omega-3 fatty acid selected from the group consisting of *all-cis*-7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis-*11,14,17-eicosatrienoic acid), eicosatetraenoic acid (ETA or *all-cis*-8,11,14,17-eicosatetraenoic acid), eicosapentaenoic acid (EPA or *all-cis*-5,8,11,14,17-eicosapentaenoic acid), docosapentaenoic acid (DPA, clupanodonic acid or *all-cis-*7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis-*6,9,12,15,18,21-tetracosenoic acid).
98. The method of item 91, wherein the method further comprises administering another therapeutic agent selected from the group consisting of niacin, acifran and acipimox.

## Claims

1. A compound of the **Formula VI:** 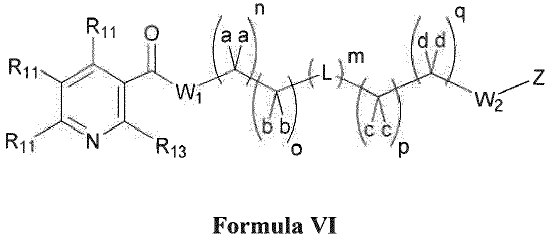 or a pharmaceutically acceptable salt, hydrate, enantiomer or stereoisomer thereof;
wherein
W₁ and W₂ are each independently null, O, S, NH, NR, or W₁ and W₂ can be taken together can form an imidazolidine or piperazine group, with the proviso that W₁ and W₂ cannot be O simultaneously;
R₁₁ is independently H, -OH, -OC(O)-R, -O-aryl, -aryl, -heteroaryl, or -heterocyclic;
R₁₃ is independently H, C₁₋C₃alkyl, -OH, -OC(O)-R, or halogen;
each a, b, c and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
each n, o, p, and q is independently 0, 1 or 2;
each L is independently 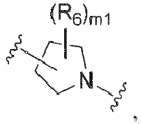 wherein the representation of L is not limited directionally left to right as is depicted, rather either the left side or the right side of L can be bound to the W₁ side of the compound of Formula VI;
R₆ is -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₂-C₃ alkene, -C₂-C₃ alkyne, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, or -S(O)₂C₁-C₃ alkyl;
m is 0, 1, 2, or 3;
ml is 0, 1, 2 or 3;
each Z is independently -H, 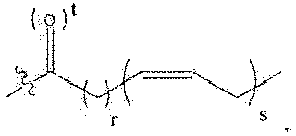 or 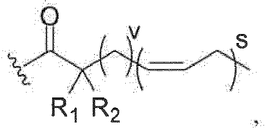 with the proviso that there is at least one 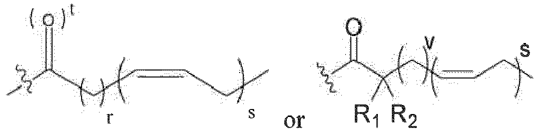 in the compound;
each r is independently 2, 3, or 7;
each s is independently 3, 5, or 6;
each t is independently 0 or 1;
each v is independently 1, 2, or 6;
R₁ and R₂ are each independently hydrogen, deuterium, -C₁-C₄ alkyl, -halogen,-OH, -C(O)C₁-C₄ alkyl, -O-aryl, -O-benzyl, -OC(O)C₁-C₄ alkyl, -C₂-C₃ alkene, -C₂-C₃ alkyne, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, or -S(O)₂C₁-C₃ alkyl; and
each R is independently -H, phenyl or straight or branched C₁-C₄ alkyl optionally substituted with OH, or halogen.

2. The compound of claim 1, wherein the compound is selected from the group consisting of: 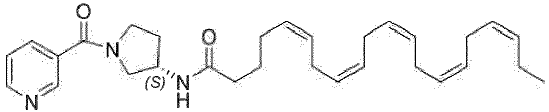
(5Z,8Z,11Z,14Z,17Z)-N-((S)-1-nicotinoylpyrrolidin-3-yl)icosa-5,8,11,14,17-pentaenamide (compound **I-23**); 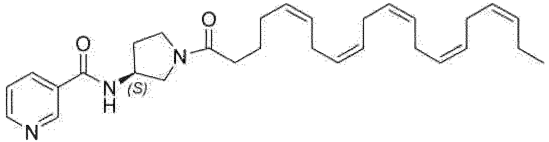
N-((S)-1-((5Z,8Z,11Z,14Z,17Z)-icosa-5,8,11,14,17-pentaenoyl)pyrrolidin-3-yl)nicotinamide (compound **I-24**); and 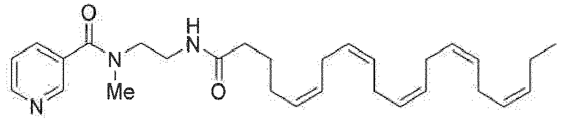
N-(2-((5Z,8Z,11Z,14Z,17Z)-icosa-5,8,11,14,17-pentaenamido)ethyl)-N-methylnicotinamide (compound **I-16**).

3. The compound of claim 2, wherein the compound is 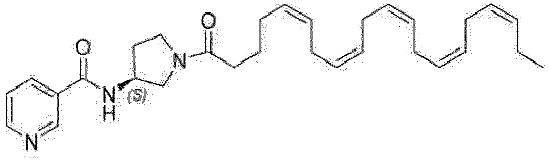
N-((S)-1-((5Z,8Z,11Z,14Z,17Z)-icosa-5,8,11,14,17-pentaenoyl)pyrrolidin-3-yl)nicotinamide (compound **I-24**).

4. The compound of claim 2, wherein the compound is 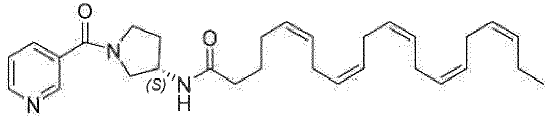
(5Z,8Z,11Z,14Z,17Z)-N-((S)-1-nicotinoylpyrrolidin-3-yl)icosa-5,8,11,14,17-pentaenamide (compound **I-23**).

5. The compound of claim 2, wherein the compound is 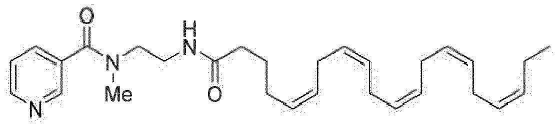
N-(2-((5Z,8Z,11Z,14Z,17Z)-icosa-5,8,11,14,17-pentaenamido)ethyl)-N-methylnicotinamide (compound **I-16**).

6. A pharmaceutical composition comprising a compound of any one of claims 1-5 and a pharmaceutically acceptable carrier.

7. A compound or composition of any one of claims 1-6 for use in the treatment of a metabolic disease in a subject in need thereof.

8. The method of claim 7, wherein the metabolic disease is selected from hypertriglyceridemia, severe hypertriglyceridemia, hypercholesterolemia, familial hypercholesterolemia, elevated cholesterol caused by a genetic condition, fatty liver disease, nonalcoholic fatty liver disease (NFLD), nonalcoholic steatohepatitis (NASH), dyslipidemia, mixed dyslipidemia, Type I hyperlipoproteinemia, Type V hyperlipoproteinemia, atherosclerosis, coronary heart disease, Type 2 diabetes, diabetic nephropathy, diabetic neuropathy, diabetic retinopathy, metabolic syndrome, or cardiovascular disease.

9. The method of claim 8, wherein the metabolic disease is hypertriglyceridemia or hypercholesterolemia.

10. A compound or composition of any one of claims 1-6 for use in the treatment of a metabolic disease by inhibiting proprotein convertase subtilisin/kexin type 9 (PCSK9).

11. The method of claim 10, wherein the metabolic disease is hypertriglyceridemia or hypercholesterolemia.

12. A compound, wherein the compound is 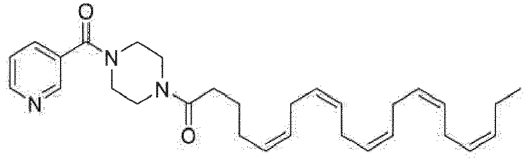
(5Z,8Z,11Z,14Z,17Z)-1-(4-nicotinoylpiperazin-1-yl)icosa-5,8,11,14,17-pentaen-1-one (compound **I-13**).

13. A pharmaceutical composition comprising the compound of claim 12 and a pharmaceutically acceptable carrier.

14. A compound or composition of any one of claims 12-13 for use in the treatment of a metabolic disease in a subject in need thereof.

15. The method of claim 14, wherein the metabolic disease is hypercholesterolemia or hypertriglyceridemia.
